# EUROPEAN PATENT SPECIFICATION

(11) **EP 4 308 105 B1**
(45) Date of publication and mention of the grant of the patent: **09.12.2026**
(21) Application number: 22772322.8
(22) Date of filing: 18.03.2022
(51) Int. Cl.: A61K 31/335, C07D 309/02, A61K 47/54, A61K 47/69, A61P 35/00, C07K 5/02, C07K 5/062, C07J 43/00

(54) **COMPOUNDS FOR REGULATING TRAINED IMMUNITY, AND THEIR METHODS OF USE**
VERBINDUNGEN ZUR REGULIERUNG DER TRAINIERTEN IMMUNITÄT UND VERFAHREN ZU DEREN VERWENDUNG
COMPOSÉS POUR RÉGULER L'IMMUNITÉ ENTRAÎNÉE, ET LEURS PROCÉDÉS D'UTILISATION

(30) Priority: 19.03.2021 US 202163163428 P
(43) Date of publication of application: 24.01.2024
(73) Proprietor: Trained Therapeutix Discovery, Inc., Dallas, Texas 75222 (US); Icahn School of Medicine at Mount Sinai, New York, NY 10029 (US)
(72) Inventor: JANSSEN, Henricus Marie, Dallas, Texas 75222 (US); HOEBEN, Freek Johannes Maria, Dallas, Texas 75222 (US); GENABEEK, Bas Van, Dallas, Texas 75222 (US); SONTJENS, Serge Hendrikus Mathijs, Dallas, Texas 75222 (US); MULDER, Willem M.J., New York, New York 10029 (US)
(74) Representative: Grünecker Patent- und Rechtsanwälte PartG mbB
(86) International application number: PCT/US2022/021035
(87) International publication number: WO 2022/198101

(56) References cited:
- US-A1- 2018 250 419
- US-A1- 2019 290 593
- OGAWA CHIKAKO ET AL: "Muramyl Dipeptide and its Derivatives: Peptide Adjuvant in Immunological Disorders and Cancer Therapy", vol. 7, no. 3, 1 September 2011 (2011-09-01), pages 180 - 197, XP055966913, ISSN: 1573-4072, Retrieved from the Internet <URL:https://www.ncbi.nlm.nih.gov/pmc/articles/PMC3241611/pdf/nihms341856.pdf> DOI: 10.2174/157340711796817913
- DATABASE Pubchem Substance [online] 5 December 2007 (2007-12-05), "SUBSTANCE RECORD SID 40944914", XP055972576, Database accession no. 40944914

## Description

### CROSS-REFERENCE TO RELATED APPLICATIONS

This application claims priority to U.S. Provisional Application No. 63/163,428, filed March 19, 2021.

### DESCRIPTION OF THE TEXT FILE SUBMITTED ELECTRONICALLY

The contents of the text file submitted electronically herewith are incorporated herein by reference in their entirety: A computer readable format copy of the Sequence Listing (filename: TRAI_005_00US_SeqList_ST25.txt, date recorded: March 17, 2021, file size 105 kilobytes).

### BACKGROUND

The immune system plays an essential role in the pathophysiology of major diseases such as atherosclerosis, diabetes, and cancer. However, most of the immunotherapy strategies currently being developed focus on either effector molecules, such as cytokines, or T lymphocytes, which are cells from the adaptive immune system. (Mulder et al. Nat. Rev. Drug Discov. 2019, 18(7), 553-566; Pardoll et al. Nat Immunol., 2012, 13, 1129-1132). In autoimmune and autoinflammatory diseases, anti-cytokine therapies can successfully neutralize bioactive cytokines, while the most intensely used immunotherapy in cancer patients comprises the application of checkpoint-inhibitor drugs. Though the innate immune system was long believed to lack memory, recent studies show that innate immune cells undergo metabolic and epigenetic rewiring, adjusting their functional programs in a process termed 'trained immunity' which has been implicated in exerting antitumor effects. (Buffen et al., 2014, PLoS Pathog. 10, e1004485; Netea et al., J. Leukoc Biol. 2017, 102, 1323-1332).

A range of pattern recognition receptors (PRRs), including TLRs, NOD2, dectin 1 and the inflammasome, can be engaged to promote trained immunity. In addition, *in vitro* studies have demonstrated that, BCG, and several other PAMPs and DAMPs, including peptidoglycans and β-glucan, can be therapeutically exploited as trained-immunity-promoting agents. However, *in vivo* therapeutic exploitation of molecules that regulate trained immunity has been hampered by toxicity, immune-related adverse effects and poor bioavailability to target the relevant myeloid cells and their progenitors. Muramyl dipeptide is a component of bacterial peptidoglycan, a recognition structure or activator for NOD2 protein. Analogues of muramyl dipeptide and their potential for immune response therapies in cancer and disease are being investigated. Ogawa et al., Current Bioactive Compounds, Volume 7, Number 3, 2011, pp. 180-197(18) discusses muramyl dipeptide and its derivatives as peptide adjuvant in immunological disorders and cancer therapy.

A need exists for therapeutic agents, and compositions thereof that engage the innate immune system, and regulate trained immunity to treat cancer, and other diseases and conditions caused by defective trained immunity.

### SUMMARY

In embodiments, provided herein is a compound of formula (I): 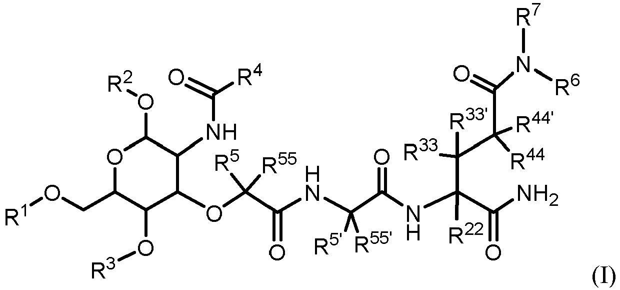 or a pharmaceutically acceptable salt thereof,
wherein:
R¹ is -H or -C(O)-R^{X};
R² and R³ are each independently selected from the group consisting of -H, alkyl, alkylene-aryl, -C(O)-alkyl, and -C(O)-aryl;
R⁴, R⁵, and R^{5'} are each alkyl;
R⁶ and R¹¹ are each independently -H, or alkyl;
R⁷ -Y-triazolyl-L;
Y is alkylene;
L is selected from the group consisting of a fatty acid chain, -alkylene-C(O)-W, - alkylene-O-C(O)-W, -alkylene-N-(alkylene-C(O)-NR¹¹-alkylene-NR¹¹-C(O)-W)₂, and - alkylene-N-(alkylene-C(O)-W)₂;
W is a fatty acid chain, -O-alkylene-C(H)(OR⁸)-alkylene-OR⁹, a phospholipid, or a sterol;
R⁸ and R⁹ are each independently R^{X} or -C(O)-R^{X};
R²², R³³, R^{33'}, R⁴⁴, R^{44'}, R⁵⁵, and R^{55'} are each independently H or R^{A};
R^{X} is a fatty acid chain;
wherein each aforementioned alkyl, alkylene, alkylene-aryl, aryl, and triazolyl is optionally substituted with one or more R^{A}, wherein R^{A} is independently selected for each occurrence from the group consisting of hydrogen, halo, alkoxy, haloalkoxy, cyano, hydroxyl, -N(R^{C})(R^{D}), -C(O)N(R^{C})(R^{D}), -N(R^{C})C(O)R^{B}, -OC(O)NR^{C}R^{D}, -NR^{C}C(O)OR^{B}, -OC(O)R^{B}, -C(O)OR^{B}, -C(O)R^{B} , -CO₂H, -NO₂, -SH, S(O)_{X}R^{B} aryl, arylalkyl, heteroaryl, heteroalalkyl, cycloalkyl, heterocycloalkyl, and R^{B}; wherein X is 0, 1, or 2;
R^{C} and R^{D} are independently selected for each occurrence from the group consisting of hydrogen, alkyl, haloalkyl -C(O)R^{B}, and -C(O)OR^{B}; or R^{C} and R^{D} are taken together with the nitrogen to which they are attached to form a heterocyclic ring optionally substituted with R^{A}; and
R^{B} is alkyl, alkenyl, or alkynyl optionally substituted with one or more fluoro.

In embodiments, the compound of formula (I) is a compound of formula (IA): 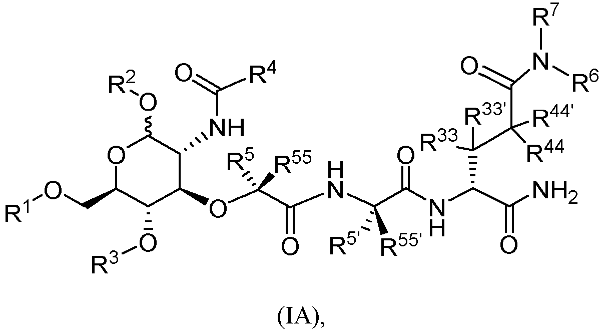 or a pharmaceutically acceptable salt thereof, wherein R¹, R², R³, R⁴, R⁵, R⁵⁵, R^{5'}, R^{55'}, R³³, R^{33'}, R⁴⁴, R^{44'}, R⁶, and R⁷ are defined herein.

In embodiments, the compound of formula (I) is a compound of formula (IB): 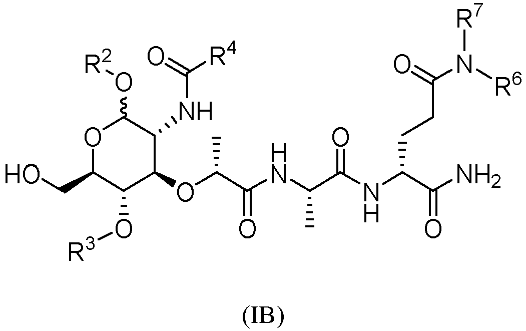 wherein R², R³, R⁴, R⁶, and R⁷ are defined herein.

In embodiments, the compound of formula (I) is a compound of formula (II): 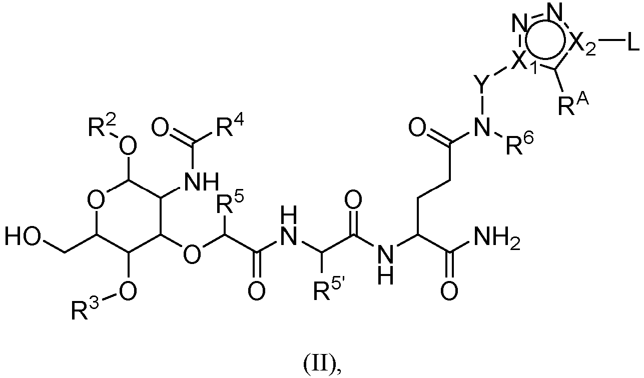 or a pharmaceutically acceptable salt thereof, wherein R², R³, R⁴, R⁵, R^{5'}, R⁶, Y, X¹, X², R^{A}, L, are defined herein.

In embodiments, the compound of formula (II) is: 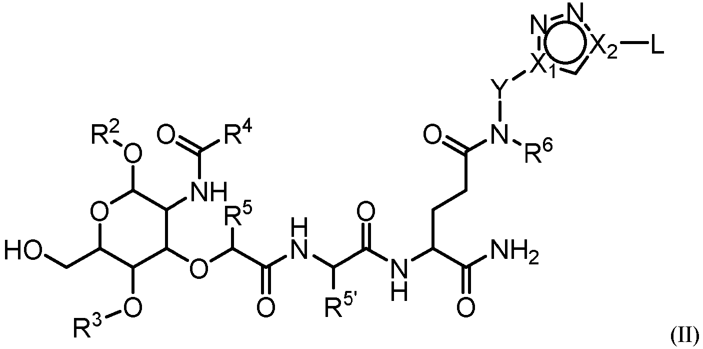 or a pharmaceutically acceptable salt thereof, wherein R², R³, R⁴, R⁵, R^{5'}, R⁶, Y, X¹, X², and L are defined herein.

In embodiments, the compound of formula (I) is a compound of formula (IIA): 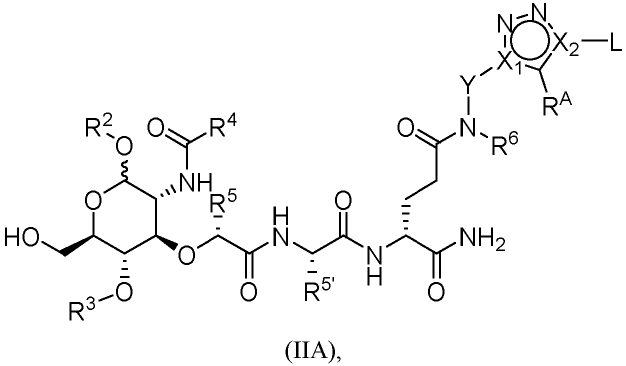 or a pharmaceutically acceptable salt thereof, wherein R², R³, R⁴, R⁵, R^{5'}, R⁶, Y, X¹, X², L, and R^{A} are defined herein.

In embodiments, the compound of formula (IIA) is: 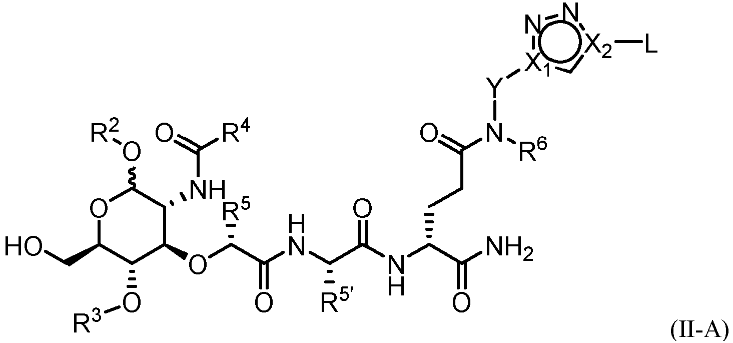 wherein R², R³, R⁴, R⁵, R^{5'}, R⁶, Y, X¹, X², and L are defined herein.

In embodiments, the present disclosure provides a nanobiologic composition comprising a high-density lipoprotein (HDL)-derived nanoparticle, wherein the nanoparticle comprises a compound of formula (I) as defined above.

In embodiments, the present disclosure provides compounds for use in a method for treating a cell-proliferation disorder, or sepsis in a patient in need thereof, comprising administering to the patient a therapeutically effective amount of a compound or nanobiologic composition disclosed herein.

### BRIEF DESCRIPTION OF FIGURES

**FIG. 1** shows Cryo-TEM pictures for the HDL-derived nanoparticles of entries A to F in Table 8 of Example 24. The 50 nm bar applies to all 6 pictures.
**FIG. 2** DLS-determined Z-averaged diameters and PDI-values for nanoparticles in formulations A to F in formulation 1 of Example 23.
**FIG. 3** is a graph showing OD values as a function of test article concentration in the NOD2 activation assay described in Example 25.
FIG. 4A**-E** depict tumor growth curves for the study described in Example 26.
FIG. 5A**-C** depict tumor growth curves for the study described in Example 27.

### DEFINITIONS

For convenience, certain terms employed in the specification, examples and claims are collected here. Unless defined otherwise, all technical and scientific terms used in this disclosure have the same meanings as commonly understood by one of ordinary skill in the art to which this disclosure belongs.

Throughout this disclosure, various patents, patent applications and publications are referenced. This disclosure will govern in the instance that there is any inconsistency between the patents, patent applications and publications cited and this disclosure.

The term "about" when immediately preceding a numerical value means a range of plus or minus an acceptable degree of variation in the art. In embodiments, the term "about" encompasses 10% of that value, e.g., "about 50" means 45 to 55, "about 25,000" means 22,500 to 27,500, etc., unless the context of the disclosure indicates otherwise, or is inconsistent with such an interpretation. For example in a list of numerical values such as "about 49, about 50, about 55, ...", "about 50" means a range extending to less than half the interval(s) between the preceding and subsequent values, e.g., more than 49.5 to less than 52.5. Furthermore, the phrases "less than about" a value or "greater than about" a value should be understood in view of the definition of the term "about" provided herein.

The phrase "pharmaceutically acceptable" as used herein refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Salts" include those obtained by reacting a compound functioning as a base, with an inorganic or organic acid to form a salt, or those obtained by reacting a compound functioning as an acid, with an inorganic or organic base to form a salt. "Salts" include derivatives of an active agent, wherein the active agent is modified by making acid or base addition salts thereof. Preferably, the salts are pharmaceutically acceptable salts. Such salts include, but are not limited to, pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, 3-(4-hydroxybenzoyl) benzoic acid, cinnamic, citric, fumaric, glycolic, lactic, maleic, malic, malonic, mandelic, oxalic, picric, pyruvic, salicylic, succinic, methanesulfonic, ethanesulfonic, 2-hydroxyethanesulfonic acid, tartaric, ascorbic, pamoic, bismethylene salicylic, ethanedisulfonic, gluconic, citraconic, aspartic, stearic, palmitic, EDTA, glycolic, p-aminobenzoic, glutamic, benzene sulfonic, 4-chlorobenzenesulfonic acid, 2-naphthalenesulfonic acid, camphorsulfonic acid, p-toluenesulfonic acids, sulphates, nitrates, phosphates, perchlorates, borates, acetates, benzoates, hydroxynaphthoates, glycerophosphates, ketoglutarates and the like. Base addition salts include but are not limited to, ethylenediamine, N-methyl-glucamine, lysine, arginine, ornithine, choline, N,N'-dibenzylethylenediamine, chloroprocaine, diethanolamine, procaine, N-benzylphenethylamine, diethylamine, piperazine, tris-(hydroxymethyl)-aminomethane, tetramethylammonium hydroxide, triethylamine, dibenzylamine, ephenamine, dehydroabietylamine, N-ethylpiperidine, benzylamine, tetramethylammonium, tetraethylammonium, methylamine, dimethylamine, trimethylamine, ethylamine, basic amino acids, e. g., lysine and arginine dicyclohexylamine and the like. Examples of metal salts include lithium, sodium, potassium, magnesium, calcium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like. Standard methods for the preparation of pharmaceutically acceptable salts and their formulations are well known in the art, and are disclosed in various references, including for example, "Remington: The Science and Practice of Pharmacy", A. Gennaro, ed., 20th edition, Lippincott, Williams & Wilkins, Philadelphia, PA.

The term "carrier" or "vehicle" as used interchangeably herein encompasses carriers, excipients, adjuvants, and diluents or a combination of any of the foregoing, meaning a material, composition or vehicle, such as a liquid or solid filler, diluent, excipient, solvent or encapsulating material involved in carrying or transporting a pharmaceutical agent from one organ, or portion of the body, to another organ or portion of the body. In addition to the adjuvants, excipients and diluents known to one skilled in the art, the carrier includes nanoparticles of organic and inorganic nature.

For example, in embodiments the present disclosure provides nanoparticle carriers (e.g., HDL-derived nanoparticles) as delivery vehicles for an active agent (e.g., a compound of formula (I), (IA), (IB), (II), (II-1), (II-2), (IIA), (IIA-1), (IIA-2) or Table 1). In embodiments, the agent is encapsulated within the nanoparticle carrier. In other embodiments, the agent is bound to the surface of the nanoparticle carrier. The association of the agent and the nanoparticle carrier may be effected by a variety of means, including noncovalent bonding, and trapping the agent in the interior of the delivery vehicle and the like. In embodiments, the association is sufficiently stable so that agent remains associated with the delivery vehicle until it is delivered to the target site in the treated subject.

The terms "pharmaceutical combination," "therapeutic combination" or "combination" as used herein, refers to a single dosage form comprising at least two therapeutically active agents, or separate dosage forms comprising at least two therapeutically active agents together or separately for use in combination therapy. Administration of a combination therapy includes: administration in the same or different composition(s) and/or combinations, either sequentially, simultaneously, or continuously, through the same or different routes. For example, one therapeutically active agent may be formulated into one dosage form and the other therapeutically active agent may be formulated into a single or different dosage forms. For example, one therapeutically active agent may be formulated into a solid oral dosage form whereas the second therapeutically active agent may be formulated into a solution dosage form for parenteral administration. In embodiments, the combination therapy optionally includes one or more pharmaceutically acceptable carriers or excipients, non-pharmaceutically active compounds, and/or inert substances.

As used herein, the phrase "a disorder characterized by cell proliferation" or "a condition characterized by cell proliferation" include, but are not limited to, cancer, benign and malignant tumors. Examples of cancer and tumors include, but are not limited to, cancers or tumor growth of the bladder, blood vessels, bone, brain, breast, cervix, chest, colon, endometrium, esophageus, eye, head, kidney, liver, lymph nodes, lung, mouth, neck, ovary, pancreas, prostate, rectum, colorectum, skin, stomach, testicles, throat, thyroid, urothelium, and uterus.

The terms "treat", "treating" or "treatment" in reference to a particular disease or disorder includes prevention of the disease or disorder, and/or lessening, improving, ameliorating or abrogating the symptoms and/or pathology of the disease or disorder. Generally the terms as used herein refer to ameliorating, alleviating, lessening, and removing symptoms of a disease or condition. A candidate compound described herein may be in a therapeutically effective amount in a formulation or medicament, which is an amount that can lead to a biological effect, such as apoptosis of certain cells (e.g., cancer cells), reduction of proliferation of certain cells, or lead to ameliorating, alleviating, lessening, or removing symptoms of a disease or condition, for example sepsis. The terms also can refer to reducing or stopping a cell proliferation rate (e.g., slowing or halting tumor growth) or reducing the number of proliferating cancer cells (e.g., removing part or all of a tumor).

The term "patient" or "subject" as used herein, includes all mammals and more particularly includes humans. The methods described herein may be useful for both human therapy and veterinary applications. In one embodiment, the subject is a human.

As used herein, "prevention" or "preventing" refers to a reduction of the risk of acquiring a given disease or disorder. For example, causing at least one of the clinical symptoms of the disease not to develop in a subject that may be exposed to or predisposed to the disease but does not yet experience or display symptoms of the disease.

As used herein, "therapeutically effective amount" means the amount of a compound or a therapeutically active agent that, when administered to a subject for treating a disease or other undesirable medical condition, is sufficient to have a beneficial effect with respect to that disease or condition. The therapeutically effective amount will vary depending on the type of the selected compound or a therapeutically active agent, the disease or condition and its severity, and the age, weight, etc. of the patient to be treated.

By "optional" or "optionally" it is meant that the subsequently described event or circumstance may or may not occur, and that the description includes instances where the event or circumstance occurs and instances in which is does not. For example, "optionally substituted aryl" encompasses both "aryl" and "substituted aryl" as defined below. It will be understood by those skilled in the art, with respect to any group containing one or more substituents, that such groups are not intended to introduce any substitution or substitution patterns that are sterically impractical, synthetically non-feasible and/or inherently unstable.

It is further noted that the claims may be drafted to exclude any optional element. As such, this statement is intended to serve as antecedent basis for use of such exclusive terminology as "solely", "only" and the like in connection with the recitation of claim elements, or the use of a "negative" limitation.

When a range of values is listed, it is intended to encompass each value and sub-range within the range. For example, "C₁-C₆ alkyl" is intended to encompass C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

The term "acyl" as used herein refers to R-C(O)- groups such as, but not limited to, (alkyl)-C(O)-, (alkenyl)-C(O)-, (alkynyl)-C(O)-, (aryl)-C(O)-, (cycloalkyl)-C(O)-, (heteroaryl)-C(O)-, and (heterocyclyl)-C(O)-, wherein the group is attached to the parent molecular structure through the carbonyl functionality. In embodiments, it is a C₁₋₁₀ acyl radical which refers to the total number of chain or ring atoms of the, for example, alkyl, alkenyl, alkynyl, aryl, cycloalkyl, or heteroaryl, portion plus the carbonyl carbon of acyl. For example, a C₄-acyl has three other ring or chain atoms plus carbonyl.

"Alkyl" or "alkyl group" refers to a fully saturated, straight or branched hydrocarbon chain. In embodiments, an alkyl group contains from one to thirty carbon atoms. In embodiments, an alkyl groups has from one to twelve carbon atoms, and which is attached to the rest of the molecule by a single bond. For example, alkyls comprising any number of carbon atoms from 1 to 12 are included. An alkyl comprising up to 12 carbon atoms is a C₁-C₁₂ alkyl, an alkyl comprising up to 10 carbon atoms is a C₁-C₁₀ alkyl, an alkyl comprising up to 6 carbon atoms is a C₁-C₆ alkyl and an alkyl comprising up to 5 carbon atoms is a C₁-C₅ alkyl. A C₁-C₅ alkyl includes C₅ alkyls, C₄ alkyls, C₃ alkyls, C₂ alkyls and C₁ alkyl (*i.e*., methyl). A C₁-C₆ alkyl includes all moieties described above for C₁-C₅ alkyls but also includes C₆ alkyls. A C₁-C₁₀ alkyl includes all moieties described above for C₁-C₅ alkyls and C₁-C₆ alkyls, but also includes C₇, C₈, C₉ and C₁₀ alkyls. Similarly, a C₁-C₁₂ alkyl includes all the foregoing moieties, but also includes C₁₁ and C₁₂ alkyls. Non-limiting examples of C₁-C₁₂ alkyl include methyl, ethyl, *n*-propyl, *i*-propyl, *sec*-propyl, *n*-butyl, *i*-butyl, *sec*-butyl, *t*-butyl, *n*-pentyl, *t*-amyl, *n*-hexyl, *n*-heptyl, *n*-octyl, *n*-nonyl, *n*-decyl, *n*-undecyl, and *n-*dodecyl. Unless stated otherwise specifically in the specification, an alkyl group can be optionally substituted. In embodiments, "alkyl" is a straight-chain hydrocarbon. In embodiments, "alkyl" is a branched hydrocarbon.

"Alkylene" or "alkylene chain" refers to a fully saturated, straight or branched divalent hydrocarbon chain. In embodiments, an alkylene groups has from one to twelve carbon atoms. Non-limiting examples of C₁-C₁₂ alkylene include methylene, ethylene, propylene, *n*-butylene, ethenylene, propenylene, *n*-butenylene and the like. The alkylene chain is attached to the rest of the molecule through a single bond and to the group through a single bond. The points of attachment of the alkylene chain to the rest of the molecule and to the group can be through one carbon or any two carbons within the chain. Unless stated otherwise specifically in the specification, an alkylene chain can be optionally substituted.

"Alkenyl" or "alkenyl group" refers to a straight or branched hydrocarbon chain. In embodiments, an alkenyl group contains from one to thirty carbon atoms. In embodiments, an alkenyl group contains from two to twelve carbon atoms, and having one or more carbon-carbon double bonds, such as a straight or branched group of 2-8 carbon atoms, referred to herein as C₂₋C₈ alkenyl. Each alkenyl group is attached to the rest of the molecule by a single bond. Alkenyl group comprising any number of carbon atoms from 2 to 12 are included. An alkenyl group comprising up to 12 carbon atoms is a C₂-C₁₂ alkenyl, an alkenyl comprising up to 10 carbon atoms is a C₂-C₁₀ alkenyl, an alkenyl group comprising up to 6 carbon atoms is a C₂-C₆ alkenyl and an alkenyl comprising up to 5 carbon atoms is a C₂-C₅ alkenyl. A C₂-C₅ alkenyl includes C₅ alkenyls, C₄ alkenyls, C₃ alkenyls, and C₂ alkenyls. A C₂-C₆ alkenyl includes all moieties described above for C₂-C₅ alkenyls but also includes C₆ alkenyls. A C₂-C₁₀ alkenyl includes all moieties described above for C₂-C₅ alkenyls and C₂-C₆ alkenyls, but also includes C₇, C₈, C₉ and C₁₀ alkenyls. Similarly, a C₂-C₁₂ alkenyl includes all the foregoing moieties, but also includes C₁₁ and C₁₂ alkenyls. Non-limiting examples of C₂-C₁₂ alkenyl include ethenyl (vinyl), 1-propenyl, 2-propenyl (allyl), iso-propenyl, 2-methyl-1-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-heptenyl, 2-heptenyl, 3-heptenyl, 4-heptenyl, 5-heptenyl, 6-heptenyl, 1-octenyl, 2-octenyl, 3-octenyl, 4-octenyl, 5-octenyl, 6-octenyl, 7-octenyl, 1-nonenyl, 2-nonenyl, 3-nonenyl, 4-nonenyl, 5-nonenyl, 6-nonenyl, 7-nonenyl, 8-nonenyl, 1-decenyl, 2-decenyl, 3-decenyl, 4-decenyl, 5-decenyl, 6-decenyl, 7-decenyl, 8-decenyl, 9-decenyl, 1-undecenyl, 2-undecenyl, 3-undecenyl, 4-undecenyl, 5-undecenyl, 6-undecenyl, 7-undecenyl, 8-undecenyl, 9-undecenyl, 10-undecenyl, 1-dodecenyl, 2-dodecenyl, 3-dodecenyl, 4-dodecenyl, 5-dodecenyl, 6-dodecenyl, 7-dodecenyl, 8-dodecenyl, 9-dodecenyl, 10-dodecenyl, and 11-dodecenyl. Unless stated otherwise specifically in the specification, an alkyl group can be optionally substituted.

"Alkynyl" or "alkynyl group" refers to a straight or branched hydrocarbon chain. In embodiments, an alkynyl group contains from one to thirty carbon atoms. In embodiments, an alkynyl group contains from two to twelve carbon atoms, and having one or more carbon-carbon triple bonds such as a straight or branched group of 2-8 carbon atoms, referred to herein as C₂-C₈ alkynyl.. Each alkynyl group is attached to the rest of the molecule by a single bond. Alkynyl group comprising any number of carbon atoms from 2 to 12 are included. An alkynyl group comprising up to 12 carbon atoms is a C₂-C₁₂ alkynyl, an alkynyl comprising up to 10 carbon atoms is a C₂-C₁₀ alkynyl, an alkynyl group comprising up to 6 carbon atoms is a C₂-C₆ alkynyl and an alkynyl comprising up to 5 carbon atoms is a C₂-C₅ alkynyl. A C₂-C₅ alkynyl includes C₅ alkynyls, C₄ alkynyls, C₃ alkynyls, and C₂ alkynyls. A C₂-C₆ alkynyl includes all moieties described above for C₂-C₅ alkynyls but also includes C₆ alkynyls. A C₂-C₁₀ alkynyl includes all moieties described above for C₂-C₅ alkynyls and C₂-C₆ alkynyls, but also includes C₇, C₈, C₉ and C₁₀ alkynyls. Similarly, a C₂-C₁₂ alkynyl includes all the foregoing moieties, but also includes C₁₁ and C₁₂ alkynyls. Non-limiting examples of C₂-C₁₂ alkenyl include ethynyl, propynyl, butynyl, pentynyl and the like. Unless stated otherwise specifically in the specification, an alkyl group can be optionally substituted.

"Aryl" refers to a hydrocarbon ring system comprising hydrogen, 6 to 18 carbon atoms and at least one aromatic ring, which is attached to the rest molecule by a single bond. For purposes of this invention, the aryl can be a monocyclic, bicyclic, tricyclic, tetracyclic ring system or other multicyclic ring system, which can include fused or bridged ring systems. Aryls include, but are not limited to, aryls derived from aceanthrylene, acenaphthylene, acephenanthrylene, anthracene, azulene, benzene, chrysene, fluoranthene, fluorene, as-indacene, s-indacene, indane, indene, naphthalene, phenalene, phenanthrene, pleiadene, pyrene, and triphenylene. Unless stated otherwise specifically in the specification, the aryl can be optionally substituted.

"Aralkyl" or "arylalkyl" refers to a group of the formula -R_{b}-R_{c} where R_{b} is an alkylene group as defined above and R_{c} is one or more aryls as defined above, for example, benzyl, diphenylmethyl and the like. Unless stated otherwise specifically in the specification, an aralkyl group can be optionally substituted.

"Carbocyclyl," "carbocyclic ring" or "carbocycle" refers to a rings structure, wherein the atoms which form the ring are each carbon, and which is attached to the rest of the molecule by a single bond. Carbocyclic rings can comprise from 3 to 20 carbon atoms in the ring. Carbocyclic rings include aryls and cycloalkyl, cycloalkenyl and cycloalkynyl as defined herein. Unless stated otherwise specifically in the specification, a carbocyclyl group can be optionally substituted.

"Cycloalkyl" refers to a stable non-aromatic monocyclic or polycyclic fully saturated hydrocarbon consisting solely of carbon and hydrogen atoms, which can include fused, spirocyclic, or bridged ring systems, having from three to twenty carbon atoms, and which is attached to the rest of the molecule by a single bond. Monocyclic cycloalkyl include, for example, cyclopropyl, cyclobutyl, cyclopentyl, cyclohexyl, cycloheptyl, and cyclooctyl. Polycyclic cycloalkyls include, for example, adamantyl, norbornyl, decalinyl, 7,7-dimethyl-bicyclo[2.2.1]heptanyl, and the like. Unless otherwise stated specifically in the specification, a cycloalkyl group can be optionally substituted.

"Cycloalkenyl" refers to a stable non-aromatic monocyclic or polycyclic hydrocarbon consisting solely of carbon and hydrogen atoms, having one or more carbon-carbon double bonds, which can include fused, spirocyclic, or bridged ring systems, having from three to twenty carbon atoms, preferably having from three to ten carbon atoms, and which is attached to the rest of the molecule by a single bond. Monocyclic cycloalkenyl include, for example, cyclopentenyl, cyclohexenyl, cycloheptenyl, cycloctenyl, and the like. Polycyclic cycloalkenyls include, for example, bicyclo[2.2.1]hept-2-enyl and the like. Unless otherwise stated specifically in the specification, a cycloalkenyl group can be optionally substituted.

"Cycloalkynyl" refers to a stable non-aromatic monocyclic or polycyclic hydrocarbon consisting solely of carbon and hydrogen atoms, having from 3 to 20 carbon atoms and one or more carbon-carbon triple bonds, which can include fused, spirocyclic, or bridged ring systems, and which is attached to the rest of the molecule by a single bond. Monocyclic cycloalkynyls include, for example, cycloheptynyl, cyclooctynyl, and the like. Unless otherwise stated specifically in the specification, a cycloalkynyl group can be optionally substituted.

"Heterocyclyl," "heterocyclic ring" or "heterocycle" refers to a stable 3- to 20-membered aromatic or non-aromatic ring which consists of 2 to 12 carbon atoms and from one to six heteroatoms selected from the group consisting of nitrogen, oxygen and sulfur. Heterocycles can be aromatic (heteroaryls) or non-aromatic. Unless stated otherwise specifically in the specification, the heterocyclyl can be a monocyclic, bicyclic, tricyclic, tetracyclic ring system or other multicyclic ring system, which can include fused, spirocyclic, or bridged ring systems; and the nitrogen, carbon or sulfur atoms in the heterocyclyl can be optionally oxidized; the nitrogen atom can be optionally quaternized; and the heterocyclyl can be partially or fully saturated. Examples of such heterocyclyls include, but are not limited to, dioxolanyl, thienyl[1,3]dithianyl, decahydroisoquinolyl, biotinyl, dihydrofuranyl, dihydroindolyl, dihydropyranyl, dihydrothienyl, dithiazolyl, homopiperidinyl, pyranyl, pyrazolinyl, thiopyranyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrrolidin-2-only, pyrazolidinyl, quinuclidinyl, thiazolidinyl, tetrahydrofuryl, tetrahydroisoquinolyl, trithianyl, tetrahydropyranyl, thiomorpholinyl, thiamorpholinyl, 1-oxo-thiomorpholinyl, and 1,1-dioxo-thiomorpholinyl. Unless stated otherwise specifically in the specification, a heterocyclyl group can be optionally substituted.

"Heteroaryl" refers to a 5- to 20-membered ring system comprising hydrogen atoms, one to thirteen carbon atoms, one to six heteroatoms selected from the group consisting of nitrogen, oxygen and sulfur, and at least one aromatic ring. For purposes of this disclosure, the heteroaryl can be a monocyclic, bicyclic, tricyclic or tetracyclic ring system, which can include fused or bridged ring systems; the heteroaryl may contain one or more non-aromatic rings (e.g., cycloalkyl or heterocyclyl) fused to the aromatic ring. The nitrogen, carbon or sulfur atoms in the heteroaryl can be optionally oxidized; the nitrogen atom can be optionally quaternized. Examples include, but are not limited to, azepinyl, acridinyl, benzimidazolyl, benzothiazolyl, benzindolyl, benzodioxolyl, benzofuranyl, benzooxazolyl, benzothiazolyl, benzothiadiazolyl, benzo[b][1,4]dioxepinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, carbazolyl, cinnolinyl, dibenzofuranyl, dibenzothiophenyl, furanyl, furanonyl, isothiazolyl, imidazolyl, indazolyl, indolyl, indazolyl, isoindolyl, indolinyl, isoindolinyl, isoquinolyl, indolizinyl, isoxazolyl, naphthyridinyl, oxadiazolyl, 2-oxoazepinyl, oxazolyl, oxiranyl, 1-oxidopyridinyl, 1-oxidopyrimidinyl, 1-oxidopyrazinyl, 1-oxidopyridazinyl, 1-phenyl-1*H*-pyrrolyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, tetrahydroquinolinyl, thiazolyl, thiadiazolyl, triazolyl, tetrazolyl, triazinyl, and thiophenyl (i.e. thienyl). Unless stated otherwise specifically in the specification, a heteroaryl group can be optionally substituted.

"Heteroarylalkyl" refers to a group of the formula -R_{b}-R_{f} where R_{b} is an alkylene chain as defined above and R_{f} is a heteroaryl as defined above. Unless stated otherwise specifically in the specification, a heteroarylalkyl group can be optionally substituted.

The term "substituted" used herein means any of the above groups (i.e., alkyl, alkenyl, alkynyl, aryl, arylalkyl, carbocyclyl, cycloalkyl, cycloalkenyl, cycloalkynyl, heterocyclyl, N-heterocyclyl, heteroaryl, etc) wherein at least one hydrogen atom is replaced by a bond to a non-hydrogen atoms such as, but not limited to: a halogen atom such as F, Cl, Br, and I; an oxygen atom in groups such as hydroxyl groups, alkoxy groups, and ester groups; a sulfur atom in groups such as thiol groups, thioalkyl groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as trialkylsilyl groups, dialkylarylsilyl groups, alkyldiarylsilyl groups, and triarylsilyl groups; and other heteroatoms in various other groups. "Substituted" also means any of the above groups in which one or more hydrogen atoms are replaced by a higher-order bond (e.g., a double- or triple-bond) to a heteroatom such as oxygen in oxo, carbonyl, carboxyl, and ester groups; and nitrogen in groups such as imines, oximes, hydrazones, and nitriles. For example, "substituted" includes any of the above groups in which one or more hydrogen atoms are replaced with NR_{g}Rₕ, NR_{g}C(=O)Rₕ, NR_{g}C(=O)NR_{g}Rₕ, NR_{g}C(=O)ORₕ, NR_{g}SO2Rₕ, OC(=O)NR_{g}Rₕ, OR_{g}, SR_{g}, SOR_{g}, SO₂R_{g}, OSO₂R_{g}, SO₂OR_{g}, =NSO₂R_{g}, and SO₂NR_{g}Rₕ. "Substituted" also means any of the above groups in which one or more hydrogen atoms are replaced with C(=O)R_{g}, C(=O)OR_{g}, C(=O)NR_{g}Rₕ, CH₂SO₂R_{g}, CH₂SO₂NR_{g}Rₕ. In the foregoing, R_{g} and Rₕ are the same or different and independently hydrogen, alkyl, alkenyl, alkynyl, alkoxy, alkylamino, thioalkyl, aryl, aralkyl, cycloalkyl, cycloalkenyl, cycloalkynyl, cycloalkylalkyl, haloalkyl, haloalkenyl, haloalkynyl, heterocyclyl, N-heterocyclyl, heterocyclylalkyl, heteroaryl, N-heteroaryl and/or heteroarylalkyl. "Substituted" further means any of the above groups in which one or more hydrogen atoms are replaced by a bond to an amino, cyano, hydroxyl, imino, nitro, oxo, thioxo, halo, alkyl, alkenyl, alkynyl, alkoxy, alkylamino, thioalkyl, aryl, aralkyl, cycloalkyl, cycloalkenyl, cycloalkynyl, cycloalkylalkyl, haloalkyl, haloalkenyl, haloalkynyl, heterocyclyl, N-heterocyclyl, heterocyclylalkyl, heteroaryl, N-heteroaryl and/or heteroarylalkyl group. In addition, "substituted" means any of the above groups in which two hydrogen atoms are each replaced by a bond to form a fused ring system containing the atoms to which the hydrogens were attached. Moreover, each of the foregoing substituents can also be optionally substituted with one or more of the above substituents.

The compounds of the disclosure may contain one or more chiral centers and/or double bonds and, therefore, exist as stereoisomers, such as geometric isomers, enantiomers or diastereomers. The term "stereoisomers" when used herein consist of all geometric isomers, enantiomers or diastereomers. These compounds may be designated by the symbols "R" or "S," depending on the configuration of substituents around the stereogenic carbon atom. The present disclosure encompasses various stereoisomers of these compounds and mixtures thereof. Stereoisomers include enantiomers and diastereomers. Mixtures of enantiomers or diastereomers may be designated "(±)" in nomenclature, but the skilled artisan will recognize that a structure may denote a chiral center implicitly. In embodiments, an enantiomer or stereoisomer may be provided substantially free of the corresponding enantiomer.

In embodiments, the compound is a racemic mixture of (S)- and (R)-isomers. In other embodiments, provided herein is a mixture of compounds wherein individual compounds of the mixture exist predominately in an (S)- or (R)-isomeric configuration. For example, the compound mixture has an (S)-enantiomeric excess of greater than about 55%, about 60%, about 65%, about 70%, about 75%, about 80%, about 85%, about 90%, about 95%, about 96%, about 97%, about 98%, about 99%, about 99.5%, or more. In other embodiments, the compound mixture has an (S)-enantiomeric excess of greater than about 55% to about 99.5%, greater than about 60% to about 99.5%, greater than about 65% to about 99.5%, greater than about 70% to about 99.5%, greater than about 75% to about 99.5%, greater than about 80% to about 99.5%, greater than about 85% to about 99.5%, greater than about 90% to about 99.5%, greater than about 95% to about 99.5%, greater than about 96% to about 99.5%, greater than about 97% to about 99.5%, greater than about 98% to greater than about 99.5%, greater than about 99% to about 99.5%, or more. In other embodiments, the compound mixture has an (R)-enantiomeric purity of greater than about 55%, about 60%, about 65%, about 70%, about 75%, about 80%, about 85%, about 90%, about 95%, about 96%, about 97%, about 98%, about 99%, about 99.5% or more. In some other embodiments, the compound mixture has an (R)-enantiomeric excess of greater than about 55% to about 99.5%, greater than about 60% to about 99.5%, greater than about 65% to about 99.5%, greater than about 70% to about 99.5%, greater than about 75% to about 99.5%, greater than about 80% to about 99.5%, greater than about 85% to about 99.5%, greater than about 90% to about 99.5%, greater than about 95% to about 99.5%, greater than about 96% to about 99.5%, greater than about 97% to about 99.5%, greater than about 98% to greater than about 99.5%, greater than about 99% to about 99.5% or more.

Individual stereoisomers of compounds of the present disclosure can be prepared synthetically from commercially available starting materials that contain asymmetric or stereogenic centers, or by preparation of racemic mixtures followed by resolution methods well known to those of ordinary skill in the art. These methods of resolution are exemplified by: (1) attachment of a mixture of enantiomers to a chiral auxiliary, separation of the resulting mixture of diastereomers by recrystallization or chromatography and liberation of the optically pure product from the auxiliary; (2) salt formation employing an optically active resolving agent; or (3) direct separation of the mixture of optical enantiomers on chiral chromatographic columns. Stereoisomeric mixtures can also be resolved into their component stereoisomers by well-known methods, such as chiral-phase gas chromatography, chiral-phase high performance liquid chromatography, crystallizing the compound as a chiral salt complex, or crystallizing the compound in a chiral solvent. Stereoisomers can also be obtained from stereomerically-pure intermediates, reagents, and catalysts by well-known asymmetric synthetic methods.

Geometric isomers can also exist in the compounds of the present disclosure. The present disclosure encompasses the various geometric isomers and mixtures thereof resulting from the arrangement of substituents around a carbon-carbon double bond or arrangement of substituents around a carbocyclic ring. Substituents around a carbon-carbon double bond are designated as being in the *"Z"* or "*E*" configuration wherein the terms "*Z*" and "*E*" are used in accordance with IUPAC standards. Unless otherwise specified, structures depicting double bonds encompass both the *E* and *Z* isomers.

Substituents around a carbon-carbon double bond alternatively can be referred to as *"cis"* or *"trans,"* where *"cis"* represents substituents on the same side of the double bond and "trans" represents substituents on opposite sides of the double bond. The arrangements of substituents around a carbocyclic ring are designated as *"cis"* or *"trans."* The term *"cis"* represents substituents on the same side of the plane of the ring and the term *"trans"* represents substituents on opposite sides of the plane of the ring. Mixtures of compounds wherein the substituents are disposed on both the same and opposite sides of plane of the ring are designated *"cis*/*trans."*

The compounds disclosed herein may exist as tautomers and both tautomeric forms are intended to be encompassed by the scope of the present disclosure, even though only one tautomeric structure is depicted.

As used herein, the term "isotopic variant" is meant to include compounds that differ only in the presence of one or more isotopically enriched atoms. Such compounds may be useful as, for example, analytical tools, probes in biological assays, or therapeutic agents. For example, an "isotopic variant" of a compound can contain one or more nonradioactive isotopes, such as for example, deuterium (²H or D), carbon-13 (¹³C), nitrogen-15 (¹⁵N), or the like. It will be understood that, in a compound where such isotopic substitution is made, the following atoms, where present, may vary, so that for example, any hydrogen may be ²H/D, any carbon may be ¹³C, or any nitrogen may be ¹⁵N, and that the presence and placement of such atoms may be determined within the skill of the art. Likewise, the invention may include the preparation of isotopic variants with radioisotopes, in the instance for example, where the resulting compounds may be used for drug and/or substrate tissue distribution studies. The radioactive isotopes tritium, i.e. ³H, and carbon-14, i.e. ¹⁴C, are particularly useful for this purpose in view of their ease of incorporation and ready means of detection. Further, compounds may be prepared that are substituted with positron emitting isotopes, such as ¹¹C, ¹⁸F, ¹⁵O and ¹³N, and would be useful in Positron Emission Topography (PET) studies for examining substrate receptor occupancy.

The term "triglyceride" as used herein means an ester derived from glycerol and three fatty acids. The fatty acids may be the same or different. The notation used in this specification to describe a triglyceride is the same as that used below to describe a fatty acid. Fatty acids can attach to the glycerol molecule in any order, e.g., any fatty acid can react with any of the hydroxyl groups of the glycerol molecule for forming an ester linkage. For example. In a non-limiting example, a triglyceride can comprise glycerol with any combination of the following fatty acids: C18:1, C14:1, C16: 1, polyunsaturated, and saturated. A triglyceride of C18:1 fatty acid simply means that the fatty acid components of the triglyceride are derived from or based upon a C18:1 fatty acid. That is, a C18:1 triglyceride is an ester of glycerol and three fatty acids of 18 carbon atoms each with each fatty acid having one double bond. Similarly, a C14:1 triglyceride is an ester of glycerol and three fatty acids of 14 carbon atoms each with each fatty acid having one double bond. Likewise, a C16:1 triglyceride is an ester of glycerol and three fatty acids of 16 carbon atoms each with each fatty acid having one double bond. Triglycerides of C18:1 fatty acids in combination with C14:1 and/or C16:1 fatty acids means that: (a) a C18:1 triglyceride is mixed with a C14:1 triglyceride or a C16: 1 triglyceride or both; or (b) at least one of the fatty acid components of the triglyceride is derived from or based upon a C18:1 fatty acid, while the other two are derived from or based upon C14:1 fatty acid and/or C16:1 fatty acid.

The term "fatty acid" and like terms mean a carboxylic acid with a long aliphatic tail that is either saturated or unsaturated. The term "long aliphatic tail" and "fatty acid chain" are used interchangeably herein. Fatty acids and fatty acid chains may be esterified to phospholipids and triglycerides. As used herein, the fatty acid chain length includes from C4 to C30 (e.g., C6 to C30), saturated or unsaturated, cis or trans, unsubstituted or substituted, branched or unbranched hydrocarbon chain (e.g., the fatty acid chain length includes from C4 to C30 (e.g., C6 to C30), saturated or unsaturated, cis or trans, unsubstituted or substituted with 1-6 side chains). For example, in embodiments, examples of a fatty acid chain include, but are not limited to, C4, C5, C6, C7, C8, C9, C10, C11, C12, C13, C14, C15, C16, C17, C18, C19, C20, C21, C22, C23, C24, C25, C26, C27, C28, C29, or C30 saturated or unsaturated, cis or trans, unsubstituted or substituted hydrocarbon chain. Unsaturated fatty acids and fatty acid chains have one or more double bonds between carbon atoms. Saturated fatty acids and fatty acid chains do not contain any double bonds. In embodiments, a fatty acid may be described herein by the capital letter "C" for carbon atom, followed by a number describing the number of carbon atoms in the fatty acid, followed by a colon and another number for the number of double bonds in the fatty acid. For example, C16:1 denotes a fatty acid of 16 carbon atoms with one double bond, e.g., palmitoleic acid. The number after the colon in this notation neither designates the placement of the double bond(s) in the fatty acid nor whether the hydrogen atoms bonded to the carbon atoms of the double bond are cis to one another. Other examples of this notation include C18:0 (stearic acid), C18:1 (oleic acid), C18:2 (linoleic acid), C18:3 (a- linolenic acid) and C20:4 (arachidonic acid).

The term "sterols" such as, but not limited to cholesterol, can also be utilized in the methods and compounds described herein. Sterols are animal or vegetable steroids which only contain a hydroxyl group but no other functional groups at C-3. In general, sterols contain 27 to 30 carbon atoms and one double bond in the 5/6 position and occasionally in the 7 /8, 8/9 or other positions. Besides these unsaturated species, other sterols are the saturated compounds obtainable by hydrogenation. One example of a suitable animal sterol is cholesterol. Typical examples of suitable phytosterols, which are preferred from the applicational point of view, are ergosterols, campesterols, stigmasterols, brassicasterols and, preferably, sitosterols or sitostanols and, more particularly, β-sitosterols or β-sitostanols. Besides the phytosterols mentioned, their esters are preferably used. The acid component of the ester may go back to carboxylic acids corresponding to formula (CA-I): RI CO-OH (CA-I); in which RI CO is an aliphatic, linear or branched acyl group containing 2 to 30 carbon atoms and O and/or 1, 2 or 3 double bonds. Typical examples are acetic acid, propionic acid, hexanoic acid, butyric acid, valeric acid, caproic acid, caprylic acid, 2-ethyl hexanoic acid, capric acid, cyclopentanepropionic acid, lauric acid, isotridecanoic acid, myristic acid, palmitic acid, palmitoleic acid, stearic acid, isostearic acid, oleic acid, elaidic acid, petroselic acid, linoleic acid, conjugated linoleic acid (CLA), linolenic acid, elaeosteric add, arachic acid, gadoleic acid, behenic acid and erucic acid.

The term "phospholipid" refers to an amphiphilic compound that consists of two hydrophobic fatty acid "tails" and a hydrophilic "head" consisting of a phosphate group. The two components are joined together by a glycerol molecule. The phosphate groups can be modified with simple organic molecules such as choline, ethanolamine or serine. Choline refers to an essential, bioactive nutrient having the chemical formula R-(CH₂)₂-N(CH₂)₄. When a phospho-moiety is R- it is called phosphocholine.

"Lysolipids", as used herein, include (acyl-, single chain) such as in non-limiting embodiments 1-myristoyl-2-hydroxy-sn-glycero-3-phosphocholine (MHPC), 1-palmitoyl-2-hydroxy-*sn*-glycero-3-phosphocholine (PHPC) and 1-stearoyl-2-hydroxy-sn-glycero-3-phosphocholine (SHPC).

The term "apolipoprotein A-I" or "apoA-I", and also "apoliprotein Al" or "apoAl", refers to a protein that is encoded by the APOAI gene in humans.

### DETAILED DESCRIPTION

Conventionally, immune systems in vertebrate animals are subdivided into two parts. The first part, innate immunity, provides an initial response to an infection within minutes to hours. Its cellular component comprises natural killer (NK) cells, innate lymphoid cells (ILCs) and phagocytes such as monocytes, macrophages and neutrophils. The innate immune system acts as a rapid first line of defense, triggered through recognition of either pathogens or endogenous danger signals by pattern recognition receptors (PRRs). Upon detecting pathogen-associated molecular patterns (PAMPs), PRRs initiate an innate immune response, which involves activating the subsequent adaptive immune system by antigen presentation, co-stimulation, and cytokine excretion. In addition, PRRs also recognize damage-associated molecular patterns (DAMPs), leading to non-infectious inflammatory responses. The second stage of the response to infection involves the immune system's second part - the adaptive response - in which T and B lymphocytes specifically recognize a pathogen, proliferate and become activated against that pathogen. These cells also build immunological memory of that specific infection. The specificity of the adaptive immune system response is mediated by recombination of the immunoglobulin genes at the lymphocyte level. Immunological memory results in a quicker and quantitatively better immune response (as compared with the primary response alone) against a previously encountered antigen. Though the innate immune system was long believed to lack memory, recent studies show that innate immune cells undergo metabolic and epigenetic rewiring, adjusting their functional programs in a process termed 'trained immunity' that is considered de facto innate immune memory.

Trained Immunity is defined by a secondary long-term hyper-responsiveness, as manifested by increased cytokine excretion caused by metabolic and epigenetic rewiring, to restimulation after a primary insult of myeloid cells and their progenitors and stem cells in the bone marrow, spleen and blood. Trained Immunity (also called innate immune memory) is also defined by a long-term increased responsiveness (e.g. high cytokine production) after restimulation with a secondary stimulus of myeloid innate immune cells, being induced by a primary insult stimulating these cells or their progenitors and stem cells in the bone marrow and spleen, and mediated by epigenetic, metabolic and transcriptional rewiring.

Trained immunity is regulated and maintained through induction of training properties to progenitor cells in the bone marrow, resulting in durable reprogramming that exceeds the myeloid cell lifespan in the bloodstream. Although trained immunity can be induced with a range of 'training agents' in cultured myeloid cells, its systemic induction requires bone marrow progenitor cell engagement.

In one aspect, the present disclosure provides compounds (e.g., of formula (I), (IA), (IB), (II), (II-1), (II-2), (IIA), (IIA-1), (IIA-2) or Table 1) that activate nucleotide-binding oligomerization domain-containing protein 2 (NOD2). The present disclosure also provides nanobiologic compositions comprising a nanoparticle carrier (e.g., HDL-derived nanoparticle) comprising a compound of the present disclosure (e.g., of formula (I), (IA), (IB), (II), (II-1), (II-2), (IIA), (IIA-1), (IIA-2) or Table 1). Nanobiologic compositions of the present disclosure comprising compounds of the present disclosure (e.g., of formula (I), (IA), (IB), (II), (II-1), (II-2), (IIA), (IIA-1), (IIA-2) or Table 1) that activate nucleotide-binding oligomerization domain-containing protein 2 (NOD2) are designed to exhibit bone marrow proclivity. These nanomaterials can be administered (e.g., intravenously) to promote trained immunity. Therapeutically inducing trained immunity may find use, for example, in overcoming immunoparalysis in sepsis and infections, in treating cell proliferation disorders (such as cancer), and augmenting immune responses.

### Compounds

In embodiments, the present disclosure provides a compound of formula (I): 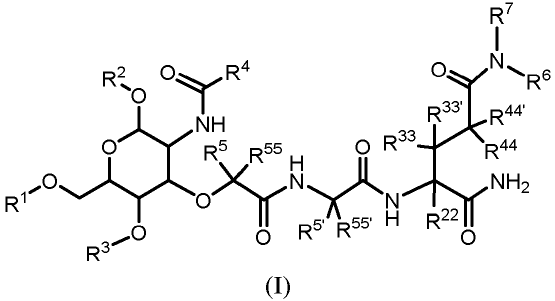 or a pharmaceutically acceptable salt thereof,
wherein:
R¹ is -H or -C(O)-R^{X};
R² and R³ are each independently selected from the group consisting of -H, alkyl, alkylene-aryl, -C(O)-alkyl, and -C(O)-aryl;
R⁴, R⁵, and R^{5'} are each alkyl;
R⁶ and R¹¹ are each independently -H, or alkyl;
R⁷ is triazolyl-L;
Y is alkylene;
L is selected from the group consisting of a fatty acid chain, -alkylene-C(O)-W, - alkylene-O-C(O)-W, -alkylene-N-(alkylene-C(O)-NR¹¹-alkylene-NR¹¹-C(O)-W)₂, and - alkylene-N-(alkylene-C(O)-W)₂;
W is a fatty acid chain, -O-alkylene-C(H)(OR⁸)-alkylene-OR⁹, a phospholipid, or a sterol;
R⁸ and R⁹ are each independently R^{X} or -C(O)-R^{X};
R²², R³³, R^{33'}, R⁴⁴, R^{44'}, R⁵⁵, and R^{55'} are each independently H or R^{A};
R^{X} is a fatty acid chain;
wherein each aforementioned alkyl, alkylene, alkylene-aryl, aryl, and triazolyl is optionally substituted with one or more R^{A}, wherein R^{A} is independently selected for each occurrence from the group consisting of hydrogen, halo, alkoxy, haloalkoxy, cyano, hydroxyl, -N(R^{C})(R^{D}), -C(O)N(R^{C})(R^{D}), -N(R^{C})C(O)R^{B}, -OC(O)NR^{C}R^{D}, -NR^{C}C(O)OR^{B}, -OC(O)R^{B}, -C(O)OR^{B}, -C(O)R^{B} , -CO₂H, -NO₂, -SH, S(O)_{X}R^{B}, aryl, heteroaryl, heteroarylalkyl, cycloalkyl, heterocycloalkyl, and R^{B}; wherein X is 0, 1, or 2;
R^{C} and R^{D} are independently selected for each occurrence from the group consisting of hydrogen, alkyl, haloalkyl -C(O)R^{B}, and -C(O)OR^{B}; or R^{C} and R^{D} are taken together with the nitrogen to which they are attached to form a heterocyclic ring optionally substituted with R^{A}; and
R^{B} is alkyl, alkenyl, or alkynyl optionally substituted with one or more fluoro.

In embodiments, the present disclosure provides a compound of formula (I): 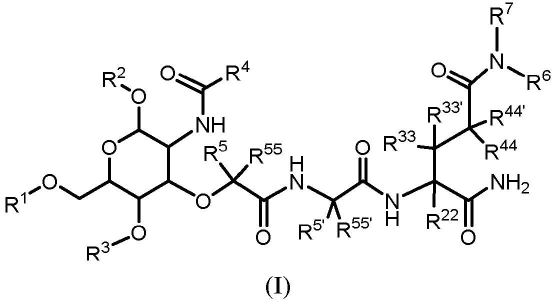 or a pharmaceutically acceptable salt thereof,
wherein:
R¹ is -H or -C(O)-R^{X};
R² and R³ are each independently selected from the group consisting of -H, alkyl, alkylene-aryl, -C(O)-alkyl, and -C(O)-aryl;
R⁴, R⁵, and R^{5'} are each alkyl;
R⁶ and R¹¹ are each independently -H, or alkyl;
R⁷ is -Y-triazolyl-L;
Y is alkylene;
L is selected from the group consisting of a fatty acid chain, -alkylene-C(O)-W, - alkylene-O-C(O)-W, -alkylene-N-(alkylene-C(O)-NR¹¹-alkylene-NR¹¹-C(O)-W)₂, and - alkylene-N-(alkylene-C(O)-W)₂;
W is a fatty acid chain, -O-alkylene-C(H)(OR⁸)-alkylene-OR⁹, a phospholipid, or a sterol;
R⁸ and R⁹ are each independently R^{X} or -C(O)-R^{X};
R²², R³³, R^{33'}, R⁴⁴, R^{44'}, R⁵⁵, and R^{55'} are each independently H or R^{A};
R^{X} is a fatty acid chain;
wherein each aforementioned alkyl, alkylene, alkylene-aryl, aryl, and triazolyl is optionally substituted with one or more R^{A}, wherein R^{A} is independently selected for each occurrence from the group consisting of hydrogen, halo, alkoxy, haloalkoxy, cyano, hydroxyl, -N(R^{C})(R^{D}), -C(O)N(R^{C})(R^{D}), -N(R^{C})C(O)R^{B}, -OC(O)NR^{C}R^{D}, -NR^{C}C(O)OR^{B}, -OC(O)R^{B}, -C(O)OR^{B}, -C(O)R^{B} , -CO₂H, -NO₂, -SH, S(O)_{X}R^{B},
aryl, arylalkyl, heteroaryl, heteroarylalkyl, cycloalkyl, heterocycloalkyl, and R^{B}; wherein X is 0,1 , or 2;
R^{C} and R^{D} are independently selected for each occurrence from the group consisting of hydrogen, alkyl, haloalkyl -C(O)R^{B}, and -C(O)OR^{B}; or R^{C} and R^{D} are taken together with the nitrogen to which they are attached to form a heterocyclic ring optionally substituted with R^{A}; and
R^{B} is alkyl, alkenyl, or alkynyl optionally substituted with one or more fluoro.

In embodiments of the compound of Formula (I), the compound is of formula (IA): 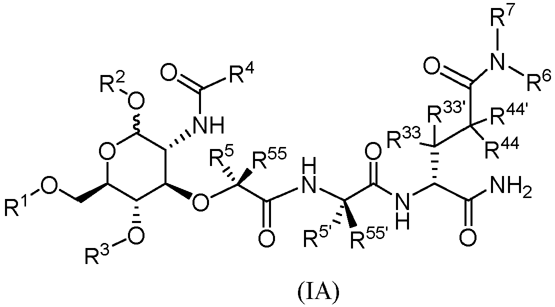 or a pharmaceutically acceptable salt thereof,
wherein:
R¹ is -H or -C(O)-R^{X};
R² and R³ are each independently selected from the group consisting of -H, alkyl, alkylene-aryl, -C(O)-alkyl, and -C(O)-aryl;
R⁴, R⁵, and R^{5'} are each alkyl;
R⁶ and R¹¹ are each independently -H, or alkyl;
R⁷ is -Y-triazolyl-L;
Y is alkylene;
L is selected from the group consisting of a fatty acid chain, -alkylene-C(O)-W, - alkylene-O-C(O)-W, -alkylene-N-(alkylene-C(O)-NR¹¹-alkylene-NR¹¹-C(O)-W)₂, and - alkylene-N-(alkylene-C(O)-W)₂;
W is a fatty acid chain, -O-alkylene-C(H)(OR⁸)-alkylene-OR⁹, a phospholipid, or a sterol;
R⁸ and R⁹ are each independently R^{X} or -C(O)-R^{X};
R²², R³³, R^{33'}, R⁴⁴, R^{44'}, R⁵⁵, and R^{55'} are each independently H or R^{A};
R^{X} is a fatty acid chain;
wherein each aforementioned alkyl, alkylene, alkylene-aryl, aryl, and triazolyl is optionally substituted with one or more R^{A}, wherein R^{A} is independently selected for each occurrence from the group consisting of hydrogen, halo, alkoxy, haloalkoxy, cyano, hydroxyl, -N(R^{C})(R^{D}), -C(O)N(R^{C})(R^{D}), -N(R^{C})C(O)R^{B}, -OC(O)NR^{C}R^{D}, -NR^{C}C(O)OR^{B}, -OC(O)R^{B}, -C(O)OR^{B}, -C(O)R^{B} , -CO₂H, -NO₂, -SH, S(O)_{X}R^{B} aryl, arylalkyl, heteroaryl, heteroarylalkyl, cycloalkyl, heterocycloalkyl, and R^{B} wherein X is 0,1 , or 2;
R^{C} and R^{D} are independently selected for each occurrence from the group consisting of hydrogen, alkyl, haloalkyl -C(O)R^{B}, and -C(O)OR^{B}; or R^{C} and R^{D} are taken together with the nitrogen to which they are attached to form a heterocyclic ring optionally substituted with R^{A}; and
R^{B} is alkyl, alkenyl, or alkynyl optionally substituted with one or more fluoro.

In embodiments, the compound of formula (I) is a compound of formula (IB): 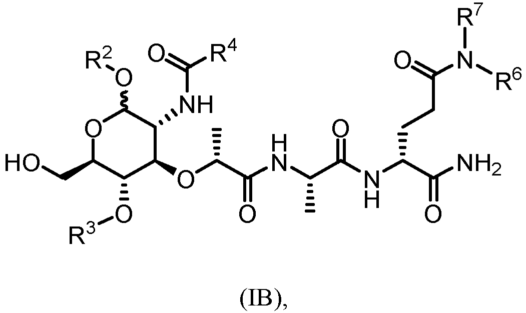 or a pharmaceutically acceptable salt thereof.

In the compounds of formula (I), (IA), or (IB), R⁷ is -Y-triazolyl-L.

In embodiments of the compounds of formula (I), (IA), or (IB), R^{A} is independently selected for each occurrence from the group consisting of hydrogen, halo, alkoxy, haloalkoxy, cyano, hydroxyl, -N(R^{C})(R^{D}), -C(O)N(R^{C})(R^{D}), -N(R^{C})C(O)R^{B}, -OC(O)NR^{C}R^{D}, -NR^{C}C(O)OR^{B}, -OC(O)R^{B}, -C(O)OR^{B}, -C(O)R^{B} , -CO₂H, -NO₂, -SH, S(O)_{X}R^{B} (wherein X is 0, 1, or 2), aryl, arylalkyl, heteroaryl, heteroarylalkyl, cycloalkyl, heterocycloalkyl.

In embodiments of the compounds of formula (I), (IA), or (IB), R^{A} is independently selected for each occurrence from the group consisting of halo, alkoxy, haloalkoxy, cyano, hydroxyl, -N(R^{C})(R^{D}), -C(O)N(R^{C})(R^{D}), -N(R^{C})C(O)R^{B}, -OC(O)NR^{C}R^{D}, -NR^{C}C(O)OR^{B}, - OC(O)R^{B}, -C(O)OR^{B}, -C(O)R^{B} , -CO₂H, -NO₂, -SH, S(O)_{X}R^{B} (wherein X is 0, 1, or 2), aryl, arylalkyl, heteroaryl, heteroarylalkyl, cycloalkyl, heterocycloalkyl.

In embodiments the compound of formula (I) is a compound of formula (II): 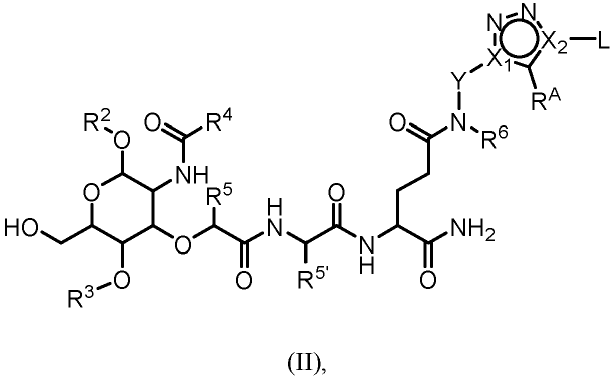 or a pharmaceutically acceptable salt thereof wherein:
X₁ is -N- and X₂ is -C-; or X₁ is -C- and X₂ is -N-;
R² and R³ are each independently selected from the group consisting of -H, alkyl, aryl, alkylene-aryl, -C(O)-alkyl, and -C(O)-aryl;
R⁴, R⁵, and R^{5'} are each alkyl;
R⁶ and R¹¹ are each independently -H, or alkyl;
Y is alkylene;
L is selected from the group consisting of a fatty acid chain, -alkylene-C(O)-W, - alkylene-O-C(O)-W, -alkylene-N-(alkylene-C(O)-NR¹¹-alkylene-NR¹¹-C(O)-W)₂, and - alkylene-N-(alkylene-C(O)-W)₂;
W is a fatty acid chain, -O-alkylene-C(H)(OR⁸)-alkylene-OR⁹, a phospholipid, or a sterol;
R⁸ and R⁹ are each independently R^{X} or -C(O)-R^{X};
R^{X} is a fatty acid chain;
wherein each aforementioned alkyl, alkylene, alkylene-aryl, and aryl is optionally substituted with one or more R^{A};
R^{A} is independently selected for each occurrence from the group consisting of hydrogen, halo, alkoxy, haloalkoxy, cyano, hydroxyl, -N(R^{C})(R^{D}), -C(O)N(R^{C})(R^{D}), -N(R^{C})C(O)R^{B}, - OC(O)NR^{C}R^{D}, -NR^{C}C(O)OR^{B}, -OC(O)R^{B}, -C(O)OR^{B}, -C(O)R^{B} , -CO₂H, -NO₂, -SH, S(O)_{X}R^{B} (wherein X is 0, 1, or 2), aryl, arylalkyl, heteroaryl, heteroarylalkyl, cycloalkyl, heterocycloalkyl, and R^{B};
R^{C} and R^{D} are independently selected for each occurrence from the group consisting of hydrogen, alkyl, haloalkyl -C(O)R^{B}, and -C(O)OR^{B}; or R^{C} and R^{D} are taken together with the nitrogen to which they are attached to form a heterocyclic ring optionally substituted with R^{A}; and
R^{B} is alkyl, alkenyl, or alkynyl optionally substituted with one or more fluoro.

In embodiments, the compound of formula (II) is:

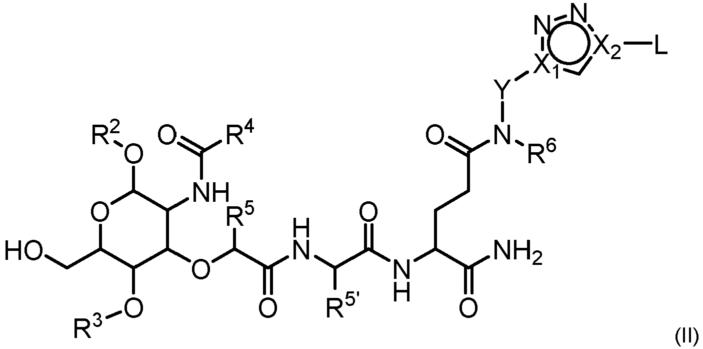

In embodiments, the compound of formula (II) is a compound of formula (II-1), or a pharmaceutically acceptable salt thereof

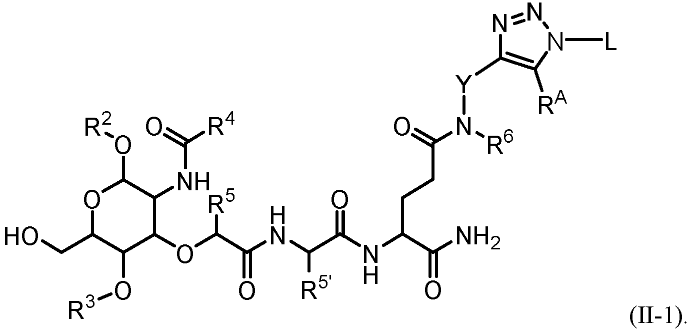

In embodiments, the compound of formula (II-1), is 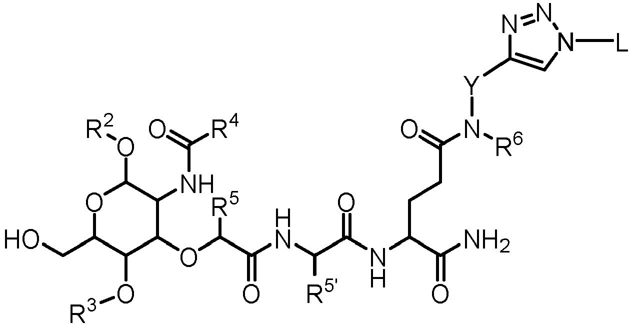 or a pharmaceutically acceptable salt thereof.

In embodiments, the compound of formula (II) is a compound of formula (II-2):

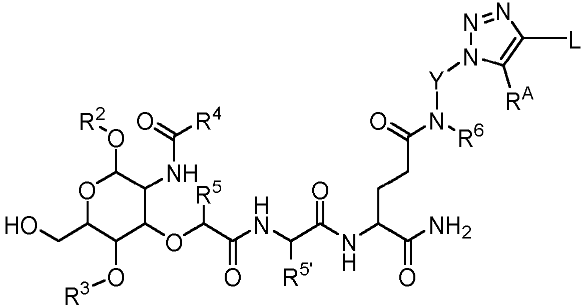

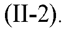

In embodiments, the compound of formula (II-2) is: 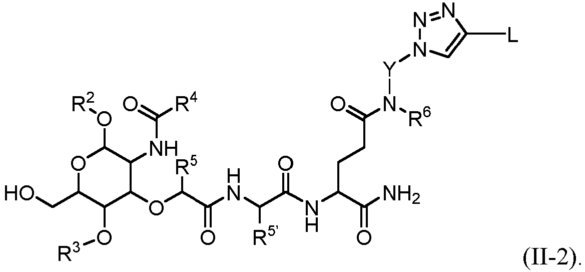 or a pharmaceutically acceptable salt thereof.

In embodiments the compound of formula (I) is a compound of formula (IIA): 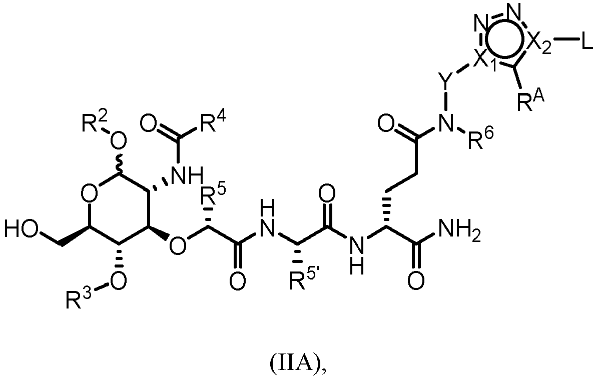 or a pharmaceutically acceptable salt thereof wherein:
X₁ is -N- and X₂ is -C-; or X₁ is -C- and X₂ is -N-
R² and R³ are each independently selected from the group consisting of -H, alkyl, aryl, alkylene-aryl, -C(O)-alkyl, and -C(O)-aryl;
R⁴, R⁵, and R^{5'} are each alkyl;
R⁶ and R¹¹ are each independently -H, or alkyl;
Y is alkylene;
L is selected from the group consisting of a fatty acid chain, -alkylene-C(O)-W, - alkylene-O-C(O)-W, -alkylene-N-(alkylene-C(O)-NR¹¹-alkylene-NR¹¹-C(O)-W)₂, and - alkylene-N-(alkylene-C(O)-W)₂;
W is a fatty acid chain, -O-alkylene-C(H)(OR⁸)-alkylene-OR⁹, a phospholipid, or a sterol;
R⁸ and R⁹ are each independently R^{X} or -C(O)-R^{X};
R^{X} is a fatty acid chain;
wherein each aforementioned alkyl, alkylene, alkylene-aryl, and aryl is optionally substituted with one or more R^{A};
R^{A} is independently selected for each occurrence from the group consisting of hydrogen, halo, alkoxy, haloalkoxy, cyano, hydroxyl, -N(R^{C})(R^{D}), -C(O)N(R^{C})(R^{D}), -N(R^{C})C(O)R^{B}, - OC(O)NR^{C}R^{D}, -NR^{C}C(O)OR^{B}, -OC(O)R^{B}, -C(O)OR^{B}, -C(O)R^{B} , -CO₂H, -NO₂, -SH, S(O)_{X}R^{B} (wherein X is 0, 1, or 2), aryl, arylalkyl, heteroaryl, heteroarylalkyl, cycloalkyl, heterocycloalkyl, and R^{B};
R^{C} and R^{D} are independently selected for each occurrence from the group consisting of hydrogen, alkyl, haloalkyl -C(O)R^{B}, and -C(O)OR^{B}; or R^{C} and R^{D} are taken together with the nitrogen to which they are attached to form a heterocyclic ring optionally substituted with R^{A}; and
R^{B} is alkyl, alkenyl, or alkynyl optionally substituted with one or more fluoro.

In embodiments, the compound of formula (IIA) is:

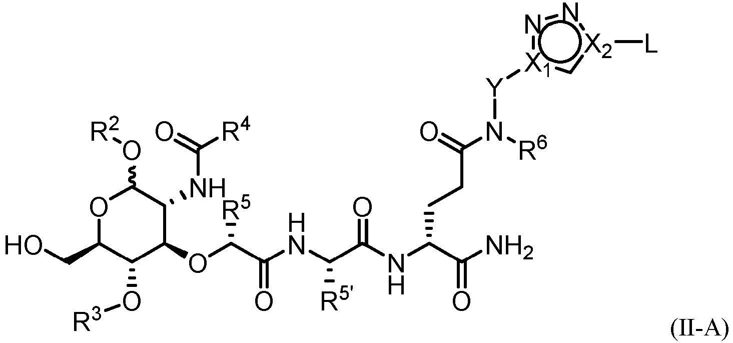

In embodiments the compound of formula (IIA) is a compound of formula (IIA-1), or a pharmaceutically acceptable salt thereof:

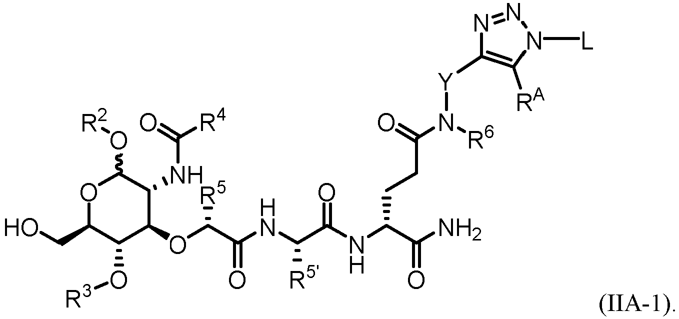

In embodiments, the compound of formula (IIA-1) is: 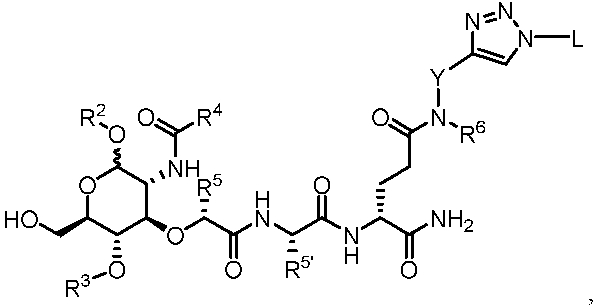 or a pharmaceutically acceptable salt thereof.

In embodiments the compound of formula (IIA) is a compound of formula (IIA-2), or a pharmaceutically acceptable salt thereof:

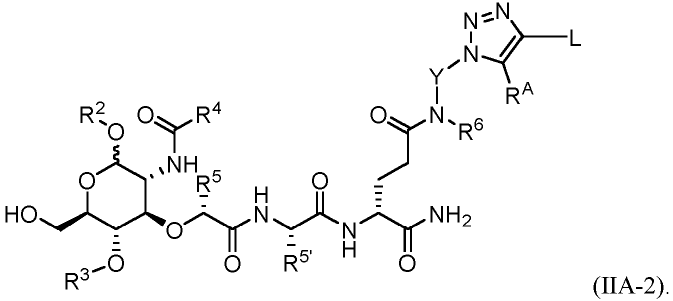

In embodiments, the compound of formula (IIA-2) is: 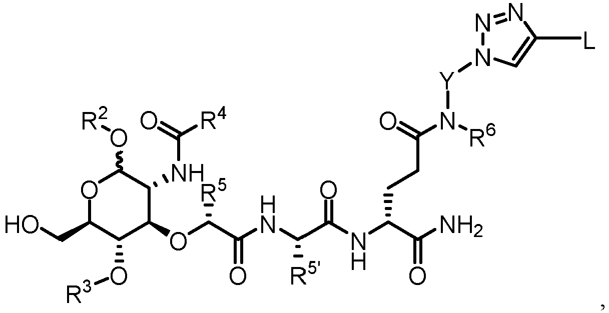 or a pharmaceutically acceptable salt thereof.

In embodiments of the compound of formula (I), (IA), (IB), (II), (II-1), (II-2), (II-A), (IIA-1) or (IIA-2), Y is alkylene. In embodiments of the compound of formula (I), (IA), (IB), (II), (II-1), (II-2), (II-A), (IIA-1) or (IIA-2), Y is C₁₋₆alkylene. In embodiments, Y is C₁₋₅alkylene. In embodiments, Y is C₁₋₃alkylene. In embodiments, Y is -CH₂-.

In embodiments of the compound of formula (I), (IA), (IB), (II), (II-1), (II-2), (II-A), (IIA-1) or (IIA-2), R^{A} is independently selected for each occurrence from the group consisting of hydrogen, halo, alkoxy, haloalkoxy, cyano, hydroxyl, -N(R^{C})(R^{D}), -C(O)N(R^{C})(R^{D}), - N(R^{C})C(O)R^{B}, -OC(O)NR^{C}R^{D}, -NR^{C}C(O)OR^{B}, -OC(O)R^{B}, -C(O)OR^{B}, -C(O)R^{B} , -CO₂H, -NO₂, -SH, S(O)_{X}R^{B} (wherein X is 0, 1, or 2), aryl, arylalkyl, heteroaryl, heteroarylalkyl, cycloalkyl, heterocycloalkyl, and R^{B}.

In embodiments of the compound of formula (I), (IA), (IB), (II), (II-1), (II-2), (II-A), (IIA-1) or (IIA-2), R^{A} is independently selected for each occurrence from the group consisting of halo, alkoxy, haloalkoxy, cyano, hydroxyl, -N(R^{C})(R^{D}), -C(O)N(R^{C})(R^{D}), -N(R^{C})C(O)R^{B}, - OC(O)NR^{C}R^{D}, -NR^{C}C(O)OR^{B}, -OC(O)R^{B}, -C(O)OR^{B}, -C(O)R^{B} , -CO₂H, -NO₂, -SH, S(O)_{X}R^{B} (wherein X is 0, 1, or 2), aryl, arylalkyl, heteroaryl, heteroarylalkyl, cycloalkyl, heterocycloalkyl, and R^{B}.

In embodiments of the compound of formula (I), (IA), (IB), (II), (II-1), (II-2), (II-A), (IIA-1) or (IIA-2), alkyl, alkylene, alkylene-aryl, and aryl is optionally substituted with one or more R^{A};

In embodiments of the compound of formula (I), (IA), (IB), (II), (II-1), (II-2), (II-A), (IIA-1) or (IIA-2), R^{C} and R^{D} are independently selected for each occurrence from the group consisting of hydrogen, alkyl, haloalkyl -C(O)R^{B}, and -C(O)OR^{B}; or R^{C} and R^{D} are taken together with the nitrogen to which they are attached to form a heterocyclic ring optionally substituted with R^{A}.

In embodiments of the compound of formula (I), (IA), (IB), (II), (II-1), (II-2), (II-A), (IIA-1) or (IIA-2), R^{B} is alkyl, alkenyl, or alkynyl optionally substituted with one or more fluoro.

In embodiments of the compound of formula (I), (IA), (IB), (II), (II-1), (II-2), (II-A), (IIA-1) or (IIA-2), R^{A} is -H.

In some embodiments of the compound of formula (I), (IA), (IB), (II), (II-1), (II-2), (II-A), (IIA-1) or (IIA-2), R² is alkyl, aryl, or alkylene-aryl. In embodiments, aryl is optionally substituted with alkyl.

In embodiments of the compound of formula (I), (IA), (IB), (II), (II-1), (II-2), (II-A), (IIA-1) or (IIA-2), R² and R³ are each independently selected from the group consisting of -H, alkyl, aryl, alkylene-aryl, -C(O)-alkyl, and -C(O)-aryl.

In embodiments of the compound of formula (I), (IA), (IB), (II), (II-1), (II-2), (II-A), (IIA-1) or (IIA-2), R² is -H or benzyl. In embodiments of the compounds of formula (I), R² is -H. In embodiments, R² is benzyl.

In embodiments of the compound of formula (I), (IA), (IB), (II), (II-1), (II-2), (II-A), (IIA-1) or (IIA-2), R⁴, R⁵, and R^{5'} are each alkyl.

In embodiments of the compound of formula (I), (IA), (IB), (II), (II-1), (II-2), (II-A), (IIA-1) or (IIA-2), R⁴ is alkyl. In embodiments, R⁴ is methyl.

In embodiments of the compound of formula (I), (IA), (IB), (II), (II-1), (II-2), (II-A), (IIA-1) or (IIA-2), R³ is -H. In embodiments R⁶ is -H. In embodiments, R³ and R⁶ are both - H.

In embodiments of the compound of formula (I), (IA), (IB), (II), (II-1), (II-2), (II-A), (IIA-1) or (IIA-2), R²², R³³, R^{33'}, R⁴⁴, R^{44'}, R⁵⁵, and R^{55'} are each -H

In embodiments of the compound of formula (I), (II), (IA), (IB), (II-1), (II-2), (II-A), (IIA-1) or (IIA-2), L comprises a cholesteryl moiety or at least one fatty acid chain comprising at least 13 carbons. In embodiments, L comprises a cholesteryl moiety. In embodiments, L comprises at least one fatty acid chain comprising at least 13 carbons. In embodiments, L comprises at least two fatty acid chains comprising at least 15 carbons. In embodiments, L comprises at least one C₁₇ fatty acid chain. In embodiments, L comprises at least two C₁₇ fatty acid chains. In embodiments, L comprises at least one fatty acid chains independently selected from a C₁₇ alkyl or a C₁₇ alkenyl. In embodiments, L comprises at least two fatty acid chains independently selected from a C₁₇ alkyl or a C₁₇ alkenyl. In embodiments, the C₁₇ fatty acid chain is derived from stearic acid or oleic acid. In embodiments, the C₁₇ fatty acid chain is derived from stearic acid. In embodiments, the C₁₇ fatty acid chain is derived from oleic fatty acid. In embodiments, L comprises two C₁₇ fatty acid chains derived from stearic acid.

In embodiments of the compound of formula (I), (IA), (IB), (II), (II-1), (II-2), (II-A), (IIA-1) or (IIA-2), L is selected from the group consisting of a fatty acid chain, -alkylene-C(O)-W, -alkylene-O-C(O)-W, -alkylene-N-(alkylene-C(O)-NR¹¹-alkylene-NR¹¹-C(O)-W)₂, and -alkylene-N-(alkylene-C(O)-W)₂.

In embodiments of the compound of formula (I), (IA), (IB), (II), (II-1), (II-2), (II-A), (IIA-1) or (IIA-2), L is selected from the group consisting of C₈₋₃₀fatty acid chain, -CH₂-C(O)-W, -CH₂-O-C(O)-W, -CH₂CH₂-N- CH₂CH₂-C(O)-NR¹¹-CH₂CH₂-NR¹¹-C(O)-W)₂, and - CH₂CH₂-N-(CH₂CH₂-C(O)-W)₂.

In embodiments of the compound of formula (I), (IA), (IB), (II), (II-1), (II-2), (II-A), (IIA-1) or (IIA-2), L is a C₈₋₃₀fatty acid chain. In embodiments, L is a C₈₋₃₀alkyl or a C₈₋₃₀alkenyl. In embodiments, L is a C₁₅₋₂₀alkyl or a C₁₅₋₂₀alkenyl.

In embodiments of the compound of formula (I), (IA), (IB), (II), (II-1), (II-2), (II-A), (IIA-1) or (IIA-2), L is -alkylene-C(O)-W, -alkylene-O-C(O)-W, -alkylene-N-(alkylene-C(O)-NR¹¹-alkylene-NR¹¹-C(O)-W)₂, or -alkylene-N-(alkylene-C(O)-W)₂.

In embodiments of the compound of formula (I), (IA), (IB), (II), (II-1), (II-2), (II-A), (IIA-1) or (IIA-2), L is -alkylene-C(O)-W. In embodiments, L is -C₁₋₆alkylene-C(O)-W. In embodiments, L is -CH₂-C(O)-W.

In embodiments of the compound of formula (I), (IA), (IB), (II), (II-1), (II-2), (II-A), (IIA-1) or (IIA-2), L is -alkylene-O-C(O)-W. In embodiments, L is -C₁₋₆alkylene-O-C(O)-W. In embodiments, L is -CH₂-O-C(O)-W.

In embodiments of the compound of formula (I), (IA), (IB), (II), (II-1), (II-2), (II-A), (IIA-1) or (IIA-2), L is -alkylene-N-(alkylene-C(O)-NR¹¹-alkylene-NR¹¹-C(O)-W)₂. In embodiments, L is -C₂₋₆alkylene-N-(-C₂₋₆alkylene-C(O)-NR¹¹-C₂₋₆alkylene-NR¹¹-C(O)-W)₂. In embodiments, L is -CH₂-CH₂-N-(CH₂-CH₂-C(O)-NR¹¹-CH₂-CH₂-NR¹¹-C(O)-W)₂.

In embodiments of the compound of formula (I), (IA), (IB), (II), (II-1), (II-2), (II-A), (IIA-1) or (IIA-2), L is -alkylene-N-(alkylene-C(O)-W)₂. In embodiments, L is -C₁₋₆alkylene-N-(C₁₋₆alkylene-C(O)-W)₂. In embodiments, L is -CH₂-CH₂-N-(CH₂-CH₂-C(O)-W)₂.

In embodiments of the compound of formula (I), (IA), (IB), (II), (II-1), (II-2), (II-A), (IIA-1) or (IIA-2), L is a C₁₈ fatty acid chain. In embodiments, L is a C₁₈ alkyl or a C₁₈ alkenyl. In embodiments, L is -CH₂(CH₂CH₂)₈-CH₃.

In embodiments of the compound of formula (I), (IA), (IB), (II), (II-1), (II-2), (II-A), (IIA-1) or (IIA-2), W is a fatty acid chain, -O-alkylene-C(H)(OR⁸)-alkylene-OR⁹, a phospholipid, or a sterol.

In embodiments of the compound of formula (I), (IA), (IB), (II), (II-1), (II-2), (II-A), (IIA-1) or (IIA-2), W is a C₈₋₃₀fatty acid chain. In embodiments, W is a C₈₋₃₀alkyl or a C₈₋₃₀alkenyl. In embodiments, W is a C₈₋₃₀alkyl. In embodiments, W is a C₈₋₃₀alkenyl. In embodiments, W is a C₁₂₋₁₈ fatty acid chain. In embodiments, W is a C₁₂₋₁₈alkyl or a C₁₂₋₁₈alkenyl. In embodiments, W is a C₁₂₋₁₈alkyl. In embodiments, W is a C₁₂₋₁₈alkenyl. In embodiments, W is a C₁₈fatty acid chain. In embodiments, W is a C₁₇ fatty acid chain. In embodiments, W is a C₁₇alkyl or a C₁₇alkenyl. In embodiments, W is a C₁₇alkyl. In embodiments, W is a C₁₇alkenyl. In embodiments, W is -(CH₂CH₂)₈-CH₃. In embodiments, W is a fatty acid chain comprising at least 15 carbons. In embodiments, W is a fatty acid chain comprising at least 18 carbons. In embodiments, W is a fatty acid chain comprising at least 17 carbons. In embodiments, W is a fatty acid chain comprising at least 18 carbons.

In embodiments of the compounds of formula (I), (IA), (IB), (II), (II-1), (II-2), (II-A), (IIA-1) or (IIA-2), W is: 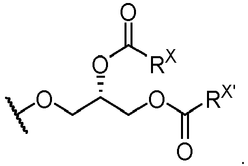 In embodiments, R^{X} and R^{X'} is each independently a fatty acid chain. In embodiments, R^{X} and R^{X'} is each independently a fatty acid chain comprising at least 15 carbons. In embodiments, R^{X} and R^{X'} is each independently a fatty acid chain comprising at least 17 carbons. In some embodiment, R^{X} and R^{X'} is each independently a -C₈₋₃₀fatty acid chain. In embodiments, R^{X} and R^{X'} is each independently a -C₈₋₃₀alkyl or a -C₈₋₃₀alkenyl. In embodiments, R^{X} and R^{X'} are both a -C₈₋₃₀alkyl. In embodiments, R^{X} and R^{X'} are both a -C₈₋₃₀alkenyl. In embodiments, R^{X} and R^{X'} is each independently a C₁₂₋₁₈fatty acid chain. In embodiments, R^{X} and R^{X'} is each independently a -C₁₂₋₁₈alkyl or a -C₁₂₋₁₈alkenyl. In embodiments, R^{X} and R^{X'} are a -C₁₂₋₁₈alkyl. In embodiments, R^{X} and R^{X'} are a -C₁₂₋₁₈alkenyl. In embodiments, R^{X} and R^{X'} is each independently a C₁₇fatty acid chain. In embodiments, R^{X} and R^{X'} are each independently a C₁₇alkyl or a C₁₇alkenyl. In embodiments, R^{X} and R^{X'} are a C₁₇alkyl. In embodiments, R^{X} and R^{X'} are a C₁₇alkenyl. In embodiments, the C₁₇ chains are independently derived from stearic acid or oleic acid. In embodiments, the C₁₇ chains are derived from stearic acid. In embodiments, the C₁₇ chains are derived from oleic acid.. In embodiments, R^{X} and R^{X'} are both -(CH₂CH₂)₈-CH₃.

In embodiments of the compounds of formula (I), (IA), (IB), (II), (II-1), (II-2), (II-A), (IIA-1) or (IIA-2), W is a sterol.

In embodiments of the compounds of formula (I), (IA), (IB), (II), (II-1), (II-2), (II-A), (IIA-1) or (IIA-2), W is cholesterol:

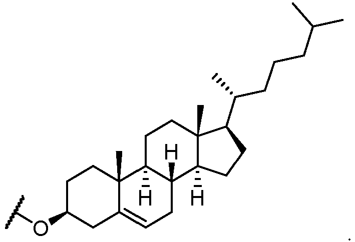

In embodiments of the compound of formula (I), (IA), (IB), (II), (II-1), (II-2), (II-A), (IIA-1) or (IIA-2),W is a phospholipid is selected from the group consisting of: a phosphatidylcholine (PC), a phosphatidylglycerol (PG), a phosphatidylserine (PS), a phosphatidylethanolamine (PE), a phosphatidic acid (PA), and a lysophosphatidylcholine. In embodiments, W is a phosphatidylcholine (PC). In embodiments, W is a phosphatidylglycerol (PG). In embodiments, W is a phosphatidylserine (PS). In embodiments, W is a phosphatidylethanolamine (PE). In embodiments, W is a phosphatidic acid (PA). In embodiments, W is a lysophosphatidylcholine.

In embodiments of the compound of formula (I), (IA), (IB), (II), (II-1), (II-2), (II-A), (IIA-1) or (IIA-2), W is: 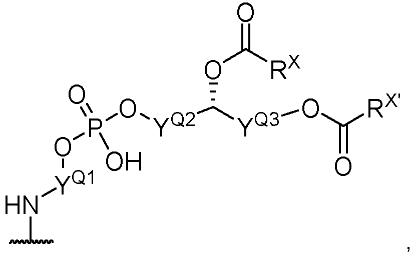 wherein
Y^{Q1}, Y^{Q2}, and Y^{Q3} are are each independently alkylene. In embodiments, Y^{Q1} is C₂₋₆alkylene, and Y^{Q2}, and Y^{Q3} are each independently -C₁₋₃alkylene. In embodiments, R^{X} and R^{X'} are each independently a fatty acid chain having at least 15 carbons, or in particular embodiments R^{X} and R^{X'} are each independently a fatty acid chain having at least 17 carbons. In embodiments, R^{X} and R^{X'} are each independently a C₈₋₃₀ fatty acid chain. In embodiments, R^{X} and R^{X'} are each independently a C₈₋₃₀ alkyl or a C₈₋₃₀ alkenyl. In embodiments, R^{X} and R^{X'} are each independently a C₁₅₋₃₀ alkyl or a C₁₅₋₃₀ alkenyl. In embodiments, R^{X} and R^{X'} are each independently a C₁₅₋₂₀ alkyl or a C₁₅₋₂₀ alkenyl. In embodiments, R^{X} and R^{X'} are each independently a C₁₇alkyl or a C₁₇ alkenyl. In embodiments, R^{X} and R^{X'} are both -(CH₂CH₂)₈-CH₃.

In embodiments of the compound of formula (I), (IA), (IB), (II), (II-1), (II-2), (II-A), (IIA-1) or (IIA-2), the W is:

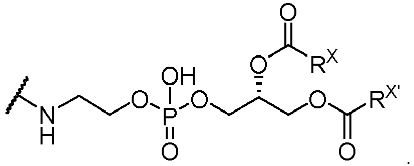

In embodiments of the compound of formula (I), (IA), (IB), (II), (II-1), (II-2), (II-A), (IIA-1) or (IIA-2), the W is: 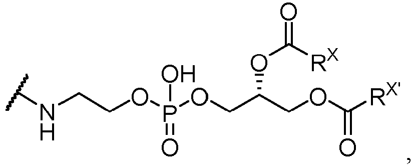 or a pharmaceutically acceptable salt thereof; wherein R^{X} and R^{X'} are each independently a C₈₋₃₀ fatty acid chain. In embodiments, the fatty acid is saturated. In embodiments, R^{X} and R^{X'} are each independently a C₈₋₃₀ alkyl or a C₈₋₃₀ alkenyl. In embodiments, R^{X} and R^{X'} are each independently a C₁₅₋₂₀ alkyl or a C₁₅₋₂₀ alkenyl. In embodiments, R^{X} and R^{X'} are each independently a C₁₇alkyl or a C₁₇ alkenyl. In embodiments, R^{X} and R^{X'} are both -(CH₂CH₂)₈-CH₃.

In embodiments of the compound of formula (I), (IA), (IB), (II), (II-1), (II-2), (II-A), (IIA-1) or (IIA-2), R⁸ and R⁹ are each independently R^{X} or -C(O)-R^{X};

In embodiments of the compound of formula (I), (IA), (IB), (II), (II-1), (II-2), (II-A), (IIA-1) or (IIA-2), R⁶ and R¹¹ are each independently -H, or alkyl.

In embodiments, the present disclosure provides a compound selected from the group consisting of: 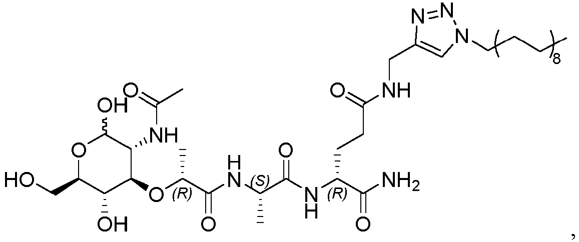 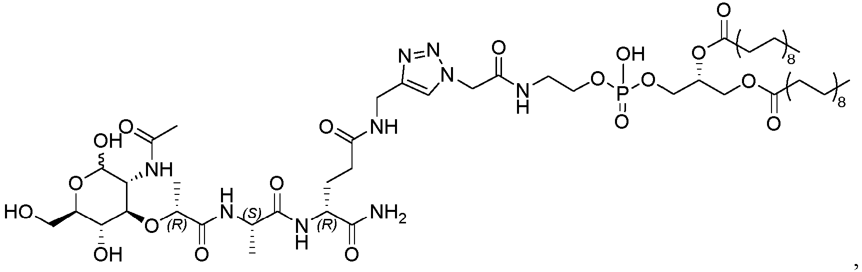 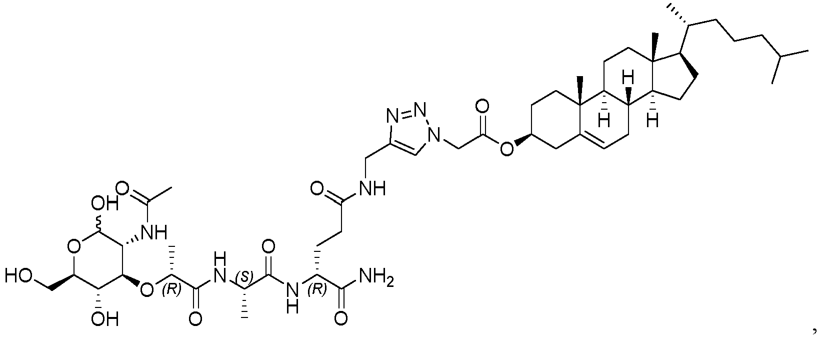 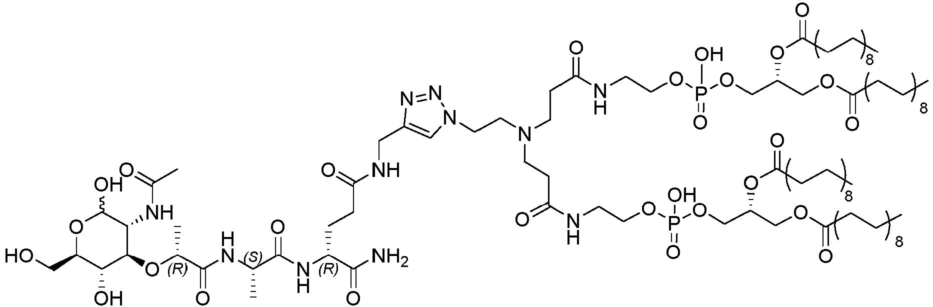 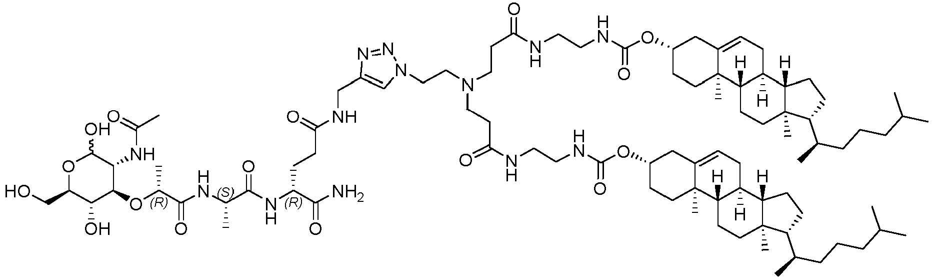 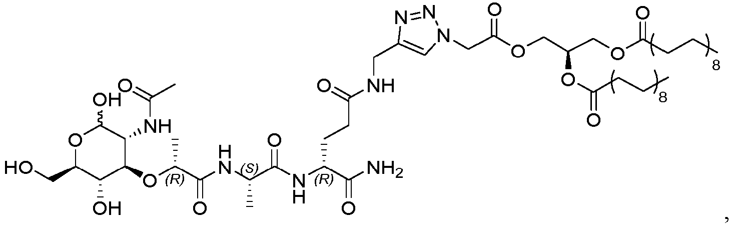 or stereoisomer thtereof (e.g., an alpha or beta anomer thereof, or a tautomer thereof).

In embodiments, the present disclosure provides a compound selected from the group consisting of: 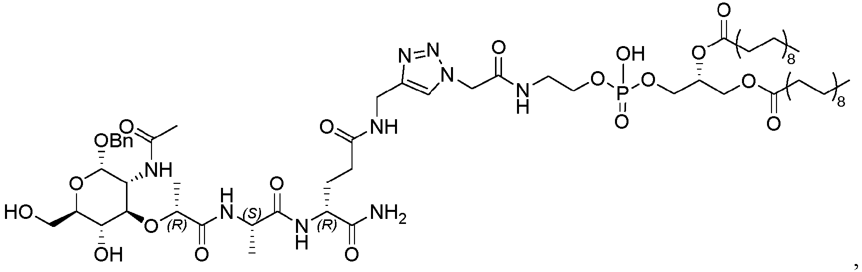 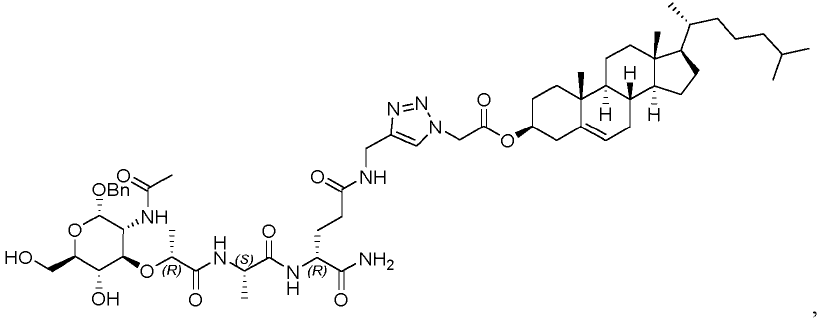 or a stereoisomer thereof (e.g., an anomer thereof, or mixture of anomers thereof).

In embodiments, the present disclosure provides a compound of formula (I), (IA), (IB), (II), (II-1), (II-2), (IIA), (IIA-1), (IIA-2) or a stereoisomer thereof.

In embodiments, the present disclosure provides a compound of formula (I), (IA), (IB), (II), (II-1), (II-2), (IIA), (IIA-1), (IIA-2) or a diastereomer, or tautomer thereof.

Disclosed herein, but not necessarily part of the invention, is one or more compounds selected from Table 1.

In embodiments, provided herein is one or more pharmaceutically acceptable salts of a compound selected from Table 1.

Disclosed herein, but not necessarily part of the invention, is one or more compounds of selected from Table 1, or a stereoisomer, or a pharmaceutically acceptable salt thereof.

**Table 1. Compounds**

| No. | Compound | |
|---|---|---|
| 1 | MDP-C18 [click] | 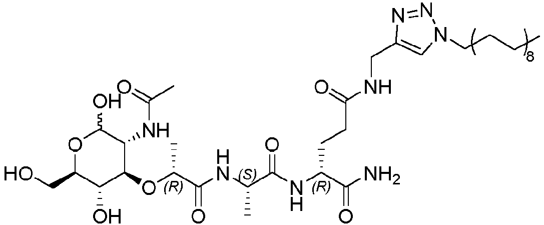 |
| 2 | MDP-DSPE [click] | 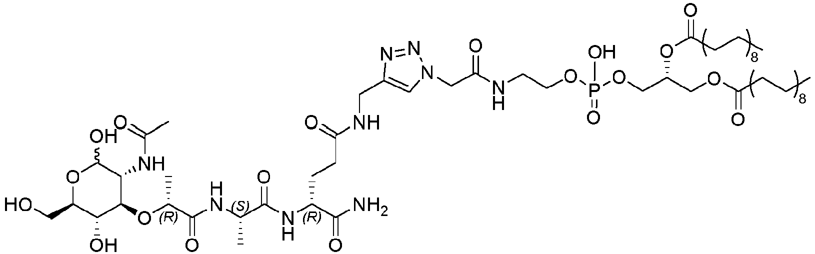 |
| 3 | MDP-chol [click] | 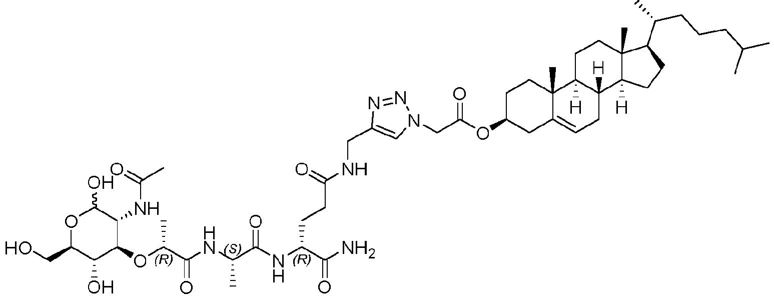 |
| 4 | MDP-DSPE₂ [click] | 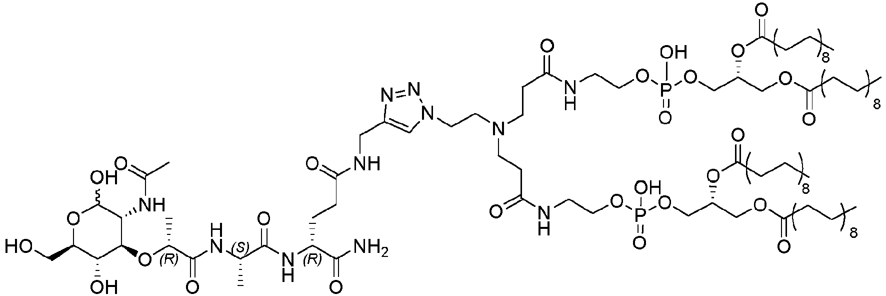 |
| 5 | MDP-Chol₂ [click] | 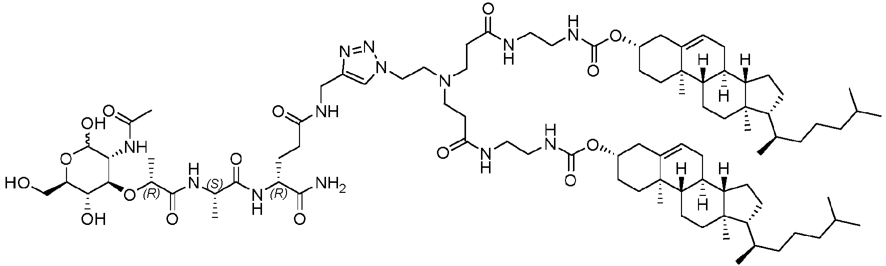 |
| 6 | MDP-DSG [click] | 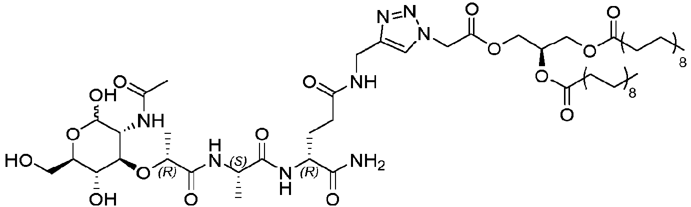 |
| 7 | MDP (Bn)-DSPE [click] | 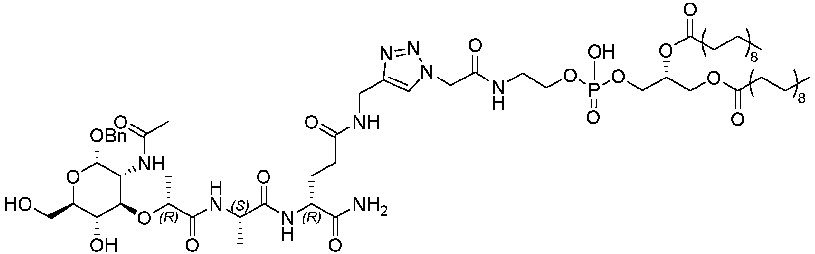 |
| 8 | MDP (Bn)-chol [click] | 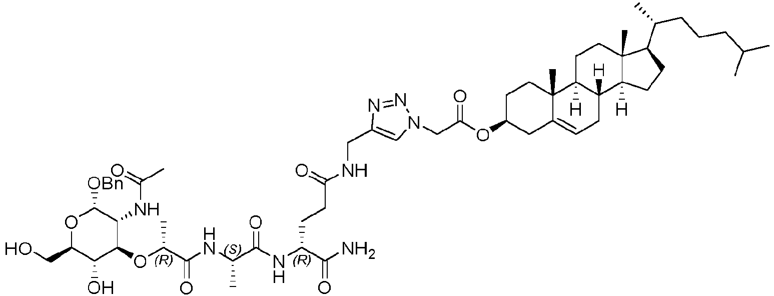 |
| 9 | MTP-b-C18 [invcli ck] | 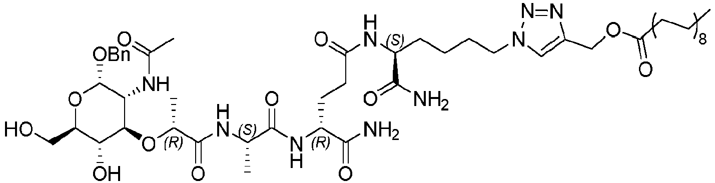 |
| 10 | MTP-b-C18 | 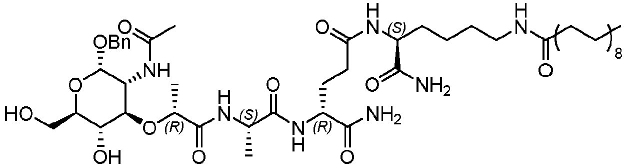 |
| 11 | MDP-C18 | 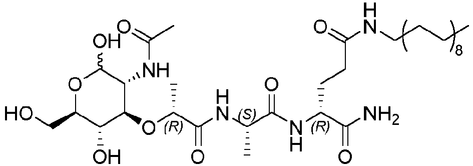 |
| 12 | MDP-DSPE | 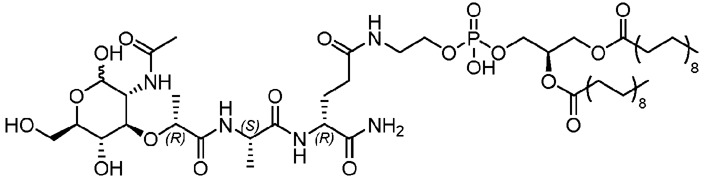 |
| 13 | MTP-b-DSG | 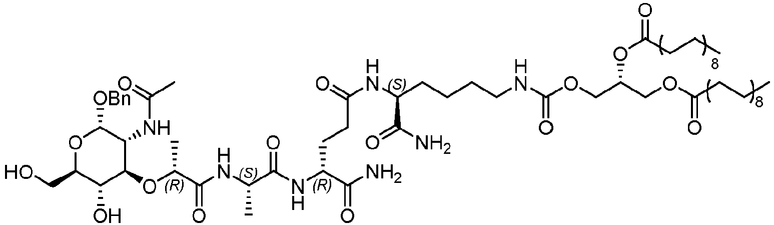 |
| 14 | MTP( Bn)-a-DPPE | 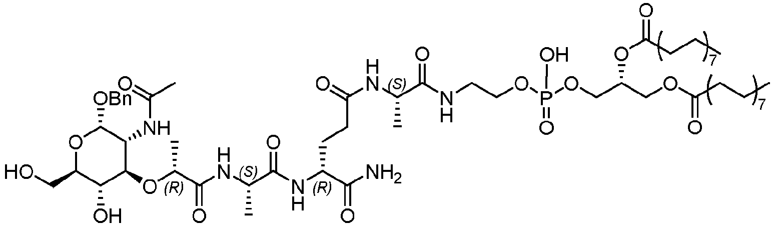 |
| 15 | MTP-a-chol | 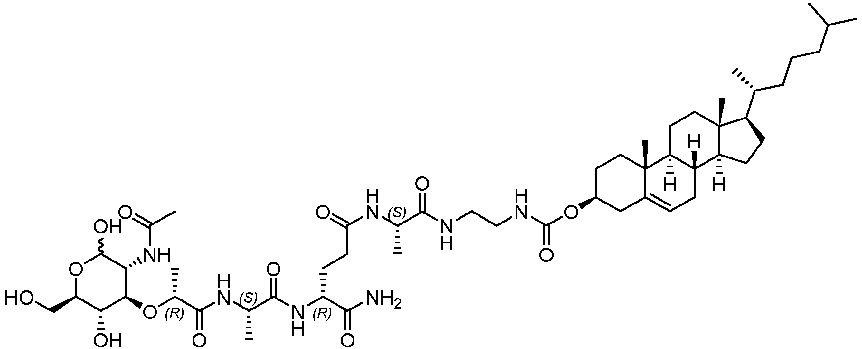 |
| 16 | MDP( Bn)-chol | 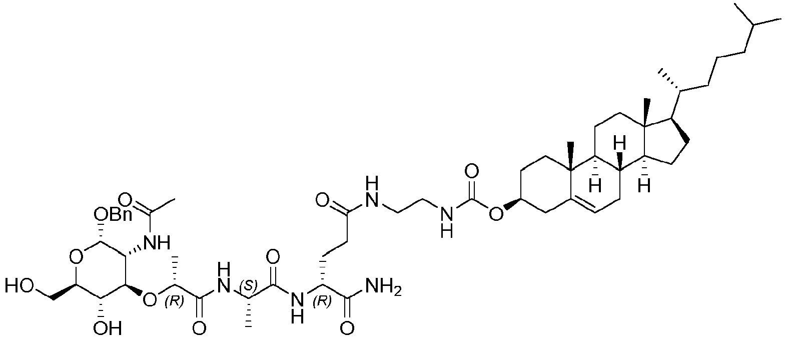 |
| 17 | MTP-a-DSPE | 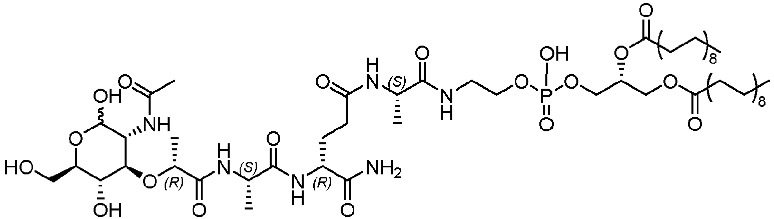 |
| 18 | MTP( Bn)-a-DSPE | 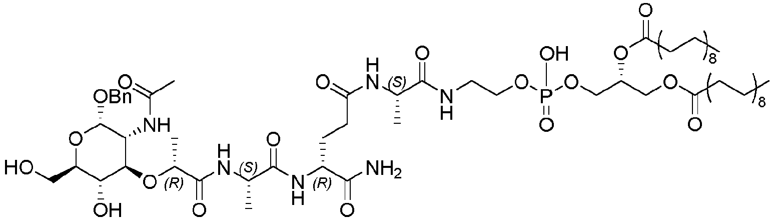 |
| 19 | MDP( Bn)-DSPE | 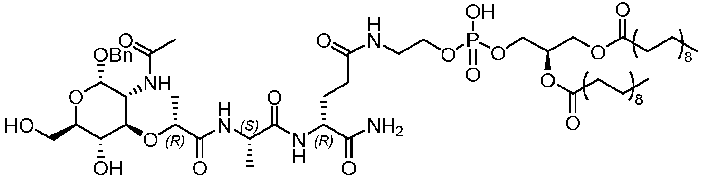 |

Muramyl tripeptide phosphatidylethanolamine; N-(N-Acetylmuramoyl)-L-alanyl-D-alpha-glutaminyl-N-[(7R)-4-hydroxy-4-oxido-10-oxo-7-[(1-oxohexadecyl)oxy]-3,5,9-trioxa-4-phosphapentacos-1-yl]-L-alaninamide (MTP-a-DPPE or Mifamurtide): Molecular weight: 1238 Dalton. CLogP = 10.59 (uncharged) and 4.80 (negatively charged). Mifamurtide (CAS-number [83461-56-7]) was prepared as according to literature procedures (e.g. Brundish, D. E.; Wade, R. (1985) J Label Compd Radiopharm. 22 (1): 29-35. doi:10.1002/jlcr.2580220105). The lipophilicity of this molecule is relatively low at CLogP 4.80 in physiological circumstances.

N-Acetylmuramyl-L-Alanyl-D-Isoglutamine-6-O-Stearoyl (MDP-C18[mur]) Molecular weight: 759 Dalton. CLogP = 5.39 (uncharged) and 1.39 (negatively charged). MDP-C18[mur] (CAS-number [60398-08-5]) was prepared as according to literature procedures (e.g. Matsumoto K. et al. (1981) Infect Immun. 32(2):748-58). The lipophilicity of this molecule is low at CLogP 1.39 in physiological circumstances is unlikely to be sufficient to ensure its robust incorporation into HDL-derived nanoparticles.

Romurtide (CAS-number [78113-36-7]) Molecular weight: 887 Dalton. CLogP = 3.90 (uncharged) and 0.61 (negatively charged) has a lipophilicity (CLogP 0.61 in physiological circumstances). The lipophilicity of this compound is low with a CLogP of the charged molecule that is close to 0.

Murabutide (CAS-number [74817-61-1]) Molecular weight: 549 Dalton. CLogP = -1.53 (uncharged) has a CLogP value that is negative. This molecule is hydrophilic given its CLogP value that is below 0.

In embodiments, compounds of the present disclosure (such as one or more compounds of formula (I), (IA), (IB), (II), (II-1), (II-2), (IIA), (IIA-1), (IIA-2) or Table 1) activate nucleotide-binding oligomerization domain-containing protein 2 (NOD2).

### Anomers and open/closed ring structures

In embodiments, the molecules of the present disclosure bear an -OH substituent at the anomeric hemi-acetal carbon of the muramyl sugar group, i.e. when R₂ = H, it is understood that both anomeric isomers alpha and beta are included in the compounds of the present disclosure.

Furthermore, in these cases where R₂ = H, it is known in the art that such molecules (in aqueous environments) actually exist in both the closed ring isomer as well as the open ring structure. Again, it is understood that both the open and closed ring isomers are included in the compounds of the present disclosure.

Below, in non-limiting examples, the top structures show the alpha and beta anomers, and the bottom structures show the general anomeric ring-closed structure (left) and the open ring structure (right).

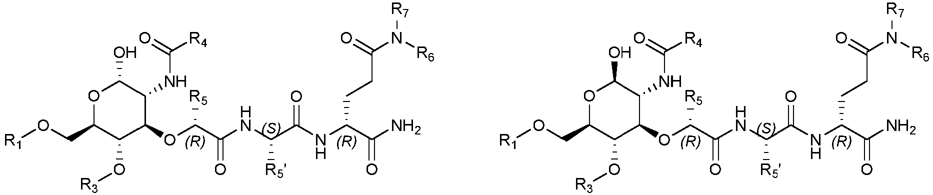

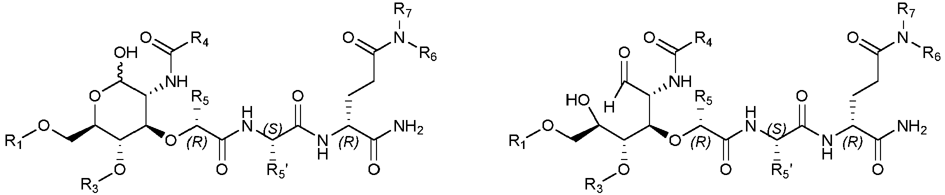

### Molecular weight

The compounds of the invention preferably have a molecular weight higher than 500 Dalton, higher than 700 Dalton, higher than 950 Dalton, or higher than 1,200 Dalton.

The compounds of the invention preferably have a molecular weight lower than 10,000 Dalton, lower than 5,000 Dalton, lower than 2,500 Dalton, or lower than 1,750 Dalton.

### Hydrophobicity

The compounds of the disclosure are, in particular embodiments, hydrophobic in nature. Hydrophobiicty can be estimated by calculation of the CLogP value. This can be done in software programs such as for example Perkin Elmer's ChemDraw or ChemDraw Professional (v18). The higher the CLogP value of a compound, the more hydrophobic a compound is.

In embodiments, the compounds of the present disclosure have a CLogP value higher than about 1, higher than about 3, higher than about 5, higher than about 7, higher than about 9, or higher than about 11.

The CLogP value represents the n-octanol/water partition coefficient (Log Po/w) of a molecule, and is a calculated value as opposed to LogP values, i.e. values assessed by experimentation. Accordingly, CLogP values may deviate from LogP values. Importantly, however, CLogP values give a good comparison between the lipophilicities of molecules. CLogP values can be assessed for molecules in either their uncharged or their charged state. This is the case for molecules that have ionogenic groups, such as molecules with carboxylic acid (-COOH) or phosphate (-OP(O)OH-O-) groups. At physiological pH (about 7.4) these particular groups are deprotonated and thus become charged. Also alkyl(ated) amine groups become charged at physiological pH, in this case by protonation.

At physiological pH, the molecules of the invention have CLogP values that are lower than 20, or lower than 15, or lower than 10. Moreover, at physiological pH, the molecules of the invention have CLogP values that are higher than 3, or higher than 4, or higher than 5, or higher than 5.5.

### Nanobiologic Compositions

Provided herein are nanobiologic compositions comprising a nanoparticle carrier and one or more compounds of the present disclosure (such as a compound of formula (I), (IA), (IB), (II), (II-1), (II-2), (IIA), (IIA-1), or (IIA-2) as disclosed herein or Table 1).

In embodiments, the compounds of the present disclosure can be formulated in a nanoparticle carrier, which can include, but is not limited to polyplexes, colloidal dispersion systems, such as macromolecule complexes, nanocapsules, microspheres, beads, and lipid-based systems including oil-in-water emulsions, micelles, mixed micelles, liposomes, lipoplexes, lipid nanoparticles, lipid nanocapsules, lipidoids, rapidly eliminated lipid nanoparticles (reLNPs), micro- and nano-emulsions, and the like, HDL-derived nanoparticles, polymeric nanoparticles, including poly (lactic-co-glycolic acid) (PLGA) nanoparticles such as PLGA microspheres, poly(lactide) (PLA) nanoparticles, poly(ε-caprolactone) (PCL) nanoparticles, poly(butyl cyanoacrylate) (PBCA) nanoparticles, demdrimers, hyperbranched polyglycerol (HPG) nanoparticles, PEG-polyaspartate micellar nanoparticles, cationic polymers including for example poly(L-lysine), polyethylenimine (PEI), DEAE-dextran, poly(amino esters) (PBAE) and chitosan, cyclodextrin nanoparticles, metallic nanopartides, surfactant based emulsions, virus like particles (e.g., particles that are primarily made up of viral structural proteins but that are not infectious or have low infectivity), peptide or protein-based particles such as albumin nanopartides, nanowires, gold nanoparticles, magnetic nanoparticles, core-shell nanoparticles, carbon nanotubes, nanocrystals, hyaluronidase, and combinations thereof.

In embodiments, the compounds of the present disclosure may be formulated in a nanoparticle carrier, such as those described in US5,567,434, US5,552,157, US5,565,213, US5,738,868, US5,795,587, US10,485,884, US2018/0263907, US2016/0317647 , US2019/0290593, US2020/0253884, US2020/0376146, and WO2018/071549.

In embodiments, the nanoparticle carrier is a high-density lipoprotein (HDL)-derived nanoparticle. The high-density lipoprotein (HDL)-derived nanoparticles are envisioned as delivery vehicles that may, for example, improve the therapeutic index of small-molecule immunomodulatory compounds and/or confer innate immune cell-specific delivery. By conferring targeting specificity for innate immune cells (such as myeloid cells, myeloid progenitor cells, and hematopoietic stem cells in the bone marrow, blood and/or spleen), the therapeutic agents encapsulated or incorporated in the HDL-derived nanoparticles may be deposited in a concentrated and localized fashion. In embodiments, the high-density lipoprotein (HDL)-derived nanoparticle comprises apoA-I or a peptide mimetic of apoA-I. In embodiments, the high-density lipoprotein (HDL)-derived nanoparticle comprises apoA-I.

Human apoA-I can be isolated or prepared by any method known in the art. In embodiments, human apoA-I is isolated from human HDL. Another known method comprises the synthesis of apoA-I by recombinant protein expression, for example in E. coli organisms. When expressed in bacteria the apoA-I may include an N-terminal methionine or a formyl-methionine. The presence of the methionine group can be assessed by mass spectroscopic (MS) methods that are known in the art. The position of the methionine in the protein sequence can be assessed after digestion of apoA-I with subsequent analysis of the peptide mixture with MS, as is also known in the art.

In embodiments, purifications of apoA-I, including any of its variations, may comprise any method known in the art (e.g. use of hydrophobic interaction chromatography, ion exchange columns, precipitations, etc.). Production methods may or may not comprise the use of affinity tags that enable the purification of the proteins; such tags require removal after purification to restore the human apoA-I identity.

In embodiments, the high-density lipoprotein (HDL)-derived nanoparticle comprises ApoA-1 Milano.

Suitable apoA-I mimetic polypeptides may have the sequence shown in Table 2 (SEQ ID NOS: 256 to 263, and 342 to 346) or in SEQ ID NOS: 1 to 341.

**Table 2. ApoA-I mimetics**

| SEQ ID NO | Amino Acid Sequence |
|---|---|
| 256 | DWLKAFYDKVAEKLKEAF (18A) |
| 257 | Ac-DWLKAFYDKVAEKLKEAF-NH₂ (2F) |
| 260 | Ac-DWFKAFYDKVAEKFKEAF-NH₂ (4F) |
| 258 | Ac-DWFKAFYDKVAEKLKEAF-NH₂ (3^{F3}) |
| 259 | Ac-DWLKAFYDKVAEKFKEAF-NH₂ (3F¹⁴) |
| 261 | Ac-DWLKAFYDKVFEKFKEFF-NH₂ (5F) |
| 262 | Ac-DWLKAFYDKFFEKFKEFF-NH₂ (6F) |
| 263 | Ac-DWFKAFYDKFFEKFKEFF-NH₂ (7F) |
| 342 | Ac-FWLKAFYDKVAEKLKEAF-NH₂ (3F-1) |
| 343 | Ac-DFLKAFYDKVAEKLKEAF-NH₂ (3F-2) |
| 344 | Ac-DWFRAFYDKVAEKFREAF-NH₂ (4F-R)*^{q}* |
| 345 | Ac-DWFKAFYDRVAERFKEAF-NH₂ (4F-R')^{q} |
| 346 | Ac-DWLXAFYDXVAEXLXEAF-NH₂ (2F') |

In embodiments, the apoA-I mimetic is DWLKAFYDKVAEKLKEAF (SEQ ID NO. 256). In embodiments, the apoA-I mimetic is Ac-DWLKAFYDKVAEKLKEAF-NH₂ (SEQ ID NO. 257). In embodiments, the apoA-I mimetic is Ac-DWFKAFYDKVAEKFKEAF-NH₂ (SEQ ID NO. 260).

In embodiments, apoA-I mimetics are optionally acetylated on the N-terminus, or optionally amidated on the C-terminus. In embodiments, the apoA-I mimetics are acetylated on the N-terminus. In embodiments, the apoA-I mimetics are amidated on the C-terminus. In embodiments, the apoA-I mimetics are acetylated on the N-terminus and amidated on the C-terminus.In embodiments, the HDL-derived nanoparticles of the present disclosure comprise one or more phospholipids. All phospholipids ranging in chain length from C4 to C30, saturated or unsaturated, cis or trans, unsubstituted or substituted with 1-6 side chains, and with or without the addition of lysolipids are contemplated for use in the nanoparticles described herein. Additionally, other synthetic variants and variants with other phospholipid headgroups are also contemplated. In embodiments, the HDL-derived nanoparticle comprises a phospholipid. In embodiments, the HDL-derived nanoparticle comprises a phospholipid and a lysolipid.

Non-limiting examples of the phospholipids that may be used in the present composition include phosphatidylcholines (PC), phosphatidylglycerols (PG), phosphatidylserines (PS), phosphatidylethanolamines (PE). In embodiments phosphatidic acid/esters (PA( may be used.

In embodiments, the phospholipid or lysolipid is one or more of the following: DDPC CAS-3436-44-0 1,2-Didecanoyl-sn-glycero-3-phosphocholine, DEPA-NA CAS-80724-31-8 1,2-Dierucoy l-sn-glycero-3-phosphate (Sodium Salt), DEPC CAS-56649-39-9 1,2-Dierucoyl-sn-glycero-3-phosphocholine, DEPE CAS-988-07-2 1,2-Dierucoyl-sn-glycero-3-phosphoethanolamine, DEPG-NA 1,2-Dierucoyl-sn-glycero-3-phospho-rac-(l-glycerol) (Sodium Salt), DLOPC CAS-998-06-11,2-Dilinoleoyl-sn-glycero-3-phosphocholine, DLPA-NA 1,2-Dilauroyl-sn-glycero-3-phosphate (Sodium Salt), DLPC CAS-18194-25-7 1,2-Dilauroyl-sn-glycero-3-phosphocholine, DLPE 1,2-Dilauroyl-sn-glycero-3-phosphoethanolamine, DLPG-NA l,2-Dilauroyl-sn-glycero- 3-phospho-rac-(l-glycerol) (Sodium Salt), DLPG-NH4 1,2-Dilauroyl-sn-glycero-3-phospho-rac-(l-glycerol) (Ammonium Salt), DLPS-NA 1,2-Dilauroyl-sn-glycero-3-phosphoserine (Sodium Salt), DMPA-NA CAS-80724-3 1,2-Dimyristoyl-sn-glycero-3-phosphate (Sodium Salt), DMPC CAS-18194-24-6 1,2-Dimyristoyl-sn-glycero-3-phosphocholine, DMPE CAS-988-07-2 1,2-Dimyristoyl-sn-glycero-3-phosphoethanolamine, DMPG-NA CAS-67232-80-8 1,2-Dimyristoyl-sn-glycero-3-phospho-rac-(l-glycerol) (Sodium Salt), DMPG-NH4 1,2-Dimyristoyl-sn-glycero-3-phospho-rac-(l-glycerol) (Ammonium Salt), DMPG-NH4/NA 1,2-Dimyristoyl-sn-glycero-3-phospho-rac-(lglycerol) (Sodium/Ammonium Salt), DMPS-NA 1,2-Dimyristoyl-sn-glycero-3-phosphoserine (Sodium Salt), DOPA-NA 1,2-Dioleoyl-sn-glycero-3-phosphate (Sodium Salt), DOPC CAS-4235-95-4 1,2-Dioleoyl-sn-glycero-3-phosphocholine, DOPE CAS-4004-5-1 1,2-Dioleoyl-sn-glycero-3-phosphoethanolamine, DOPG-NA CAS-62700-69-0 1,2-Dioleoyl-sn-glycero-3-phospho-rac-(l-glycerol)(Sodium Salt), DOPS-NA CAS-70614-14-1 1,2-Dioleoyl-sn-glycero-3-phosphoserine (Sodium Salt), DPPA-NA CAS-71065-87-7 1,2-Dipalmitoyl-sn-glycero-3-phosphate (Sodium Salt), DPPC CAS-63-89-8 1,2-Dipalmitoyl-sn-glycero-3-phosphocholine, DPPE CAS-923-61-5 1,2-Dipalmitoyl-sn-glycero-3-phosphoethanolamine, DPPG-NA CAS-67232-81-9 1,2-Dipalmitoyl-sn-glycero-3-phospho-rac-(l-glycerol) (Sodium Salt), DPPG-NH4 CAS-73548-70-6 1,2-Dipalmitoylsn-glycero-3-phospho-rac-(l-glycerol) (Ammonium Salt), DPPS-NA l,2-Dipalmitoyl-sn- glycero-3-phosphoserine (Sodium Salt), DSPA-NA CAS-108321-18-2 1,2-Distearoyl-snglycero-3-phosphate (Sodium Salt), DSPC CAS-816-94-4 1,2-Distearoyl-sn-glycero-3-phosphocholine, DSPE CAS-1069-79-0 1,2-Distearoyl-sn-glycero-3-phosphoethanolamine, DSPG-NA CAS-67232-82-0 1,2-Distearoyl-sn-glycero-3-phospho-rac-(l-glycerol) (Sodium Salt), DSPG-NH4 CAS-108347-80-4 1,2-Distearoyl-sn-glycero-3-phospho-rac-(l- glycerol) (Ammonium Salt), DSPS-NA 1,2-Distearoyl-sn-glycero-3-phosphoserine (Sodium Salt), EPC Egg-PC , HEPC Hydrogenated Egg PC, HSPC Hydrogenated Soy PC, LYSOPC MYRISTIC CAS-18194-24-6 l-Myristoyl-sn-glycero-3-phosphocholine, LYSOPC PALMITIC CAS-17364-16-8 l-Palmitoyl-sn-glycero-3-phosphocholine, LYSOPC STEARIC CAS-19420-57-6 l-Stearoyl-sn-glycero-3-phosphocholine, Milk Sphingomyelin, MPPC l-Myristoyl-2-palmitoyl-sn-glycero 3-phosphocholine, MSPC l-Myristoyl-2-stearoyl-sn-glycero-3-phosphocholine, PMPC l-Palmitoyl-2-myristoyl-sn-glycero-3-phosphocholine, POPC CAS-26853-31-6 l -Palmitoyl-2-oleoyl-sn-glycero-3-phosphocholine, POPE l -Palmitoyl-2-oleoyl-sn-glycero-3-phosphoethanolamine, POPG-NA CAS-81490-05-3l-Palmitoyl-2-oleoyl-sn-glycero-3[Phospho-rac-(l-glycerol)] (Sodium Salt), PSPC 1- Palmitoyl-2-stearoyl-sn-glycero-3-phosphocholine, SMPC l -Stearoyl-2-myristoyl-snglycero-3-phosphocholine, SOPC l -Stearoyl-2-oleoyl-sn-glycero-3-phosphocholine, SPPC1-S tearoy 1-2-palmitoy l-sn-glycero-3-phosphocholine. In some preferred embodiments, specific non-limiting examples of phospholipids include: dimyristoylphosphatidylcholine (DMPC), soy lecithin, dipalmitoylphosphatidylcholine (DPPC), distearoylphosphatidylcholine (DSPC), dilaurylolyphosphatidylcholine (DLPC), dioleoylphosphatidylcholine (DOPC), dilaurylolylphosphatidylglycerol (DLPG), dimyristoylphosphatidylglycerol (DMPG), dipalmitoylphosphatidylglycerol (DPPG), distearoylphosphatidylglycerol (DSPG), dioleoylphosphatidylglycerol (DOPG), dimyristoyl phosphatidic acid (DMPA), dimyristoyl phosphatidic acid (DMPA), dipalmitoyl phosphatidic acid (DPPA), dipalmitoyl phosphatidic acid (DPPA), dimyristoyl phosphatidylethanolamine (DMPE), dipalmitoyl phosphatidylethanolamine (DPPE), dimyristoyl phosphatidylserine (DMPS), dipalmitoyl phosphatidylserine (DPPS), dipalmitoyl sphingomyelin (DPSP), distearoyl sphingomyelin (DSSP), and mixtures thereof.

In embodiments, the phospholipid is l,2-dimyristoyl-sn-glycero-3-phosphatidylcholine (DMPC), and the lysolipid is l-myristoyl-2-hydroxy-sn-glycero-phosphocholine (MHPC).

In embodiments, the phospholipid is 1-palmitoyl-2-oleoyl-sn-glycero-3-phosphocholine (POPC), and the lysolipid is1-palmitoyl-2-hydroxy-sn-glycero-3-phosphocholine (PHPC).

In embodiments, when the present composition comprises (or consists essentially of, or consists of) two or more types of lipids (such as a phospholipid, or a lysolipid), the weight ratio of two types of phospholipids ranges from about 1:10 to about 10:1, including about 1:9, about 1:8, about 1:7, about 1:6, about 1:5, about 1:4, about 1:3, about 1:2, about 1:1, about 2:1, about 3:1, about 4:1, about 5:1, about 6:1, about 7:1, about 8:1, about 9:1, to about 10:1, including all values and ranges therebetween.

In embodiments, the HDL-derived nanoparticles comprise DMPC, and MHPC, and the weight ratio of DMPC to MHPC may range from about 1: 10 to about 10: 1, from about 2:1 to about 4:1, from about 1:1 to about 5:1, from about 2:1 to about 5:1, from about 6:1 to about 10:1, from about 7:1 to about 10:1, from about 8:1 to about 10:1, from about 7:1 to about 9:1, or from about 8:1 to about 9:1. The weight ratio of DMPC to MHPC may be about 1:10, about 1:9, about 1:8, about 1:7, about 1:6, about 1:5, about 1:4, about 1:3, about 1:2, about 1:1, about 2:1, about 3:1, about 4:1, about 5:1, about 6:1, about 7:1, about 8:1, about 9:1, or about 10:1, including all values and ranges therebetween.

In embodiments, the HDL-derived nanoparticles comprise POPC and PHPC, and the weight ratio of POPC to PHPC may range from about 1: 10 to about 10: 1, from about 2: 1 to about 4:1, from about 1:1 to about 5:1, from about 2:1 to about 5:1, from about 6:1 to about 10:1, from about 7:1 to about 10:1, from about 8:1 to about 10:1, from about 7:1 to about 9:1, or from about 8:1 to about 9:1. The weight ratio of POPC to PHPC may be about 1:10, about 1:9, about 1:8, about 1:7, about 1:6, about 1:5, about 1:4, about 1:3, about 1:2, about 1:1, about 2:1, about 3:1, about 4:1, about 5:1, about 6:1, about 7:1, about 8:1, about 9:1, or about 10:1.

In embodiments, the phospholipids in nanoparticles of the present disclosure comprise (or consists essentially of, or consists of) a mixture of a two-chain diacyl- phospholipid and a single chain acyl-phospholipid/lysolipid.

In embodiments, the high-density lipoprotein (HDL)-derived nanoparticle comprises apoA-I or a peptide mimetic of apoA-I, and a phospholipid. In embodiments, the high-density lipoprotein (HDL)-derived nanoparticle comprises apoA-I or a peptide mimetic of apoA-I, a phospholipid, and a compound of Formula (I), (II), (II-1), (II-2), (II-A), (IIA-1) or (IIA-2).

In embodiments, the high-density lipoprotein (HDL)-derived nanoparticle comprises i) apoA-I or a peptide mimetic of apoA-I; ii) a phospholipid; iii) a lysolipid, and iv) cholesterol. In embodiments, the high-density lipoprotein (HDL)-derived nanoparticle comprises i) apoA-I or a peptide mimetic of apoA-I; ii) a phospholipid; iii) a lysolipid, iv) cholesterol and a compound of Formula (I), (II), (II-1), (II-2), (II-A), (IIA-1) or (IIA-2)..

In embodiments, the high-density lipoprotein (HDL)-derived nanoparticle comprises i) apoA-I or a peptide mimetic of apoA-I; ii) a phospholipid; and iii) cholesterol. In embodiments, the high-density lipoprotein (HDL)-derived nanoparticle comprises i) apoA-I or a peptide mimetic of apoA-I; ii) a phospholipid; iii) cholesterol, and a compound of Formula (I), (II), (II-1), (II-2), (II-A), (IIA-1) or (IIA-2).

In embodiments, the high-density lipoprotein (HDL)-derived nanoparticle comprises i) apoA-I or a peptide mimetic of apoA-I; ii) a phospholipid; iii) a lysolipid, iv) a hydrophobic matrix core and v) cholesterol. In embodiments, the high-density lipoprotein (HDL)-derived nanoparticle comprises i) apoA-I or a peptide mimetic of apoA-I; ii) a phospholipid; iii) a lysolipid, iv) a hydrophobic matrix core v) cholesterol and a compound of Formula (I), (II), (II-1), (II-2), (II-A), (IIA-1) or (IIA-2).

In embodiments, the high-density lipoprotein (HDL)-derived nanoparticle comprises i) apoA-I or a peptide mimetic of apoA-I; ii) a phospholipid; iii) a lysolipid, iv) a triglyceride v) cholesterol, and a compound of Formula (I), (II), (II-1), (II-2), (II-A), (IIA-1) or (IIA-2).

In embodiments, the structure and properties of the HDL-derived nanoparticles (e.g., particle size, rigidity, viscosity, loading, etc.) can be modified by incorporating a hydrophobic matrix. As used herein, hydrophobic matrix refers to a core or filler or structural modifier of the nanobiologic. Non-limiting examples of suitable hydrophobic matrix molecules include, triglycerides, fatty acid esters, hydrophobic polymers, sterol esters, or combinations thereof.

For example, the inclusion of one or more triglycerides and/or one or more polymers in the nanoparticles disclosed herein, may facilitate modulation of nanoparticle size (e.g., from about 10 nm to over 100 nm) and shape (from discoisal to spherical). In turn, the size, rigidity, and viscosity of the HDL-derived nanoparticle may also affect loading and biodistribution. In a non-limiting example, a HDL-derived nanoparticle comprising phospholipids and apoA-I may have a diameter of about 10nm to about 50nm, and adding a hydrophobic matrix molecule (such as triglycerides), swells the HDL-derived nanoparticle from a minimum of about 10 nm to at least about 30 nm. Adding more triglycerides can further increase the diameter of the HDL-derived nanoparticle to at least 50 nm, at least 75 nm, at least 100 nm, at least 150 nm, at least 200 nm, at least 300 nm, and up to 400 nm, including all values and ranges therebetween.

Any suitable suitable synthetic or natural fatty acid or fatty acid ester, known in the art are contemplated for use in the HDL-derived nanoparticles of the present disclosure. Non-limiting examples of fatty acids of use include: arachidonic acid, oleic acid, arachidic acid, lauric acid, sad, capric acid, myristic acid, Palmic acid, stearic acid, linoleic acid, linolenic acid, dicaprate, three decanoins, glycerin mono-fatty acid ester, Dilaurin, 1-Sunsoft 767, laurocapram (1-dodecyl-aza-cycloheptane-2-ketone), acylcarnitines, acyl group choline or C ₁-C ₁₀arrcostab (such as isopropyl myristate IPM), monoglyceride, diglyceride or its pharmaceutically acceptable salt.

Any suitable synthetic or natural triglycerides, known in the art are contemplated for use in the HDL-derived nanoparticles of the present disclosure. Non-limiting examples of triglycerides of use include: tricaprylin, tristearin, triolein, tripalmitin, 1,2-dipalmitoolein, 1,3-dipalmitoolein, 1-palmito-3-stearo-2-olein, 1-palmito-2-stearo-3-olein, 2-palmito-1-stearo-3-olein, trilinolein, 1,2-dipalmitolinolein, 1-palmito-dilinolein, 1-stearo-dilinolein, 1,2-diacetopalmitin, 1,2-distearo-olein, 1,3-distearo-olein, trimyristin, trilaurin and combinations thereof. Suitable triglycerides may be added to the present compositions in neat form. Additionally, or alternatively, oils and/or processed oils containing suitable triglycerides may be added to the compositions. Non-limiting examples of oils include coconut oil, corn germ oil, olive oil, palm seed oil, cottonseed oil, palm oil, rapeseed oil, sunflower oil, whale oil, soybean oil, peanut oil, linseed oil, tall oil, and combinations thereof.

The hydrophobic polymer or polymers may be selected from the group of polymers approved for human use (i.e. biocompatible and FDA-approved). Such polymers include, for example, but are not limited to the following polymers, derivatives of such polymers, co-polymers, block co-polymers, branched polymers, and polymer blends: polyalkenedicarboxlates, polyanhydrides, poly(aspartic acid), polyamides, polybutylenesuccinates (PBS), polybutylenesuccinates-co-adipate (PBSA), poly(ε-caprolactone) (PCL), polycarbonates including poly-alkylene carbonates (PC), polyesters including aliphatic polyesters and polyesteramides, polyethylenesuccinates (PES), polyglycolides (PGA), polyimines and polyalkyleneimines (Pl, PAI), polylactides (PLA (polylactic acid), PLLA, PDLLA), polylactic-co-glycolic acid (PLGA), poly(l-lysine), polymethacrylates, polypeptides, polyorthoesters, poly-p-dioxanones (PPDO), (hydrophobic) modifiedpolysaccharides, polysiloxanes and poly-alkyl-siloxanes, polyureas, polyurethanes, and polyvinyl alcohols, and biodegradable polyalkyl-cyanoacrylate.

In embodiments of the HDL-derived nanoparticle of the present disclosure, the addition of cholesterol to the nanoparticle carrier stabilizes the composition and improves entrapment efficiency. Typically, the HDL-derived nanoparticle comprises from about 1 mol% to about 100 mol% of cholesterol relative to phospholipid (e.g., relative to DMPC), including about 1% mol%, about 2 mol%, about 3 mol%, about 4 mol%, about 5 mol%, about 6 mol%, about 7 mol%, about 8 mol%, about 9 mol%, about 10 mol %, about 11 mol%, about 12 mol%, about 13 mol%, about 14 mol%, about 15 mol%, about 16 mol%, about 17 mol%, about 18 mol%, about 19 mol%, about 20 mol%, about 21 mol%, about 22 mol%, about 23 mol%, about 24 mol%, about 25 mol%, about 26 mol%, about 27 mol%, about 28 mol%, about 29 mol%, about 30 mol%, about 35 mol%, about 40 mol%, about 45 mol%, about 50 mol%, about 55 mol%, about 60 mol%, about 65 mol%, about 70 mol%, about 75 mol%, about 80 mol%, about 85 mol%, about 90 mol%, about 95 mol%, to about 100 mol% (i.e. 1:1 mol/mol mixture of of cholesterol and phospholipd (e.g. DMPC) including all ranges and values therebetween. In embodiments, the HDL-derived nanoparticle comprises from about 1 mol% to about 30 mol% cholesterol. In embodiments, the HDL-derived nanoparticle comprises from about 15 mol% to about 25 mol% cholesterol, relative to phospholipid. In embodiments, the HDL-derived nanoparticle comprises from about 20 mol% cholesterol, relative to phospholipid. In embodiments, the HDL-derived nanoparticle comprises from about 10 mol% to about 35 mol% cholesterol, relative to phospholipid. In embodiments, the HDL-derived nanoparticle comprises from about 15 mol% to about 30 mol% cholesterol, relative to phospholipid. In embodiments, the HDL-derived nanoparticle comprises from about 15 mol% to about 25 mol% cholesterol, relative to phospholipid. In embodiments, the HDL-derived nanoparticle comprises from about 28 mol% to about 23 mol% cholesterol, relative to phospholipid. In embodiments, the HDL-derived nanoparticle comprises from about 20 mol% to about 27 mol% cholesterol, relative to phospholipid.

In embodiments, the HDL-derived nanoparticle is cholesterol free. In embodiments, the molar ratio of cholesterol: phospholipid, in the HDL-derived nanoparticle is about 0: 1, about 0.025: 1, about 0.05: 1, about 0.075: 1, about 0.1: 1, about 0.125: 1, about 0.15: 1, about 0.175: 1, about 0.2: 1, about 0.225: 1, about 0.25: 1, about 0.275: 1, about 0.3: 1, about 0.325: 1, about 0.35: 1, about 0.375: 1, about 0.4: 1, about 0.425: 1, about 0.45: 1, about 0.475:1 or about 0.5: 1, including all values therebetween. In embodiments, the molar ratio of cholesterol: phospholipids ranges from about 0: 1 to about 0.5: 1, including about 0: 1, about 0.025: 1, about 0.05: 1, about 0.075: 1, about 0.1: 1, about 0.125: 1, about 0.15: 1, about 0.175: 1, about 0.2: 1, about 0.225: 1, about 0.25: 1, about 0.275: 1, about 0.3: 1, about 0.325: 1, about 0.35: 1, about 0.375: 1, about 0.4: 1, about 0.425: 1, about 0.45: 1, about 0.475:1 to about 0.5: 1, including all ranges therebetween. In embodiments, the molar ratio of cholesterol: phospholipids ranges from about 0.05: 1 to about 0.25: 1. In embodiments, the molar ratio of cholesterol is about 0.2: 1.

In embodiments, the HDL-derived nanoparticle comprises one or more phospholipids and cholesterol in a molar ratio in the range of about 1:0.05 to about 1:0.25. In embodiments, the HDL-derived nanoparticle comprises one or more phospholipids and cholesterol in a molar ratio of about 1: 0.2.

In embodiments, the weight percentage of cholesterol ranges from about 0% (w/w) to about 15% (w/w) of the nanoparticle, lipid, or composition, including from about 1% (w/w), about 1.5% (w/w), about 2% (w/w), about 2.5% (w/w), about 3% (w/w), about 3.5% (w/w), about 4% (w/w), about 4.5% (w/w), about 5% (w/w), about 5.5% (w/w), about 6% (w/w), about 6.5% (w/w), about 7% (w/w), about 7.5% (w/w), about 8% (w/w), about 8.5% (w/w), about 9% (w/w), about 9.5% (w/w), about 10% (w/w), about 10.5% (w/w), about 11% (w/w/), about 11.5% (w/w), about 12% (w/w), about 12.5% (w/w), about 13% (w/w), about 13.5% (w/w), about 14% (w/w), about 14.5% (w/w), to about 15% (w/w). In embodiments, the weight percentage of cholesterol ranges from about 0% (w/w) to about 15%, (w/w) of the nanoparticle, lipid, or composition, including from about 1% (w/w), about 1.5% (w/w), about 2% (w/w), about 2.5% (w/w), about 3% (w/w), about 3.5% (w/w), about 4% (w/w), about 4.5% (w/w), about 5% (w/w), about 5.5% (w/w), about 6% (w/w), about 6.5% (w/w), about 7% (w/w), about 7.5% (w/w), about 8% (w/w), about 8.5% (w/w), about 9% (w/w), about 9.5% (w/w), about 10% (w/w), about 10.5% (w/w), about 11% (w/w/), about 11.5% (w/w), about 12% (w/w), about 12.5% (w/w), about 13% (w/w), about 13.5% (w/w), about 14% (w/w), about 14.5% (w/w), to about 15% (w/w). In embodiments, the weight percentage is the weight percetage of cholesterol relative to phospholipids. In embodiments, the weight percentage of cholesterol ranges from about 1 to 10% cholesterol (w/w%) of the composition. the weight percentage of cholesterol ranges from about 2 to 8% cholesterol (w/w%) of the composition. In embodiments, the weight percentage of cholesterol ranges from about 3.5 to 7.5% cholesterol (w/w%) of the composition. In embodiments, the weight percentage of cholesterol ranges from about 5 to 10% cholesterol (w/w%) of the composition. In embodiments, the weight percentage of cholesterol is about 3.6 (w/w%) of the composition. In embodiments, the weight percentage of cholesterol is about 7.2 (w/w%) of the composition. In embodiments, the weight percentage of cholesterol is about 5.9 (w/w%) of the composition.

In embodiments, the size and circulating time of the nanoparticles can be modulated, for example, by controlling the ratio of lipids-to-APOAl and the ratio of lipids to polymer or lipids to triglyceride.

In embodiments, the HDL-derived nanoparticle comprises from about a 5:1 to 1000:1 ratio (e.g., on a molar basis) of phospholipids: apoA-I or a mimetic of apoA-I, including about 5:1, about 10:1, about 20:1, about 30:1, about 40:1, about 50:1, about 60:1, about 70:1, about 80:1, about 90:1, about 100:1, about 110:1, about 120:1, about 130:1, about 140:1, about 150:1, about 160:1, abot 170:1, about 180:1, about 190:1, about 200:1, about 210:1, about 220:1 about 230:1, about 240:1, about 250:1, about 260:1, about 270:1, about 280:1, about 290:1, about 300:1, about 310:1, about 320:1, about 330:1, about 340:1, about 350:1, about 360:1, about 370:1, about 380:1, about 390:1, about 400:1, about 410:1, about 420:1, about 430:1, about 440:1, about 450:1, about 460:1, about 470:1, about 480:1, about 490:1, about 500:1, about 510:1 , about 520:1, about 530:1, about 540:1, about 550:1, about 560:1, about 570:1, about 580:1, about 590:1, about 600:1, about 610:1, about 620:1, about 630:1, about 640:1, about 650:1, about 660:1, about 670:1, about 680:1, about 690:1, about 700:1, about 710:1, about 720:1 about 730:1, about 740:1, about 750:1, about 760:1, about 770:1, about 780:1, about 790:1, about 800:1, about 810:1, about 820:1, about 830:1, about 840:1, about 850:1, about 860:1, about 870:1, about 880:1, about 890:1, about 900:1, about 910:1, about 920:1, about 930:1, about 940:1, about 950:1, about 960:1, about 970:1, about 980:1, about 990:1, to about 1000:1, including all subranges and values therebetween. In embodiments, the HDL-derived nanoparticle comprises from about a 10:1 to 1000:1 ratio (e.g., on a molar basis) of phospholipids: apoA-I or a mimetic of apoA-I. In embodiments, the HDL-derived the nanoparticle comprises from about a 70:1 to 125:1 ratio (e.g., on a molar basis) of phospholipids: apoA-I. In embodiments, the HDL-derived the nanoparticle comprises from about a 5:1 to 10:1 ratio (e.g., on a molar basis) of mimetic of apoA-I.

In embodiments, the HDL-derived nanoparticle comprises from about a 2:1 to 3:1 ratio by weight of phospholipids: apoA-I or a mimetic of apoA-I.

In embodiments, the HDL-derived nanoparticle comprises from about, or at least about 0.1 mol% to about 100 mol% of a compound of Formula I relative to phospholipid (e.g., DMPC), including about, or at least about 0.1 mol%, about or at least about 0.5 mol%, about or at least about 0.75 mol%, about, or at least about 1% mol%, about, or at least about 2 mol%, about, or at least about 3 mol%, about, or at least about 4 mol%, about, or at least about 5 mol%, about, or at least about 6 mol%, about, or at least about 7 mol%, about, or at least about 8 mol%, about, or at least about 9 mol%, about, or at least about 10 mol %, about, or at least about 11 mol%, about, or at least about 12 mol%, about, or at least about 13 mol%, about, or at least about 14 mol%, about, or at least about 15 mol%, about, or at least about 16 mol%, about, or at least about 17 mol%, about, or at least about 18 mol%, about, or at least about 19 mol%, about, or at least about 20 mol%, about, or at least about 21 mol%, about, or at least about 22 mol%, about, or at least about 23 mol%, about, or at least about 24 mol%, about, or at least about 25 mol%, about, or at least about 26 mol%, about, or at least about 27 mol%, about, or at least about 28 mol%, about, or at least about 29 mol%, to about, at least about 30 mol%, about or at least about or at least about 35 mol%, about or at least about 40 mol%, about or at least about 45 mol%, about or at least about 50 mol%, about or at least about 55 mol%, about or at least about 60 mol%, about or at least about 65 mol%, about or at least about 70 mol%, about or at least about 75 mol%, about or at least about 80 mol%, about or at least about 85 mol%, about or at least about 90 mol%, about or at least about 95 mol%, to about or at least about 100 mol% (1:1 mol/mol mixture of compound and phospholipd (e.g. DMPC)), including all ranges and values therebetween. In embodiments, the HDL-derived nanoparticle comprises from about 10 mol% to about 30 mol% of a compound of Formula I relative to phospholipid. In embodiments, the HDL-derived nanoparticle comprises from about 12 mol% to about 25 mol% compound, relative to phospholipid.

In embodiments, the nanoparticle size ranges from about 5 nm to about 500 nm in diameter, including about 5 nm, about 10 nm, about 15 nm, about 20 nm, about 25 nm, about 30 nm, about 35 nm, about 40 nm, about 50 nm, about 55 nm, about 60 nm, about 65 nm, about 70 nm, about 75 nm, about 80 nm, about 85 nm, about 90 nm, about 95 nm, to about 100 nm, about 110 nm, about 120 nm, about 130 nm, about 140 nm, about 150 nm about 160 nm, about 170 nm, about 180 nm, about 190 nm, about 200 nm, about 210 nm, about 220 nm, about 230 nm, about 240 nm, about 250 nm, about 260 nm, about 270 nm, abou 280 nm, about 290 nm, about 300 nm, about 310 nm, about 320 nm, about 330 nm, about 340 nm, about 350 nm, about 360 nm, about 370 nm, about 380 nm, about 390 nm, about 400 nm, about 410 nm, about 420 nm, about 430 nm, about 440 nm, about 450 nm, about 460 nm, about 470 nm, about 480 nm, about 490 nm, to about 500 nm, including all ranges and values therebetween. In embodiments, the nanoparticle size is less than about 50 nm. In embodiments, the nanoparticle size is about 50 nm to about 100 nm, or about 5 nm to about 30 nm. In embodiments, the nanoparticle sizes are measured by dynamic light scattering (DLS). In embodiments, to target immune cells in tissue with limited access to circulation, nanoparticles having long blood half-lives and small size (<50 nm) may be used. In embodiments, to target immune cells in well-perfused tissues, nanoparticles having short blood half-lives and large size (about 100 nm) may be used. These tissues include spleen, liver, kidney, lungs, and bone marrow.

In embodiments, the HDL-derived nanoparticle is discoidal in shape. In embodiments, the HDL-derived nanoparticle is spherical in shape. In embodiments the HDL-derived nanoparticle morphology is visualized by transmission electron microscopy (TEM).

In embodiments, the length of the HDL-derived nanoparticle is about 5 to about 100 nm in length, including about 5 nm, about 10 nm, about 15 nm, about 20 nm, about 25 nm, about 30 nm, about 35 nm, about 40 nm, about 50 nm, about 55 nm, about 60 nm, about 65 nm, about 70 nm, about 75 nm, about 80 nm, about 85 nm, about 90 nm, about 95 nm, to about 100 nm in length, including all ranges and values therebetween. In embodiments, the HDL-derived nanoparticle is about 10 nm to 80 nm in length. In embodiments, the HDL-derived nanoparticle is about 15 nm to 50 nm in length. In embodiments, the HDL-derived nanoparticle is longer than about 10 nm, or longer than about 15 nm. In embodiments, the HDL-derived nanoparticle has a thicknesses of about 1 nm to 10 nm, including about 1 nm, about 2 nm, about 3 nm, about 4 nm, about 5 nm, about 6 nm, about 7 nm, about 8 nm, about 9 nm, to about 10 nm, including all ranges and values therebetween. In embodiments, the thicknesses of the HDL-particles are about 1 to 10 nm, or 2 to 7 nm, or 3 to 6 nm. In embodiments, the dimensions (e.g., length and thickness) are recorded by cryo-TEM. In embodiments, the HDL-particles have a worm-like morphology by cryo-TEM.

In embodiments, the HDL-derived nanoparticle is discoidal in shape with a diameter between about 5 nm to about 50 nm (e.g., as measured by dynamic light scattering (DLS)), including about 5 nm, about 10 nm, about 15 nm, about 20 nm, about 25 nm, about 30 nm, about 40 nm, to about 50 nm, including all subranges and values therebetween. In embodiments, the nanodisc is about 5 nm to about 30 nm in diameter.

In embodiments, the HDL-derived nanoparticle is spherical in shape with a diameter between about 10 nm to about 400 nm in diameter (e.g., as measured by dynamic light scattering (DLS)), including about 10 nm, about 15 nm, about 20 nm, about 30 nm, about 40 nm, about 50 nm, about 60 nm, about 70 nm, about 80 nm, about 100 nm, about 110 nm, about 120 nm, about 130 nm, about 140 nm, about 150 nm, about 160 nm, about 170 nm, about 180 nm, about 190 nm, about 200 nm, about 210 nm, about 220 nm, about 230 nm, about 240 nm, about 250 nm, about 260 nm, about 270 nm, about 280 nm, about 290 nm, about 300 nm, about 310 nm, about 330 nm, about 340 nm, about 350 nm, about 360 nm, about 370 nm, about 380 nm, about 390 nm, to about 400 nm in diameter, including all values and ranges therebetween. In embodiments, the nanosphere is between about 15 nm to about 250 nm in diameter. In embodiments, the nanosphere is between about 30 nm, about about 100 nm in diameter.

Stability of the HDL-derived nanoparticle can be assessed by performing DLS measurements. In embodiments, the HDL-derived nanoparticle is stable for at least about 1 week, or at least about 2 weeks, or at least about 5 weeks e.g., by DLS.

In embodiments, the nanobiologic composition promotes a hyper-responsive innate immune response in the patient in need thereof. In embodiments, the hyper-responsive innate immune response is promoted for at least about 7 to about 30 days. In embodiments, the hyper-responsive innate immune response is promoted for at least 30 to 100 days. In embodiments, the hyper-responsive innate immune response is promoted for more than 100 days and up to 3 years. In embodiments, the nanobiologic composition is administered once and wherein the hyper-responsive innate immune response is promoted for at least 30 days. In embodiments, the nanobiologic composition is administered at least once per day in each day of a multiple-dosing regimen, and wherein the hyper-responsive innate immune response is promoted for at least 30 days.

Production methods can prepare uniform size HDL-derived nanoparticles, or a non-uniform sized mixture of HDL-derived nanoparticles, either by not filtering, or by preparing a range of different sized HDL-derived nanoparticles and re-combining them in a post-production step. The larger the size of the HDL-derived nanoparticles, the more drug can be incorporated. However, larger sizes e.g. >120 nm, can limit, prevent or slow diffusion of the HDL-derived nanoparticles into the tissues of the patient being treated. Smaller HDL-derived nanoparticles do not hold as much drug per particle, but are able to access the bone marrow, blood, or spleen, or other localized tissue affected by trained immunity, e.g. myeloid cells, myeloid progenitor cells, and hematopoietic stem cells in the bone marrow, blood and/or spleen, and so forth (biodistribution).

Using a non-uniform mixture of nanoparticles sizes in a single administration or regimen can produce an immediate reduction in innate immune hyper-responsiveness, and simultaneously produce a durable, long-term reduction in innate immune hyper-responsiveness that can last days, weeks, months, and years, wherein the nanobiologic has reversed, modified, or reregulated the metabolic, epigenetic, and inflammasome pathways of the hematopoietic stem cells (HSC), the common myeloid progenitors (CMP), and the myeloid cells such as monocytes, macrophages and other short-lived circulating cells.

In embodiments, the maximum loading capacity of the HDL-derived nanoparticle can be determined dividing the volume of the interior of the HDL-derived nanoparticle by the volume of a drug-load spheroid.
Particle: assume a 100nm spherical particle having 2.2 nm-3.0 nm phospholipid wall, yielding a 94 nm diameter interior with volume (L) @ 4/3n(r)3.
Drug: assume STIMULATOR at 12x12x35 Angstrom or as a cylinder 1.2xl.2x3.5 nm, where multiple drug molecule cylinders, e.g. seven or nine, etc. could assume a 3.5nm diameter spheroid having a radius of 1.75nm Vol(small) @ 4/3n(r)3.
Maximum Loading Capacity (calc): -487k 3.5 nm spheroids within a 100nm particle.

### Formulations

When employed as pharmaceuticals, the compounds and HDL-derived nanoparticles of the present disclosure are typically administered in the form of a pharmaceutical composition. Such compositions can be prepared in a manner well known in the pharmaceutical art and comprise at least one active compound. In embodiments, the pharmaceutical composition comprises a nanobiologic composition of the present disclosure, and a pharmaceutically acceptable carrier.

Generally, the compounds of this invention are administered in a pharmaceutically effective amount. The amount of the compound actually administered will typically be determined by a physician, in the light of the relevant circumstances, including the condition to be treated, the chosen route of administration, the actual compound -administered, the age, weight, and response of the individual patient, the severity of the patient's symptoms, and the like.

The pharmaceutical compositions of this invention can be administered by a variety of routes including oral, rectal, intraocular, transdermal, subcutaneous, intravenous, intramuscular, intraperitoneal, intradermal, directly into cerebrospinal fluid, intratracheal, and intranasal. Depending on the intended route of delivery, the compounds of this invention are preferably formulated as either injectable or oral compositions or as salves, as lotions or as patches all for transdermal administration. In embodiments, the composition is administered intraveneously or intraarterially.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, a compound as described herein is usually a minor component (from about 0.1 to about 50% by weight or preferably from about I to about 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable carriers known in the art. As before, the active compound in such compositions is typically a minor component, often being from about 0.05 to 10% by weight with the remainder being the injectable carrier and the like.

Transdermal compositions are typically formulated as a topical ointment or cream containing the active ingredient(s), generally in an amount ranging from about 0.01 to about 20% by weight, preferably from about 0.1 to about 20% by weight, preferably from about 0.1 to about 10% by weight, and more preferably from about 0.5 to about 15% by weight. When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or the formulation.

Nanoparticles described herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

For injection, nanoparticles described herein can be provided in an injection grade saline solution, in the form of an injectable liposome solution, slow-release polymer system or the like.

Nanoparticles described herein can also be administered in sustained release forms or from sustained release drug delivery systems. A description of representative sustained release materials can be found in Remington's Pharmaceutical Sciences.

### Methods

Provided herein are compounds for use in methods of treating a subject susceptible to or afflicated with immune-related diseases and conditions, including, for example, immunoparalysis in sepsis and infections, cell proliferation disorders (such as cancer), and other diseases and conditions caused by defective trained immunity.

In embodiments, the present disclosure provides compounds for use in methods for treating a cell-proliferation disorder, comprising administering to a subject in need thereof a therapeutically effective amount of a nanobiologic composition comprising a high-density lipoprotein (HDL)-derived nanoparticle comprising a compound of the present disclosure (such as a compound of Formula I). In embodiments, the compounds, compositions provided herein are useful for treating cancer by inducing trained immunity.

In embodiments, the cell proliferation disorder is cancer. In embodiments, the cancer is one or more of the following cancers: advanced malignancy, amyloidosis, neuroblastoma, meningioma, hemangiopericytoma, multiple brain metastase, glioblastoma multiforms, glioblastoma, brain stem glioma, poor prognosis malignant brain tumor, malignant glioma, recurrent malignant giolma, anaplastic astrocytoma, anaplastic oligodendroglioma, neuroendocrine tumor, rectal adenocarcinoma, Dukes C & D colorectal cancer, unresectable colorectal carcinoma, metastatic hepatocellular carcinoma, Kaposi's sarcoma, karotype acute myeloblastic leukemia, Hodgkin's lymphoma, non-Hodgkin's lymphoma, cutaneous T-Cell lymphoma, cutaneous B-Cell lymphoma, diffuse large B-Cell lymphoma, low grade follicular lymphoma, malignant melanoma, malignant mesothelioma, malignant pleural effusion mesothelioma syndrome, peritoneal carcinoma, papillary serous carcinoma, gynecologic sarcoma, soft tissue sarcoma, scelroderma, cutaneous vasculitis, Langerhans cell histiocytosis, leiomyosarcoma, fibrodysplasia ossificans progressive, hormone refractory prostate cancer, resected high-risk soft tissue sarcoma, unrescectable hepatocellular carcinoma, Waldenstrom's macroglobulinemia, smoldering myeloma, indolent myeloma, fallopian tube cancer, androgen independent prostate cancer, androgen dependent stage IV non-metastatic prostate cancer, hormone-insensitive prostate cancer, chemotherapyinsensitive prostate cancer, papillary thyroid carcinoma, follicular thyroid carcinoma, medullary thyroid carcinoma, and leiomyoma. In embodiments, the cancer is selected from the group consisting of bladder cancer, cancer of the blood vessels, bone cancer, brain cancer, breast cancer, cervical cancer, chest cancer, colon cancer, endometrial cancer, esophageal cancer, eye cancer, head cancer, kidney cancer, liver cancer, cancer of the lymph nodes, lung cancer, mouth cancer, neck cancer, ovarian cancer, pancreatic cancer, prostate cancer, rectal cancer, colorectal cancer, skin cancer, stomach cancer, testicular cancer, throat cancer, thyroid cancer, urothelial cancers, and uterine cancer. In embodiments, the cancer is selected from the group consisting of breast cancer, prostate cancer, melanoma, colorectal cancer, lung cancer, pancreatic cancer, and glioblastoma. In embodiments, the cancer is metastatic. In embodiments, the cancer is refractory or resistance to chemotherapy or radiation; in particular, refractory to thalidomide.

In embodiments, the cancer is selected from the group consisting of bladder cancer, cancer of the blood vessels, bone cancer, brain cancer, breast cancer, cervical cancer, chest cancer, colon cancer, endometrial cancer, esophageal cancer, eye cancer, head cancer, kidney cancer, liver cancer, cancer of the lymph nodes, lung cancer, mouth cancer, neck cancer, ovarian cancer, pancreatic cancer, prostate cancer, rectal cancer, colorectal cancer, skin cancer, stomach cancer, testicular cancer, throat cancer, thyroid cancer, urothelial cancers, and uterine cancer.

In embodiments, the cancer is selected from the group consisting of breast cancer, prostate cancer, melanoma, colorectal cancer, lung cancer, pancreatic cancer, and glioblastoma.

In embodiments, the present disclosure provides compounds for use in methods for treating sepsis comprising administering to a subject in need thereof a therapeutically effective amount of a nanobiologic composition of the present disclosure. In embodiments, the patient has sepsis associated with a bacterial, viral or fungal infection of the lungs, abdomen, kidney, or bloodstream.

The compounds of the present disclosure and their carriers disclosed herein may be used to augment immune responses. Accordingly, disclosed herein are methods of inducing immune responses comprising administering an immunogenic composition to a subject, wherein the composition comprises (i) at least one antigen and (ii) a compound disclosed herein, optionally in a nanoparticle carrier, such as a HDL-derived nanoparticle or liposome.

The antigens are typically from pathogens, though neoantigens from subjects having cancer may also be used. Illustrative pathogen antigens may be from a virus, a bacteria, a parasite or a yeast. In aspects, the antigen may be a secreted from a pathogen; for example, an exotoxin or an endotoxin.

Exemplary viruses include Adenovirus, Adeno-associated virus (AAV), Chikungunya, Dengue, Influenza, Ebola, Epstein-Barr, Hanta, Hepatitis (e.g., Hepatitis A, B, C, D, E), CMV, HPV (e.g., one or more of HPV1-18), Coronavirus, (e.g., SARS, MERS, COVID-19), Polio, Rabies, Zika. Exemplary bacteria include Vibrio cholerae, E. coli, Salmonella spp., N. gonorrheae, N. meningitidis, Streptococcus pyogenes, Mycobacterium tuberculosis, Legionella pneumophila, Brucella bortus, and Listeria monocytogenes.

The antigen may be, for example, a polypeptide, including a glycosylated peptide, or a carbohydrate. In aspects, the immunogenic composition may contain a nucleic acid that encodes an antigen, typically polypeptide that is transcribed and/or translated from the nucleic acid. The nucleic acid may be a DNA or an RNA, or a derivative of DNA or of RNA. Common derivatives of RNA include covalent modification to the molecule to enhance stability and/or expression. In aspects, the nucleic acid encoding the polypeptide may be within a plasmid or a viral vector, such as adenoviral vectors, adeno-associated virus vectors, baculoviral vectors, lentiviral vectors, and the like.

In aspects, the administration may be preventative; for example, to vaccinate the subject prior to exposure to the pathogen. In other aspects, the administration may be a treatment; for example, inducing an immune response against a tumor carrying neo-antigens in a subject suffering from cancer. In embodiments, the nanobiologic composition is administered in a treatment regimen comprising two or more doses to the patient to generate an accumulation of drug in myeloid cells, myeloid progenitor cells, and hematopoietic stem cells in the bone marrow, blood and/or spleen.

In embodiments, the nanobiologic composition is administered intravenously or intra-arterially.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Oral dose levels range from about 0.01 to about 20 mg/kg of the compound of the invention, including all ranges and values there between. For example, dose levels range from about 0.1 to about 10 mg/kg or from about 1 to about 5 mg/kg.

Transdermal doses are generally selected to provide similar or lower blood levels than are achieved using injection doses. Modes of administration suitable for mucosal sites are also envisioned herein and include without limitation: intra-anal swabs, enemas, intranasal sprays, and aerosolized or vaporized compounds and/or compositions for delivery to the lung mucosa. One of skill in the art would choose an appropriate delivery models based on a variety of parameters, including the organ or tissue site in a patient with a disease or condition that is most severely affected by the disease or condition.

The compounds of this invention can be administered as the sole active agent or they can be administered in combination with one or more additional pharmaceutical agents, including other compounds that demonstrate the same or a similar therapeutic activity and are determined to safe and efficacious for such combined administration. In embodiments, the additional pharmaceutical agent is an inhibitor of a checkpoint protein. In embodiments, the methods provided herein further comprise co-administering a cancer drug as a combination therapy with the nanobiologic composition.

A compound or composition described herein can be provided in a kit. In some embodiment the kit includes (a) a compound described herein, or a composition that includes a compound described herein (wherein, e.g., the compound can be an NOD2 modulator described herein), and, optionally (b) informational material. The informational material can be descriptive, instructional, marketing or other material that relates to the methods described herein and/or the use of a compound or compositoin described herein for the methods described herein. In embodiments, the informational material can include information about production of the compound. In embodiments, the informational material relates to methods for administering the compound. In embodiments, the informational material can include instructions to administer a compound or composition described herein in a suitable manner to perform the methods described herein, e.g., in a suitable dose, dosage form, or mode of administration (e.g., a dose, dosage form, or mode of administration described herein). In embodiments, the informational material can include instructions to administer a compound described herein to a suitable subject, e.g., a human, e.g., a human having or at risk for a disorder described herein.

The kit can include one or more containers for the composition containing a compound or composition described herein. In embodiments, the kit contains separate containers, dividers or compartments for the composition and informational material. For example, the composition can be contained in a bottle, vial, or syringe, and the informational material can be contained in a plastic sleeve or packet. In embodiments, the separate elements of the kit are contained within a single, undivided container. For example, the composition is contained in a bottle, vial or syringe that has attached thereto the informational material in the form of a label. In embodiments, the kit includes a plurality (e.g., a pack) of individual containers, each containing one or more unit dosage forms (e.g., a dosage form described herein) of a compound or composition described herein. For example, the kit includes a plurality of syringes, ampules, foil packets, or blister packs, each containing a single unit dose of a compound described herein. The containers of the kits can be air tight, waterproof (e.g., impermeable to changes in moisture or evaporation), and/or light-tight.

Also provided herein is a process for manufacturing a nanobiologic composition of the present disclosure, the process comprising:
a) forming a lipid film comprising: i) a compound of the present disclosure; ii) one or more phospholipids; optionally iii) a hydrophobic matrix comprising one or more triglycerides, fatty acid esters, hydrophobic polymers, or sterol esters, or a combination thereof; and optionally iv) cholesterol; under conditions effective to form the lipid film; and
b) dissolving the lipid film in a solvent to form a lipid solution; and contacting the lpid solution with apoA-I or a peptide mimetic of apoA-I under conditions effective to form a HDL-derived nanoparticle comprising a compound of the present disclosure.

In embodiments, provided herein is a nanobiologic composition prepared according to the methods disclosed herein.

### EXAMPLES

The therapeutic agents described herein and nanoparticles comprising same may be prepared from known or commercially available starting materials and reagents by one skilled in the art of organic synthesis.

### Materials and methods

All chemicals were purchased from commercial sources and used without further purification unless specified. N-methylmorpholine was redistilled, collecting the fraction from 110 °C to 112 °C. Cholesterol azidoacetate was synthesized according known procedures (RSC Adv. 2015, 5, 12094), as was 1-azidooctadecane. Dry solvents were obtained with an MBRAUN Solvent Purification System (MB-SPS). Toluene was dried over 4Å molecular sieves before use. Glassware used for reactions carried out under argon atmosphere was dried with a heat gun prior to use. Thin-layer chromatography (TLC) was performed using 60-F254 silica gel plates from Merck and visualized by UV light at 254 nm, permanganate staining and/or cerium molybdate (CeMo) staining. Normal and reversed-phase automated column chromatography was conducted on a Biotage Isolera One or Grace Reveleris X2 Flash Chromatography System using Biotage Sfär Silica, Büchi FlashPure ID Silica or Büchi FlashPure ID C18 columns. Elution gradients are specified in column volumes (CVs). Non-stabilized THF was used for the water/THF gradients.

NMR spectra were recorded on Bruker 400 MHz Ultrashield spectrometer (400 MHz for 1H NMR). Deuterated solvents used are indicated in each case. Chemical shifts (δ) are expressed in ppm and are referred to the residual peak of the solvent. Peak multiplicity is abbreviated as s: singlet; d: doublet; t: triplet; dt: doublet of triplets; ddt: doublet of doublets of triplets; td: triplet of doublets; tt: triplet of triplets; q: quartet; ABq: AB quartet; dq: doublet of quartets; qd: quartet of doublets; sept: septet; m: multiplet; bs: broad singlet. Matrix assisted laser desorption/ionization time-of-flight (MALDI-TOF) mass spectra were obtained on a PerSeptive Biosystems Voyager DE-PRO spectrometer using α-cyano-4-hydroxycinnamic acid (CHCA) or trans-2-[3-(4-tert-butylphenyl)-2-methyl-2-propenylidene]-malononitrile (DCTB) as matrix. Gas chromatography-mass spectrometry (GC-MS) measurements were conducted on a Shimadzu GC-17A gas chromatograph with a Shimadzu AOC-20i auto injector, Shimadzu GCMS-QP5000 gas chromatograph mass spectrometer and Phenomenex Zebron ZB-35 column (l = 30 meters, ID = 0.25 mm, film thickness = 0.25 µm). High-pressure liquid chromatography mass spectrometry (HPLC-ESI-MS) experiments using a water/acetonitrile gradient were performed a Shimadzu setup with 2x LC-20 AD pumps, DGU-20A3 degasser, SIL-20AC autosampler, SPD-M20A PDA and ThermoScientific LCQ fleet MS. Column: Phenomenex Kinetex 5 um EVO C18 100Å LC (50x2.1 mm). Gradient: water/MeCN (+ 0.1% formic acid) from 5 to 100% MeCN, 0.300 mL/min. Electrospray ionization (ESI) was used to create the charges for MS-detection. HPLC-MS and HPLC-ELSD experiments with a water/THF or water/MeOH gradient were performed on a Shimadzu Nexera-i LC-2040C 3D Plus with Shimadzu LCMS-8045. Column: Alltech Alltima C18 (150x3.2mm; 5 um; no. 88383). Gradient: water/THF (+ 0.1% TFA) or water/MeOH (+ 0.1% TFA), 0.400 mL/min. This HPLC setup was also used in combination with ELSD (evaporative light scattering detection).

Alternatively, HPLC-MS(SIM) and HPLC-ELSD were performed on a Phenomenex Kinetex 5 micrometer EVO C18 100A LC-column (50x2.1 mm) employing a gradient from A to B eluent, where A = 20 mM NH₄HCO₂ in H₂O with 0.1 v/v % formic acid, and B = 2-propanol/MeCN/H₂O 85:15:5, also with 20 mM NH₄HCO₂ and 0.1 v/v % formic acid.

### Abbreviations

HPLC = high performance liquid chromatography; ELSD = evaporative light scattering detection; ESI-MS = electrospray ionization mass spectrometry; SIM = selected ion mode; NMR = nuclear magnetic resonance.

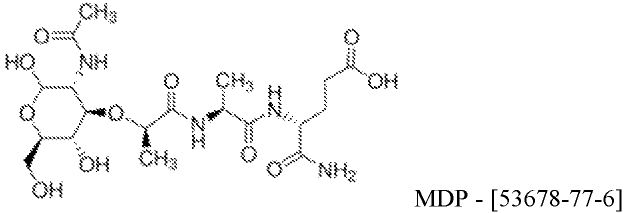

MDP = Muramyl dipeptide muramyl (or N-Acetylmuramyl-L-alanyl-D-isoglutamine) CAS [53678-77-6]. Was either prepared according to standard peptide synthesis or bought from commercial sources.

NHS = N-hydroxy succinimide; DiC or DIC = N,N'-diisopropylcarbodiimide; EDC = N-(3-dimethylaminopropyl)-N'-ethylcarbodiimide (used hydrochloride); PyBOP = (benzotriazol-1-yloxytripyrrolidinophosphonium hexafluorophosphate); SPPS = solid phase peptide synthesis.

TEA = triethyl-amine; THF = tetrahydrofuran; MeOH = methanol; DMF = dimethylformamide; FA = formic acid; TFA = trifluoro-acetic acid.

### Building blocks

### DSPE-azidoacetate (DSPE-CO-CH₂-N₃)

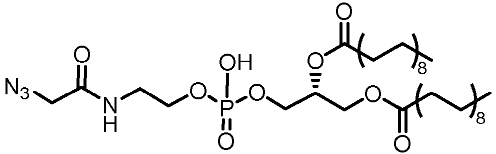

(2R)-3-(((2-Aminoethoxy)(hydroxy)phosphoryl)oxy)propane-1,2-diyl distearate (260 mg, 0.35 mmol), 2,3,5,6-tetrafluorophenyl 2-azidoacetate (prepared according to D.J. Vugts et al., Bioconjugate Chem. 2011, 22, 2072-2081; 87 mg, 0.35 mmol, 1 eq) and N,N-diisopropylethylamine (184 µL, 1.1 mmol, 3 eq) were combined in chloroform (2 mL). The mixture was stirred at 50 °C for 1 h during which the white suspension cleared. Chloroform (200 mL) was added and the organic layer was gently washed twice with 1 M HCl (100 mL). After drying with MgSO4, filtration and removal of the solvent in vacuo, the compound was purified with column chromatography (flash SiO₂) using an elution gradient of 5 % to 40 % MeOH in chloroform. This yielded pure DSPE-azidoacetate (244 mg, 0.29 mmol, 84 %) as a white solid. ¹H-NMR (400 MHz, CDCl₃/CD₃OD 9:1): δ = 5.23 (dt, J = 9.0, 4.6 Hz, 1H), 4.35 (dd, J = 12.0, 3.7 Hz, 1H), 4.22-3.99 (m, 5H), 3.95 (s, 2H), 3.53 (t, J = 5.1 Hz, 2H), 2.33 (q, J = 7.6 Hz, 4H), 1.61 (td, J = 7.4, 4.2 Hz, 4H), 1.48-1.16 (m, 56H), 0.88 (t, J = 6.7 Hz, 6H). ¹³C-NMR (101 MHz, CDCl₃): δ = 173.7, 173.4, 168.3, 69.7, 69.6, 66.1, 66.0, 65.2, 62.1, 52.5, 40.01, 39.95, 34.3, 34.2, 34.1, 32.1, 29.9, 29.80, 29.7, 29.62, 29.59, 29.50, 29.47, 29.46, 29.4, 29.30, 29.26, 25.00, 24.97, 24.9, 22.8, 14.2. ³¹P-NMR (162 MHz, CDCl₃): δ = -0.48. MALDI-TOF MS: m/z Calc. for C₄₃H₈₃N₄O₉P 830.59; Obs. [M+Na]⁺ 853.62, [M-H+2Na]⁺ 875.58.

### MDP-propargyl

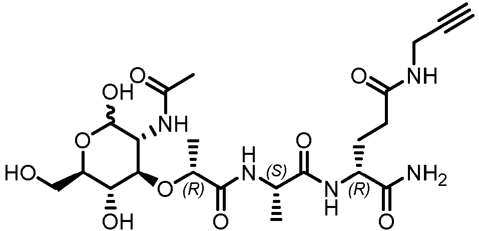

A 50 mL round-bottomed flask was filled with MDP (0.113 g, 0.23 mmol, 1.00 eq). The material was dissolved in dry DMF (~1.5 mL, 0.15 M) and the flask purged with argon.

EDC.HCl (0.066 g, 0.34 mmol, 1.50 eq) and N,N-diisopropylethylamine (0.050 g, 0.068 mL, 0.39 mmol, 1.70 eq) and 4-(N,N-dimethylamino)pyridine (0.0028 g, 0.023 mmol, 0.10 eq) were added, and the resulting clear solution stirred at RT for 5 min. Next, prop-2-yn-1-amine (0.018 g, 0.021 mL, 0.32 mmol, 1.40 eq) was added syringe. Stirring was continued at RT. After 21 h reaction time, LC-MS (water/MeOH) confirmed full conversion of the MDP starting material. The reaction mixture was concentrated in vacuo, giving crude product as a yellow glass. The material was purified twice by automated column chromatography (reversed-phase (C18); product:C18-silica 1:100; detection: 200-400 nm), eluting with water/MeOH 90/10-82/18. Pure fractions were lyophilized, giving pure product as a white solid (0.050 g, 41%).

1H NMR (400 MHz, MeOD) δ 5.16 (d, J = 3.4 Hz, 1H), 4.42 - 4.23 (m, 3H), 3.95 (t, J = 2.3 Hz, 2H), 3.94 - 3.57 (m, 5H), 3.52 - 3.39 (m Hz, 1H), 2.58 (t, J = 2.6 Hz, 1H), 2.33 - 2.26 (m, 2H), 2.25 - 2.13 (m, 1H), 2.00 - 1.85 (m, 4H), 1.45 - 1.33 ppm (m, 6H). ¹³C NMR (100 MHz, MeOD) δ 175.26, 174.83, 173.89, 173.06, 172.08, 91.01, 79.15, 78.92, 76.68, 71.86, 70.83, 70.21, 63.35, 61.22, 54.13, 52.66, 49.48, 31.54, 28.11, 27.10, 23.85, 21.46, 18.31, 16.21 ppm. HPLC-MS (water/MeCN): t (product) = 0.76 and 1.02 min. Found: m/z = 512.08 [M-H₂O+H]⁺; 552.33 [M+Na]⁺ (pos. mode); 325.17 [M-muramyl]- (neg. mode).

### MDP(Bn)

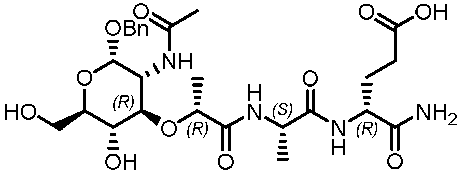

MDP(Bn) was synthesized using standard SPPS methods in an 100 mL glass reactor vessel with glass frit filter bottom. Sufficient agitation of the reaction mixture was ensured by applying a constant argon flow through the glass frit filter, whilst excess reagent and washing solutions were removed by vacuum filtration. The crude MDP(Bn) was purified twice by automated column chromatography (reversed-phase (C18); product:C18-silica 1:200; detection: 200-400 nm), eluting with water/MeCN + 0.1% formic acid 90/10-82/18. Pure fractions were lyophilized, giving pure product as a fluffy, white material (0.309 g, 67%).

¹H NMR (400 MHz, DMF-d7) δ 8.18 (d, J = 8.6 Hz, 1H), 8.15 (d, J = 8.6 Hz, 1H), 7.75 (d, J = 6.5 Hz, 1H), 7.53 - 7.28 (m, 6H), 7.11 - 7.03 (m, 1H), 4.86 (d, J = 3.5 Hz, 1H), 4.76 (d, J = 12.3 Hz, 1H), 4.72 - 4.56 (m, 1H), 4.51 (d, J = 12.3 Hz, 1H), 4.47 - 4.32 (m, 3H), 4.01 (ddd, J = 10.7, 8.4, 3.5 Hz, 1H), 3.83 (dd, J = 11.6, 2.2 Hz, 1H), 3.78 - 3.61 (m, 3H), 3.60 - 3.40 (m, 1H), 2.39 (t, J = 7.8 Hz, 2H), 2.24 - 2.12 (m, 1H), 1.97 - 1.83 (m, 4H), 1.40 (d, J = 7.0 Hz, 3H), 1.34 ppm (d, J = 6.7 Hz, 3H). ¹³C NMR (100 MHz, DMF-d7) δ 174.30, 173.74, 173.60, 172.82, 170.19, 138.47, 128.58, 127.93, 127.79, 97.05, 80.16, 77.36, 73.94, 70.66, 68.61, 61.76, 53.64, 52.59, 49.41, 35.63, 30.57, 30.47, 27.78, 22.65, 19.05, 18.04 ppm. HPLC-MS (water/MeCN): t(product) = 3.11 min. Found: m/z = 583.08 [M+H]⁺.

### MDP(Bn)-propargyl

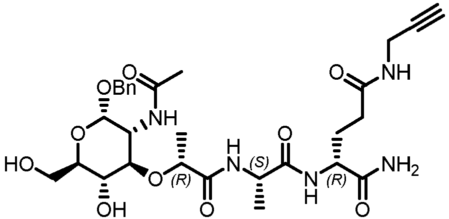

A 5 mL round-bottomed flask was charged with MDP(Bn) (0.110 g, 0.19 mmol, 1.00 eq) under argon atmosphere. The material was dissolved in dry DMF (0.5 mL). PyBOP (0.127 g, 0.25 mmol, 1.30 eq) and N,N-diisopropylethylamine (0.049 g, 0.066 mL, 0.38 mmol, 2.00 eq) were added, resulting in a clear, colorless solution. The mixture was stirred for 5 minutes at RT. Next, prop-2-yn-1-amine (0.021 g, 0.024 mL, 0.38 mmol, 2.00 eq) was added and the resulting light yellow mixture stirred at RT. After 2 h reaction time, the reaction mixture was concentrated in vacuo, giving crude product as a beige, sticky solid. The material was purified by automated column chromatography (reversed-phase (C18); product:C18-silica 1:100; detection: 200-400 nm), eluting with water/MeCN 90/10-80/20. Pure fractions were lyophilized, giving pure product as a white solid (0.096 g, 82%).

¹H NMR (400 MHz, DMF-d7) δ 8.25 (t, J = 5.5 Hz, 1H), 8.20 - 8.15 (m, 2H), 7.75 (d, J = 6.6 Hz, 1H), 7.51 - 7.28 (m, 6H), 7.08 - 7.03 (m, 1H), 5.47 (d, J = 6.3 Hz, 1H), 4.86 (d, J = 3.5 Hz, 1H), 4.76 (d, J = 12.3 Hz, 1H), 4.63 (t, J = 6.0 Hz, 1H), 4.51 (d, J = 12.4 Hz, 1H), 4.47 - 4.27 (m, 3H), 4.05 - 3.95 (m, 3H), 3.83 (ddd, J = 11.5, 5.7, 2.2 Hz, 1H), 3.75 - 3.61 (m, 3H), 3.54 - 3.46 (m, 1H), 3.04 (t, J = 2.5 Hz, 1H), 2.32 - 2.26 (m, 2H), 2.21 - 2.11 (m, 1H), 1.95 - 1.83 (m, 4H), 1.39 (d, J = 7.0 Hz, 3H), 1.34 ppm (d, J = 6.8 Hz, 3H). ¹³C NMR (100 MHz, DMF-d7) δ 173.79, 173.59, 172.74, 171.93, 170.19, 138.47, 128.58, 127.93, 127.80, 97.05, 81.35, 80.16, 77.36, 73.94, 72.14, 70.63, 68.62, 61.76, 53.65, 52.94, 49.38, 35.63, 32.21, 30.47, 28.31, 28.25, 22.66, 19.01, 18.06 ppm. HPLC-MS (water/MeCN): t (product) = 3.35 min. Found: m/z = 620.17 [M+H]⁺.

### MTP-b on resin

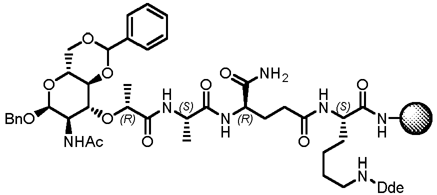

MTP-b on-resin was synthesized using standard SPPS methods in an 100 mL glass reactor vessel with glass frit filter bottom. Sufficient agitation of the reaction mixture was ensured by applying a constant argon flow through the glass frit filter, whilst excess reagent and washing solutions were removed by vacuum filtration. After performing the final post-coupling wash, the resin was washed again with DCM (2x 20 mL) and dried in an argon flow. The material was stored at -20 °C. A sample was cleaved from the resin using TFA/TIPS/water 95/2.5/2.5 (0.1 mL, 10 min) and checked with HPLC-MS (water/MeCN). HPLC-MS (water/MeCN): t (product) = 3.65 min. Found: m/z = 874.42 [M+H]⁺ (pos. mode); m/z = 918.08 [M+HCOO]⁻ (neg. mode).

### MTP-b-N3

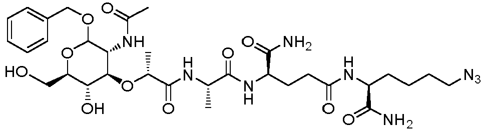

A 20 mL PE syringe with PE frit was charged with on-resin MTP-b (429 mg, approx. 0.15 mmol MTP-b) and the resin was swollen in DMF (12 mL) for 30 min. The resin was treated twice with 2 % hydrazine hydrate solution in DMF (12 mL) for 15 min. After filtration the resin was washed with DMF (4 x 12 mL) for 1 min. A solution of CuSO₄·5H₂O (0.6 mg, 2.4 µmol, 1.5 mol %), imidazole-1-sulfonyl azide HCl-salt (170 mg, 0.77 mmol, 5 eq) and N,N-diisopropylethylamine (0.34 mL, 1.9 mmol, 12 eq) in DMF (12 mL) was added to the resin and the beads were agitated at room temperature for 24 h (slight overpressure was relieved every now and then). After filtration the resin was washed with DMF (5 x 12 mL) for 1 min and dichloromethane (4 x 10 mL) for 1 min. The resin was then subjected to cleavage in TFA/TIPS/H₂O 95:2.5:2.5 (4 mL) for 2 h. After filtration the resin was washed with TFA (4 mL) for 5 min. The combined TFA filtrates were concentrated in vacuo (keeping the temperature as low as possible to avoid TFA-ester formation). Automated column chromatography (reversed-phase (C18); detection: λ = 200 nm), using an elution gradient of 5 % to 60 % MeCN in H₂O (both containing 0.1 % TFA) yielded impure compound. This was further purified with RP-HPLC using an elution gradient of 26 % to 35 % MeCN in H₂O (both containing 0.1 % TFA) yielding pure product (37.5 mg, 51 µmol, 34 %) as a white fluffy solid after lyophilization. ¹H-NMR (400 MHz, DMF-d7/D₂O 9:1): δ = 8.45 (t, J = 9.1 Hz, 2H), 8.26 (d, J = 7.9 Hz, 1H), 7.99 (d, J = 6.5 Hz, 1H), 7.81 (d, J = 2.8 Hz, 2H), 7.64-7.45 (m, 5H), 7.31 (d, J = 17.2 Hz, 2H), 5.82 (d, J = 6.3 Hz, 1H), 5.03 (d, J = 3.5 Hz, 1H), 4.93 (d, J = 12.4 Hz, 1H), 4.68 (d, J = 12.4 Hz, 1H), 4.63-4.46 (m, 4H), 4.19 (dd, J = 10.7, 3.6 Hz, 1H), 4.00 (dd, J = 6.8, 6.3 Hz, 1H), 3.92-3.79 (m, 3H), 3.66 (t, J = 9.0 Hz, 1H), 3.52 (t, J = 6.8 Hz, 2H), 2.53 (t, J = 7.5 Hz, 2H), 2.37 (dtd, J = 16.5, 7.9, 4.3 Hz, 1H), 2.10 (s, 3H), 2.09-1.93 (m, 2H), 1.88-1.55 (m, 5H), 1.58 (d, J = 7.1 Hz, 3H), 1.52 (d, J = 6.7 Hz, 3H). ¹³C-NMR (100 MHz, DMF-d7/D₂O 9:1): δ = 174.9, 174.24, 174.17, 174.00, 173.93, 173.14, 173.06, 172.9, 172.8, 170.90, 170.8, 138.2, 128.6, 127.9, 127.8, 96.8, 80.0, 77.3, 73.7, 70.3, 68.6, 61.5, 53.6, 53.5, 53.2, 53.13, 53.10, 52.7, 52.6, 51.2, 49.4, 49.3, 32.1, 31.7, 28.5, 28.2, 23.2, 22.53, 22.48, 18.9, 17.80, 17.76. ESI-MS: m/z Calc. for C₃₂H₄₉N₉O₁₁ 735.36; Obs. [M+H]⁺ 736.25, [M+Na]⁺ 758.42.

### DSG 4-nitrophenylcarbonate

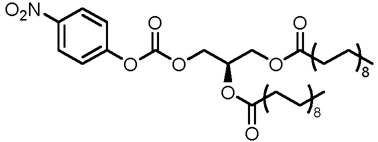

A 25 mL round-bottom flask was filled with a solution of commercially available [(2S)-3-hydroxy-2-octadecanoyloxypropyl] octadecanoate (0.601 g, 0.96 mmol, 1.00 eq) in chloroform (6.5 mL, ~0.15 M). Pyridine (0.122 g, 0.125 mL, 1.54 mmol, 1.60 eq) was added and the resulting clear solution cooled in icewater. Next, solid 4-nitrophenyl chloroformate (0.252 g, 1.25 mmol, 1.30 eq) was added in small portions. The light yellow reaction mixture was stirred at RT overnight. Full conversion of the alcohol was confirmed by ¹H NMR (CDCl₃). Subsequently, the reaction mixture was precipitated in MeOH (100 mL) and collected by filtration through glass filter. The material was washed with MeOH (30 mL total) and Et₂O (10 mL total) and dried in a vacuum oven 30 °C. Product (0.713 g, 94%) was obtained as a white solid.

¹H NMR (400 MHz, CDCl₃) δ 8.29 (d, J = 9.1 Hz, 2H), 7.39 (d, J = 9.1 Hz, 2H), 5.38 (p, J = 5.2 Hz, 1H), 4.50 (dd, J = 11.7, 3.9 Hz, 1H), 4.41 - 4.32 (m, 2H), 4.22 (dd, J = 12.0, 5.6 Hz, 1H), 2.35 (dt, J = 9.8, 7.5 Hz, 4H), 1.68 - 1.58 (m, 4H), 1.37 - 1.17 (m, 56H), 0.88 ppm (t, J = 6.7 Hz, 6H). ¹³C NMR (101 MHz, CDCl₃) δ 173.24, 172.92, 155.35, 152.29, 145.56, 125.36, 121.76, 68.33, 66.97, 61.63, 34.16, 34.03, 31.93, 29.71, 29.68, 29.64, 29.49, 29.37, 29.29, 29.13, 29.07, 24.87, 22.70,14.12 ppm.

### 2,3,5,6-Tetrafluorophenyl stearate

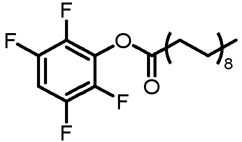

Trietylamine (2.8 mL, 5 eq.) was added slowly to a solution of stearoyl chloride (1.26 g, 4.2 mmol) and tetrafluorophenol (0.72 g, 1.04 eq.) in DCM (10 mL), causing the immediate formation of a white precipitate. The heterogeneous reaction mixture was stirred for another 2 hours, diluted with 25 mL DCM and extracted with water (50 mL), 0.1 M HCl (2x50mL), dried with MgSO4 and evaporated to dryness. The resulting solid was redissolved in 50 mL diethyl ether and extracted again with 1M NaHCO₃ (50 mL), 0.1 M HCl (50 mL), water (50 mL) and brine (2x50 mL). The organic phase was dried with MgSO4 (augmented with a small amount of activated carbon) and evaporated to dryness. The resulting crude material was redissolved in chloroform and flushed over a silica plug and again evaporated to dryness to afford 1.2 g (67%) of the desired compound as a white solid. ¹H NMR (400 MHz, CDCl₃) δ 6.98 (tt, J = 9.9, 7.0 Hz, 1H), 2.66 (t, J = 7.4 Hz, 2H), 1.78 (p, J = 7.4 Hz, 2H), 1.26 (s, 28H), 0.88 (t, J = 6.7 Hz, 3H) ppm. ¹⁹F NMR (376 MHz, CDCl₃) δ -139.21 (ddd, J = 24.0, 11.8, 7.8 Hz), -153.03 - -153.20 (m). ¹³C NMR (101 MHz, CDCl₃) δ 169.56, 147.23 (m), 144.74 (m), 141.94, 141.86 (m), 139.41 (m), 129.78 (m), 103.01 (t), 33.42, 31.92, 29.69, 29.65, 29.61, 29.54, 29.39, 29.36, 29.14, 28.85, 24.78, 22.68, 14.09 ppm.

### Synthetic approaches

*First approach.* Compounds of the invention can be prepared by using the starting reactants MDP or MDP(Bn) - *vide supra.* These molecules have a functional carboxylic acid group that originates from the glutamic acid (Glu) building block. The COOH-group enables conjugation to amine-functional reactants that comprise lipophilic groups. Such lipophilic groups can for example be C18-moieties, for examples those derived from stearic acid, oleic acid, stearyl alcohol, oleyl alcohol, stearyl amine or oleyl amine; or sterol moieties, for example those derived from cholesterol. Saturated linear lipophilic moieties are preferred, as well as moieties derived from cholesterol. Particularly useful building blocks are PE-phospholipids such as DSPE ([1069-79-0]) or DOPE ([4004-05-1]); these molecules are already amine functional. Mixed acyl PE-phospholipids can also be useful (e.g. 16:0-18:1 PE or 18:0-18:1 PE or 18:0-16:0 PE). Another useful building block is cholesterol. Yet other useful building blocks are diglycerides, such as 1,2-dioctadecanoyl-sn-glycerol (18:0 DG [51063-97-9]) or 1-2-dioleoyl-sn-glycerol (18:1 DG [24529-88-2]). Mixed acyl diglycerides can also be used (e.g. 16:0-18:1 DG or 18:0-18:1 DG or 18:0-16:0 DG). PE-phospholipids as well as the diglycerides have two lipophilic chains, and such building blocks are preferably used in this approach.
This first approach is illustrated in Examples 10 to 19.

*Second approach.* In a particularly suitable alternative modular approach, copper-catalyzed azide-alkyne cycloadditions ("click reactions") are employed to connect the MDP (or MTP) reactant to the lipophilic reactant. Here, MDP, MDP(Bn), MTP or MTP(Bn) building blocks are used that have azide (-N₃) or alkyne (-C≡C-H) functionalities. Non-limiting examples of such molecules are MDP-propargyl, MDP(Bn)-propargyl or MTP-b-N₃ - *vide supra.* In the copper-catalyzed click reactions, these molecules can be coupled to alkyne- or azide-functional molecules that comprise lipophilic groups. Using the click-reaction, azide- or alkyne functional intermediates are targeted that can be prepared easily and modularly and that are stable. This allows simple isolation and storage of intermediates. Furthermore, the copper-catalyzed click cycloadditions can be performed - and are best performed - in an aqueous environment (such as e.g. THF/water or tBuOH/water) or in an aqueous two-phase liquid/liquid solvent combination (such as e.g. dichloromethane/water). In these reaction media, both the hydrophilic MDP (or MTP) reactant (with or without Bn-group) and the lipophilic reactant are conveniently soluble, highly improving ease of conjugation and reaction yields. In this click-approach, the lipophilic groups comprised in the azide or alkyne reactants can be C14, C16 or C18-moieties, such as those derived from stearic acid, palmitic acid, myristic acid, oleic acid, palmitoleic acid, myristoleic acid, stearyl alcohol or amine, palmityl alcohol or amine, myristyl alcohol or amine, oleyl alcohol or amine, palmitoleyl alcohol or amine, myristoleyl alcohol or amine; or sterol moieties, for example those derived from cholesterol. Saturated linear lipophilic moieties are preferred, as well as moieties derived from cholesterol. Particularly useful building blocks are PE-phospholipids, mixed acyl PE-phospholipids, diglycerides (DG) or mixed-acyl diglycerides, with C14, C16 and/or C18 moieties in them, as well as cholesterol. Lipophilic azide or alkyne reactants that comprise two lipophilic chains or that comprise a cholesteryl group are preferred.
This second approach is illustrated in Examples 1 to 9.

Note that both approaches allow the introduction of an extra amino-acid unit attached to the glutamic acid unit of MDP or MDP(Bn). Suitable amino acid units are those derived from L-Lysine or L-Alanine. With an extra amino acid unit connected, the MDP (muramyl dipeptide) moiety is converted to an MTP (muramyl tripeptide) moiety, either with or without Bn-group. Illustrations are found in Examples 9-10, 13-15 and 17-18.

### Example 1. Synthesis of MDP- C18 [click] (1)

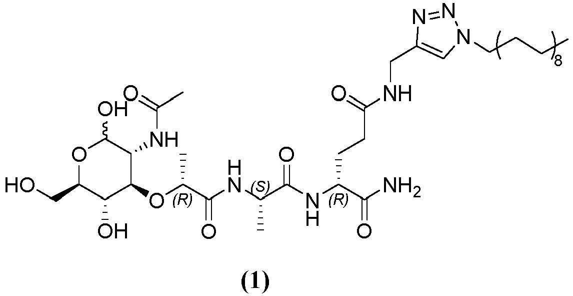

Molecular weight: 825 Dalton. CLogP = 4.15.

*This synthesis illustrates general conditions for Cu-click type reactions.*

A 5 mL vial was charged with MDP-propargyl (0.011 g, 0.02 mmol, 1.00 eq). To this was added L-ascorbic acid (0.4 M aq. solution, 104 µL, 41.5 µmol ascorbic acid, 2.00 eq). To the resulting slightly opaque solution, a solution of 1-azidooctadecane (0.012 g, 0.04 mmol, 2.00 eq) in DCM (0.8 mL) was added, followed by aqueous copper(II) sulfate pentahydrate solution (0.2 M, 104 µL, 20.8 µmol Cu, 1.00 eq). The bi-layered reaction mixture was then stirred at 1400 rpm at RT, resulting in a light yellow/green emulsion. After 16 h, the reaction mixture was concentrated in a stream of N2, giving crude product as a light brown brown sludge. The material was taken up in chloroform/MeOH 4:1 and impregnated on celite (90 mg, ~1:5 loading ratio). Purification was done by automated column chromatography (product:silica 1:500; detection: 200-400 nm), eluting with chloroform/MeOH/water 90/9/1-70/27/3, giving product (0.005 g, 31%) as a white solid.

¹H NMR (400 MHz, MeOD) δ 7.64 - 7.61 (m, 1H) , 5.33 (d, J = 3.4 Hz, 1H), 4.58 - 4.21 (m, 7H), 3.85 - 3.46 (m, 6H), 2.35 - 2.26 (m, 2H), 2.23 - 2.08 (m, 1H), 2.06 - 1.84 (m, 6H), 1.42 - 1.36 (m, 6H), 1.35 - 1.22 (m, 30H), 0.88 ppm (t, J = 6.8 Hz, 3H). ¹³C NMR (100 MHz, MeOD) δ 175.75, 174.66, 174.04, 173.58, 171.91, 144.63, 122.53, 91.00, 76.01, 71.84, 71.18, 67.16, 61.98, 54.15, 52.98, 50.67, 34.78, 32.20, 32.03, 30.34, 29.80, 29.76, 29.72, 29.65, 29.52, 29.46, 29.12, 27.49, 26.60, 22.83, 22.79, 22.69, 19.34, 16.95, 16.57, 14.15 ppm. HPLC-MS (water/MeCN): t (product) = 5.64 min. Found: m/z = 825.33 [M+H]+.

### Example 2. Synthesis of MDP-DSPE [click] (2)

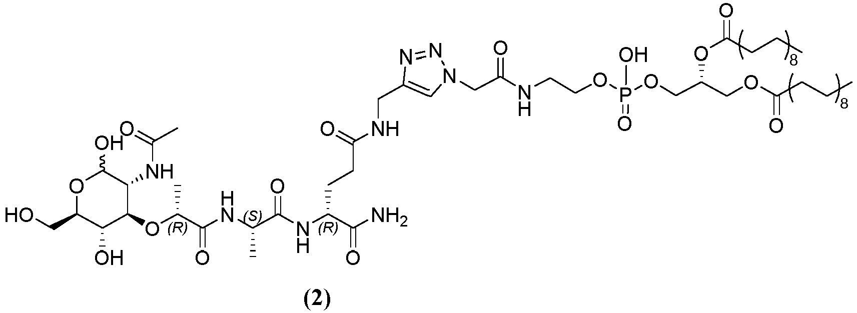

Molecular weight: 1361 Dalton. CLogP = 11.56 (uncharged) and 5.78 (negatively charged).

MDP-propargyl (24.4 mg, 46 µmol) was dissolved in 0.4 M ascorbic acid (0.24 mL, 2 eq) and a solution of DSPE-azidoacetate (38.5 mg, 46 µmol, 1 eq) in dichloromethane (0.5 mL) was added. Under vigorous stirring 0.2 M CuSO₄·5H₂O (0.24 mL, 1 eq) was added and the two-phase system was vigorously stirred at room temperature for 19 h. The solvents were removed *in vacuo* and the greenish solid was subjected to column chromatography (flash SiO₂) using an elution gradient of 20 % to 50 % MeOH in chloroform, concluded by (45% MeOH + 5% H₂O) in chloroform (a significant amount of compound only elutes after the addition of H₂O). This yielded impure product which was further purified with automated column chromatography (reversed-phase (C₁₈); product:C₁₈-silica 1:200; detection: *λ* = 210 nm), using an elution gradient of 25 % to 70 % THF in H₂O. This yielded pure product (19.5 mg, 14 µmol, 31 %) as a white fluffy solid after lyophilization.
¹H NMR (400 MHz, CDCl₃+MeOD) δ 7.93 (s, 1H), 5.29 - 5.22 (m, 2H), 5.16 (s, 2H), 4.51 - 4.41 (m, 4H), 4.38 - 4.25 (m, 2H), 4.20 (dd, *J=* 12.1, 6.8 Hz, 1H), 4.05 - 3.89 (m, 4H), 3.88 - 3.77 (m, 2H), 3.77 - 3.63 (m, 6H), 3.55 - 3.42 (m, 3H), 2.34 (q, *J=* 7.3 Hz, 6H), 2.22 (dddd, *J =* 18.4, 13.5, 8.4, 5.7 Hz, 1H), 1.98 (d, *J=* 5.7 Hz, 4H), 1.93 (s, 0H), 1.62 (q, *J=* 6.4 Hz, 5H), 1.46 - 1.36 (m, 6H), 1.28 (s, 61H), 0.89 (t, *J =* 6.8 Hz, 6H). Peaks between 4.9 and 4.6 ppm are not visible due to overlap with the H₂O peak. MALDI-TOF MS: *m*/*z* Calc. for C₆₅H₁₁₈N₉O₁₉P 1359.83; Obs. [M+Na]⁺ 1382.83, [M-H+2Na]⁺ 1404.84. HPLC-MS (H₂O/THF, gradient: 65-95% THF): t (product) = 2.33 min; *m*/*z* = 1360.80 [M+H]⁺ (SIM mode).

### Example 3. Synthesis of MDP-chol [click] (3)

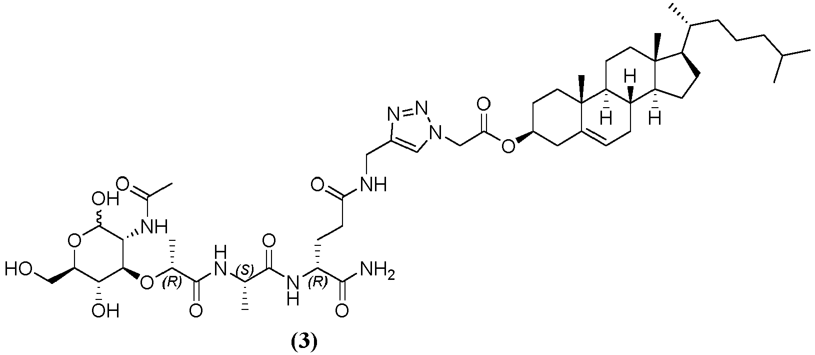

Molecular weight: 999 Dalton. CLogP = 5.04.

MDP-propargyl (25 mg, 47 µmol) was dissolved in 0.4 M ascorbic acid (0.24 mL, 2 eq) and a solution of cholesterol azidoacetate (26.6 mg, 57 µmol, 1.2 eq) in dichloromethane (0.5 mL) was added. Under vigorous stirring 0.2 M CuSO₄·5H₂O (0.24 mL, 1 eq) was added and the two-phase system was vigorously stirred at room temperature for 17 h. H₂O / brine 1:1 (50 mL) was added and the bluish aqueous layer was extracted with chloroform / MeOH 2:1 (5 × 20 mL). The combined organic layers were dried using Na₂SO₄, filtrated and the solvent was removed in vacuo. The resulting colorless solid was purified with repeated column chromatography (flash SiO₂) using an elution gradient of 6 % to 20 % MeOH in chloroform. This yielded pure product (24.4 mg, 24 µmol, 52 %) as a white fluffy solid after lyophilization from THF / H₂O. ¹H-NMR (400 MHz, THF-d8/D₂O 95:5): δ = 7.81 (s, 1H), 5.28 (d, J = 4.9 Hz, 1H), 5.16 (s, 2H), 5.10 (d, J = 3.4 Hz, 1H), 4.59-4.46 (m, 1H), 4.46-4.16 (m, 5H), 3.75-3.17 (m, 6H), 2.26 (d, J = 8.2 Hz, 2H), 2.20 (t, J = 7.5 Hz, 2H), 2.12-1.99 (m, 1H), 1.98-1.69 (m, 8H), 1.58-1.33 (m, 5H), 1.32-1.23 (m, 7H), 1.18 (s, 5H), 1.13-0.97 (m, 4H), 0.94 (s, 3H), 0.84 (d, J = 6.5 Hz, 3H), 0.77 (dd, J = 6.6, 1.4 Hz, 8H), 0.61 (s, 3H). ¹³C-NMR (100 MHz, THF-d8/D₂O 95:5): δ = 175.2, 174.9, 173.7, 173.4, 171.9, 166.6, 165.1, 139.6, 122.4, 91.0, 78.7, 76.7, 75.4, 72.0, 70.2, 61.1, 56.8, 56.2, 54.0, 52.6, 50.5, 50.2, 49.5, 42.2, 39.8, 39.4, 37.7, 36.8, 36.4, 36.1, 35.8, 34.4, 31.9, 31.8, 29.6, 28.1, 27.9, 27.5, 27.4, 22.5, 22.2, 22.1, 21.9, 20.9, 18.8, 18.7, 18.2, 16.8, 13.5, 11.3. MALDI-TOF MS: m/z Calc. for C₅₁H₈₂N₈O₁₂ 998.60; Obs. [M+Na]⁺ 1021.58. HPLC-MS (H₂O/THF, gradient: 65-95% THF): t (prod) = 2.20 min; m/z = 999.60 [M+H]⁺ (SIM mode). Note: THF-d8/D₂O 95:5 was found to be an optimal solvent combination for NMR characterization. Nevertheless, the spectrum suffers from overlap and is very complicated. Therefore, integration is tentative.

### Example 4. Synthesis of MDP- DSPE₂[click] (4)

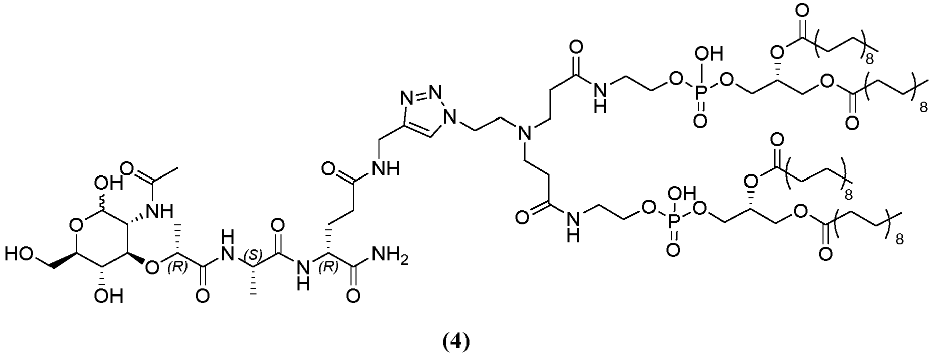

Molecular weight: 2192 Dalton.

### Example 5. MDP-Chol2[click]

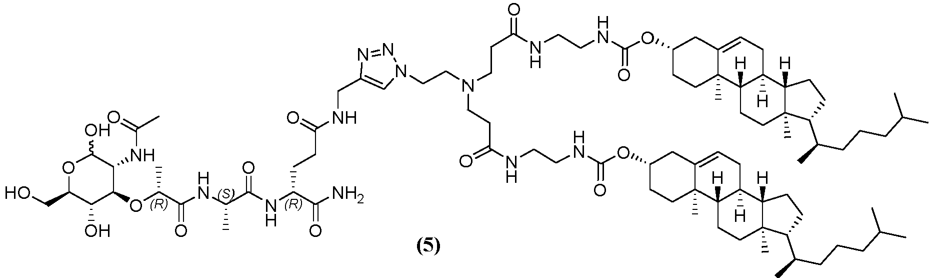

Molecular weight: 1669 Dalton. CLogP = 15.31.

### Example 6. MDP-DSG[click] (6)

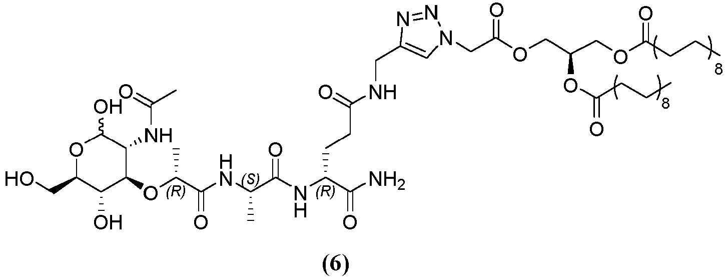

Molecular weight: 1238 Dalton. CLogP = 12.45.

### Example 7. MDP(Bn)-DSPE [click] (7)

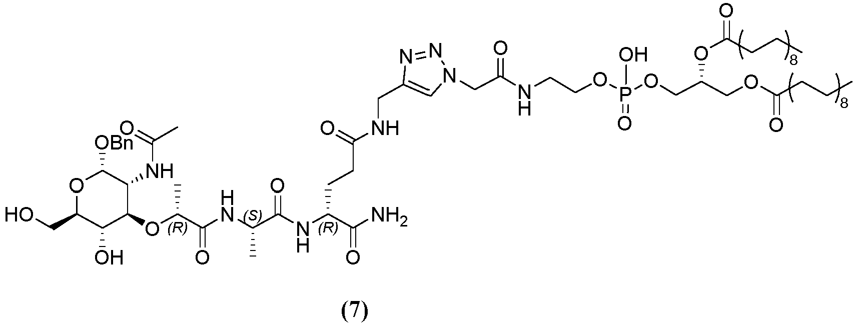

Molecular weight: 1451 Dalton. CLogP = 13.85 (uncharged) and 8.06 (negatively charged).

Following the general conditions for Cu-click reactions, MDP(Bn)-propargyl (0.028 g, 0.045 mmol, 1.00 eq) and DSPE-azidoacetate (0.039 g, 0.047 mmol, 1.05 eq) were reacted overnight. During the reaction, some material precipitated out, resulting in a white suspension/emulsion. Afterwards, the reaction mixture was diluted with chloroform/MeOH 1:1. The resulting clear solution was impregnated on celite (~200 mg, 1:3 loading ratio). The impregnated crude product was first purified by automated column chromatography (reversed-phase (C18); product:C18-silica 1:200; detection: ELSD and UV 200-400 nm), eluting with water/THF 60/40-20/80. The combined product fractions were lyophilized and then purified again by automated column chromatography (normal phase (silica); product:silica 1:300; detection: ELSD), eluting with chloroform/MeOH/water 90/9/1-75/22.5/2.5. Pure fractions were concentrated in vacuo, dissolved in water/THF 70/30 and lyophilized. Thus, pure product was obtained as a white fluffy solid (0.027 g, 41%).

¹H NMR (400 MHz, CDCl₃+MeOD 1:1) δ 7.99 (d, J = 8.2 Hz, 1H), 7.90 (s, 1H), 7.43 - 7.23 (m, 5H), 5.24 (m, 1H), 5.13 (s, 2H), 4.93 (d, J = 3.5 Hz, 1H), 4.73 (d, J = 12.0 Hz, 1H), 4.51 (d, J = 12.0 Hz, 2H), 4.48 - 4.40 (m, 2H), 4.37 - 4.15 (m, 3H), 4.05 - 3.89 (m, 5H), 3.86 - 3.73 (m, 2H), 3.71 - 3.53 (m, 3H), 3.49 - 3.42 (m, 2H), 2.37 - 2.28 (m, 6H), 2.25 - 2.11 (m, 1H), 2.05 - 1.89 (m, 4H), 1.67 - 1.56 (m, 4H), 1.52 - 1.19 (m, 72H), 0.89 ppm (t, J = 6.8 Hz, 6H). 13C NMR (100 MHz, MeOD) δ 174.97, 174.73, 173.93, 173.66, 173.57, 173.46, 171.92, 166.34, 137.16, 128.28, 128.09, 127.84, 124.43, 96.38, 79.09, 76.52, 72.55, 70.40, 69.75, 69.28, 63.66, 63.44, 62.56, 61.14, 53.18, 52.60, 52.13, 49.45, 40.52, 34.62, 34.12, 33.96, 31.81, 31.73, 29.57, 29.53, 29.43, 29.41, 29.23, 29.21, 29.03, 29.00, 27.12, 24.82, 24.77, 22.52, 22.20, 18.70, 16.75, 13.69 ppm. MALDI-TOF: m/z calcd for C₇₂H₁₂₄N₉O₁₉P + 2Na⁺-H⁺: 1494.85 [M+2Na-H]⁺; found: 1494.94. HPLC-MS (water/THF, gradient: 55-95% THF): t (product) = 5.09 min; m/z = 1450.9 [M+H]⁺ and 1472.9 [M+Na]⁺ (SIM mode).

### Example 8. Synthesis of MDP(Bn)-chol[click] (8)

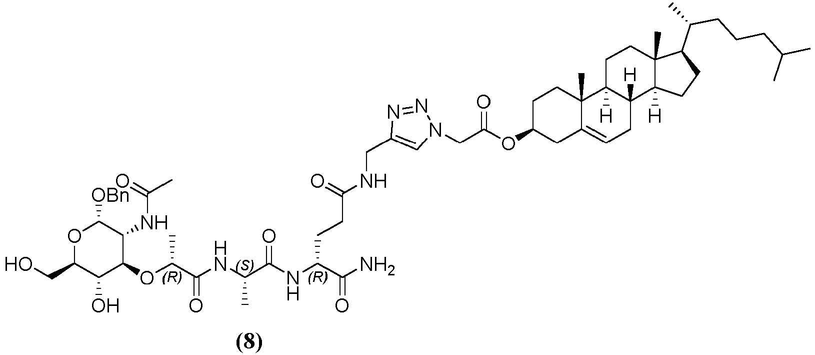

Molecular weight: 1089 Dalton. CLogP = 6.83.

Following the general conditions for Cu-click reactions, MDP(Bn)-propargyl (0.030 g, 0.048 mmol, 1.00 eq) and cholesterol azidoacetate (0.025 g, 0.053 mmol, 1.10 eq) were reacted overnight, resulting in a white emulsion. The reaction mixture was then concentrated in vacuo and impregnated on celite (150 mg). The impregnated crude product was purified by automated column chromatography (reversed-phase (C18); product:C18-silica 1:200; detection: 200-400 nm), eluting with water/THF 70/30-15/85. The combined product fractions were lyophilized and then purified again by automated column chromatography (normal phase (silica); product:silica 1:350; detection: ELSD), eluting with chloroform/MeOH 96/4-86/14. Pure fractions were concentrated in vacuo, giving pure product as a white solid (0.028 g, 53%).

¹H NMR (400 MHz, CDCl₃+MeOD 1:1) δ 7.78 (s, 1H), 7.39 - 7.28 (m, 5H), 5.38 (d, J = 5.1 Hz, 1H), 5.16 (d, J = 1.6 Hz, 2H), 4.93 (d, J = 3.6 Hz, 1H), 4.70 (d, J = 11.8 Hz, 2H), 4.56 - 4.37 (m, 3H), 4.30 (dt, J = 8.9, 4.4 Hz, 1H), 4.27 - 4.17 (m, 2H), 4.02 (dd, J = 10.1, 3.6 Hz, 1H), 3.93 - 3.67 (m, 24H), 3.67 - 3.53 (m, 3H), 3.40 (d, J = 3.2 Hz, 0H), 2.44 - 2.32 (m, 2H), 2.27 (td, J = 7.1, 3.7 Hz, 2H), 2.12 (dtd, J = 14.9, 7.5, 4.3 Hz, 1H), 2.07 - 1.74 (m, 8H), 1.72 - 1.42 (m, 5H), 1.38 (dd, J = 13.0, 7.0 Hz, 7H), 1.34 - 0.94 (m, 13H), 0.92 (d, J = 6.4 Hz, 3H), 0.87 (dd, J = 6.6, 1.8 Hz, 6H), 0.69 ppm (s, 3H). ¹³C NMR (100 MHz, CDCl₃+MeOD 1:1) δ 175.17, 174.60, 173.71, 173.62, 171.76, 166.21, 145.18, 139.13, 137.21, 128.65, 128.37, 128.25, 124.35, 123.47, 96.91, 79.14, 76.83, 76.49, 72.44, 69.79, 61.63, 56.82, 56.28, 53.17, 52.57, 51.14, 50.14, 49.78, 42.45, 39.84, 39.65, 38.01, 36.97, 36.68, 36.32, 35.93, 34.85, 32.02, 31.97, 29.81, 28.34, 28.14, 27.74, 27.58, 24.39, 23.95, 22.87, 22.80, 22.77, 22.61, 21.16, 19.34, 18.98, 18.80, 16.90, 11.94 ppm. MALDI-TOF: m/z calcd for C₅₈H₈₈N₈O₁₂+Na⁺: 1111.64 [M+Na]⁺; found: 1111.65. HPLC-MS (water/THF, gradient: 65-95% THF): t (product) = 2.79 min; m/z = 1089.70 [M+H]⁺ (SIM mode).

### Example 9. Synthesis of MTP-b-C18[invclick] (9) (Reference example)

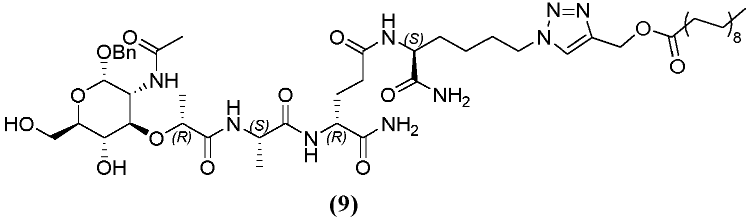

Molecular weight: 1058 Dalton. CLogP = 5.68.

MTP-b-N3 (26 mg, 35 µmol) and prop-2-yn-1-yl stearate (11.3 mg, 35 µmol, 1 eq) were suspended in THF (0.36 mL) and 0.4 M ascorbic acid (0.18 mL, 2 eq) was added yielding a clear solution. Prop-2-yn-1-yl stearate was prepared using known procedures. Under vigorous stirring 0.2 M CuSO₄·5H₂O (0.18 mL, 1 eq) was added and the mixture was vigorously stirred at room temperature (initially gelation occurred but gentle heating resulted in a yellow solution). After 1 h, HPLC-MS (THF / H₂O) indicated the absence of starting compounds and the opaque solution was lyophilized. The crude product was adsorbed onto Celite from chloroform / MeOH 2:1 and subjected to column chromatography (flash SiO₂) using an elution gradient of 10 % to 25 % MeOH in chloroform. Column chromatography was repeated using a similar gradient yielding pure product (31.5 mg, 30 µmol, 84 %) as a white fluffy solid after lyophilization from THF / H₂O. ¹H-NMR (400 MHz, THF-d8/D₂O 4:1): δ = 7.92 (s, 1H), 7.31 (d, J = 7.5 Hz, 2H), 7.23 (t, J = 7.5 Hz, 2H), 7.15 (t, J = 7.3 Hz, 1H), 5.04 (s, 2H), 4.74 (d, J = 3.5 Hz, 1H), 4.63 (d, J = 12.2 Hz, 1H), 4.40 (d, J = 12.2 Hz, 1H), 4.31-4.15 (m, 6H), 3.96 (dd, J = 10.5, 3.6 Hz, 1H), 3.66 (d, J = 3.2 Hz, 2H), 3.59-3.45 (m, 3H), 2.22 (dt, J = 15.3, 7.7 Hz, 4H), 2.09 (tt, J = 12.9, 6.0 Hz, 1H), 1.87-1.70 (m, 6H), 1.64-1.54 (m, 1H), 1.47 (q, J = 7.3 Hz, 2H), 1.32 (d, J = 7.2 Hz, 3H), 1.28 (d, J = 6.7 Hz, 3H), 1.18 (s, 30H), 0.78 (t, J = 6.6 Hz, 3H). ¹³C-NMR (100 MHz, THF-d8/D₂O 4:1): δ = 175.5, 174.9, 174.8, 173.73, 173.66, 173.2, 171.7, 142.3, 137.8, 128.1, 128.0, 127.4, 124.3, 96.6, 80.0, 77.3, 72.8, 69.1, 68.8, 60.9, 57.2, 53.2, 53.1, 52.2, 49.7, 49.5, 33.6, 31.8, 31.5, 30.9, 29.7, 29.54, 29.50, 29.4, 29.24, 29.20, 29.0, 27.7, 22.54, 22.49, 21.9, 18.6, 16.9, 13.5. MALDI-TOF MS: m/z Calc. for C₅₃H₈₇N₉O₁₃ 1057.64; Obs. [M+Na]⁺ 1080.63, [M+K]⁺ 1096.65. HPLC-MS (H₂O/THF, gradient: 65-95% THF): t (prod) = 2.15 min; m/z = 1058.60 [M+H]⁺ (SIM mode).

### Example 10. Synthesis of MTP-b-C18 (10) (Reference example)

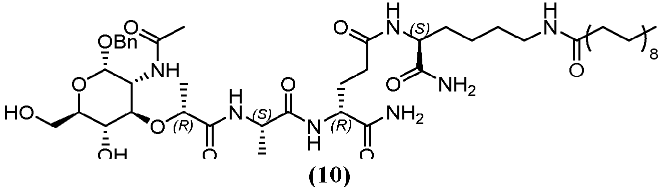

Molecular weight: 976 Dalton. CLogP = 5.31

A 10 mL PE syringe with PE frit was charged with MTP-b on-resin (137 mg, approx. 0.0493 mmol MTP-b, 1.00 eq). The resin was swollen in DMF (5 mL) for 30 min. Next, the resin was treated twice with 2% hydrazine hydrate solution in DMF (10 mL) for 15 min. The hydrazine solution was removed and the resin washed with DMF (4x 5 mL). Next, a solution of 2,3,5,6-tetrafluorophenyl stearate (0.064 g, 0.15 mmol, 3.00 eq) and 4-methylmorpholine (0.030 g, 0.033 mL, 0.30 mmol, 6.00 eq) in DMF/DCM (1+1 mL; ~0.075 M) was added. The beads were agitated overnight at RT. Afterwards, the supernatant was removed and the resin washed with DMF/DCM 50/50 (4x 5 mL) and DCM (2x 5 mL). The resin was then treated with TFA/TIPS/water 95/2.5/2.5 (200 uL) for 1 h. The filtrate was collected and the resin washed with additional cleavage cocktail. The combined filtrates were concentrated in vacuo, giving the crude product as a white solid. The material was impregnated on celite (200 mg, 1:4 loading ratio) from THF/water solution (95/5). The impregnated crude product was purified by automated column chromatography (reversed-phase (C18); product:C18-silica 1:250; detection: ELSD), eluting with water/THF 50/50-10/90. The combined product fractions were lyophilized and then purified again by automated column chromatography (normal phase (silica); product:silica 1:500; detection: ELSD), eluting with dichloromethane/MeOH 90/10-70/30. Pure fractions were concentrated in vacuo, giving the product as a white solid (0.016 g, 33%). ¹H NMR (400 MHz, CDCl₃+TFA-d3) δ 7.40 - 7.24 (m, 5H), 4.94 - 4.89 (m, 1H), 4.71 - 4.65 (m, 1H), 4.57 - 4.17 (m, 6H), 4.03 - 3.74 (m, 4H), 3.38 (bs, 2H), 2.60 - 2.22 (m, 5H), 2.30 (s, 1H), 2.11 - 1.55 (s, 11H), 1.50 - 1.17 (m, 36H), 0.87 ppm (t, J = 6.6 Hz, 3H). HPLC-MS (water/MeCN): t (product) = 5.64 min. Found: m/z = 976.33 [M+H]⁺ (pos. mode); 1020.25 [M+HCOO]⁻ (neg. mode).

### Example 11. Synthesis of MDP-C18 (11) (Reference example)

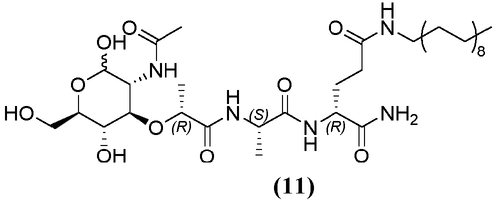

Molecular weight: 744 Dalton. CLogP = 4.79.

MDP (10 mg, 20 µmol), octadecyl amine (5.2 mg, 0.95 eq.), NHS (2.4 mg, 1 eq.) and EDC-HCl (7.9 mg, 2 eq.) were stirred in DMF (0.7 mL) at 50°C for 3 hours. The reaction mixture was subsequently allowed to cool to room temperature and stirred for another 16 hours. The resulting dispersion was heated to 40°C to redissolve all the precipitated solids and subsequently precipitated with 5 mL ether. The collected precipitate was washed 2 more times with ether, dried and then suspended in demineralized water, collected by centrifugation, resuspended in demineralized water, and again collected by centrifugation. The resulting solid was lyophilized to remove all water to afford 13.8 mg (96%) of the desired compound as a white powder. ¹H NMR (400 MHz, DMF-d7) δ 8.31 (d, *J =* 7.7 Hz, minor isomer), 8.23 (d, *J =* 8.0 Hz, major isomer), 8.12 (d, *J* = 7.9 Hz, minor isomer), 8.09 (d, *J =* 7.6 Hz, major isomer), 7.96 (d, *J* = 6.6 Hz, minor isomer), 7.90 (d, *J =* 6.5 Hz, major isomer), 7.77 (m, 1H), 7.47 (m, 1H), 7.10 (m, minor isomer), 7.01 (m, major isomer), 6.86 (d, *J =* 6.0 Hz, minor isomer), 6.74 (dd, *J* = 4.1, 1.2 Hz, major isomer), 5.44 - 5.29 (m, 1H), 5.16 (t, *J =* 3.7 Hz, major isomer), 4.79 (t, *J =* 6.1 Hz, minor isomer), 4.60 (dd, *J* = 8.2, 6.0 Hz, minor isomer), 4.57-4.22 (m, 4H), 3.93-3.57 (m, 5H), 3.45 (m, 1H), 3.13 (m, 2H), 2.42-2.07 (m, 3H), 2.00-1.78 (m, 4H), 1.56 - 1.07 (m, 38H), 0.88 (m, 3H) ppm. ¹³C NMR (101 MHz, DMF-d7) δ 174.25, 174.20, 173.93, 173.89, 172.75, 172.56, 172.07, 172.02, 171.76, 170.11, 96.92, 91.66, 82.46, 79.55, 77.39, 77.00, 72.97, 71.30, 70.95, 62.05, 57.49, 54.49, 53.16, 53.03, 49.54, 39.26, 32.55, 32.05, 29.83, 29.49, 28.62, 28.41, 27.18, 22.86, 22.76, 22.73, 19.19, 17.80, 17.62, 13.93 ppm. ESI-MS: m/z 743.50 (calc.), found 744.42 (M+H⁺), 788.33 (M-FA⁻).

### Example 12. Synthesis of MDP-DSPE (12) (Reference example)

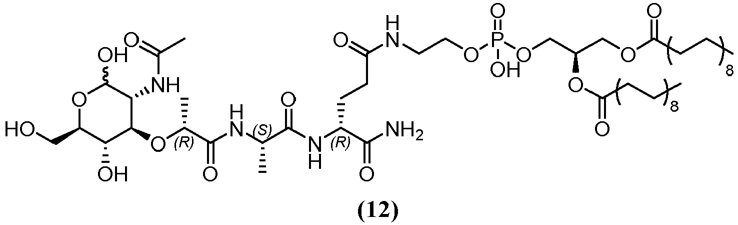

Molecular weight: 1223 Dalton. CLogP = 12.96 (uncharged) and 7.18 (negatively charged).

MDP (14 mg, 29 µmol), NHS (5.6 mg, 1.7 eq.), and DIC (7.3 mg, 2 eq) were stirred in 0.9 mL DMF for 2 hours to activate the MDP. The resulting mixture was added to a dispersion of DSPE (17 mg, 0.8 eq.) in 2.7 mL tert-butanol with TEA (9 mg, 3.1 eq.) at 50°C and stirred for 3½ hours at that temperature. The resulting mixture was evaporated to dryness and the resulting material was purified by repeated column chromatography (SiO₂, CHCl₃/MeOH/H₂O, 70/30/5, 5:4:1 and gradient 95/5/0 to 60/40/0) to afford 6 mg (21%) of the desired compound as a white fluffy material after lyophilization from water/THF. ¹H-NMR (400 MHz, CDCl₃/CD₃OD 5:) δ 5.29 (d, major isomer), 5.24 (m, 1H), 4.54 (d, minor isomer), 4.47 (m, obscured by HDO), 4.41 (dd, obscured by HDO), 4.31 (m, obscured by HDO), 4.19 (dd, 1H), 4.00-3.90 (m, mixture of isomers), 3.87-3.75 (m, mixture of isomers), 3.72 (m, 1H), 3.63 (m, 1H), 3.53-3.30 (m, obscured by CD₃OD), 2.37-2.26 (m, 5H), 2.25-2.10 (m, 2H), 2.10-1.92 (m, 4H), 1.61 (m, 4H), 1.50-1.15 (br.m, 62H), 0.88 (t, 6H) ppm. ³¹P-NMR (162 MHz, CDCl₃/CD₃OD 5:1) δ 0.14 (br.m) ppm. ¹³C-NMR (101 MHz, CDCl₃/CD₃OD 5:1) δ 174.63, 174.00, 173.27, 173.08, 172.92, 172.89, 171.34, 90.19, 75.19, 71.09, 70.18, 69.73, 69.65, 63.24, 62.76, 61.86, 60.84, 53.13, 52.04, 48.77, 48.19, 39.71, 33.48, 33.32, 31.29, 31.14, 28.91, 28.87, 28.76, 28.74, 28.57, 28.54, 28.37, 28.34, 26.44, 24.14, 24.10, 21.88, 21.78, 18.35, 15.98, 13.14 ppm. MALDI-MS: m/z 1221.77 (calc.), found 1220.82 (M-H⁻), negative mode. HPLC-ELSD (C18, 65-95% THF/H₂O): single peak plus shoulder for the alpha and beta isomers.

### Example 13. Synthesis of MTP-b-DSG (13) (Reference example)

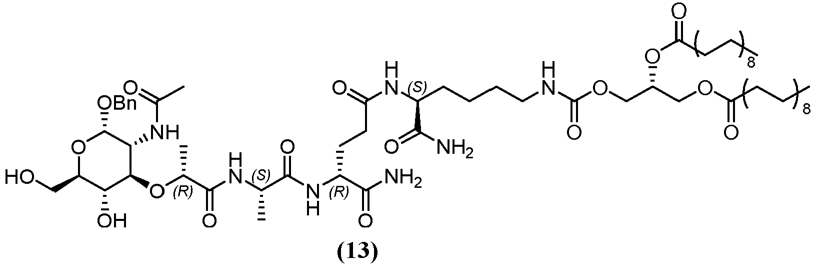

Molecular weight: 1361 Dalton. CLogP = 15.05.

A 20 mL PE syringe with PE frit was charged with MTP-b on-resin (0.291 g, approx. 0.11 mmol MTP-b, 1.00 eq). The resin was swollen in DMF (5 mL) for 30 min. Next, the resin was treated twice with 2% hydrazine hydrate solution in DMF (5 mL) for 15 min. The hydrazine solution was removed and the resin washed with DMF (4x 5 mL). Next, a solution of DSG 4-nitrophenylcarbonate (0.249 g, 0.32 mmol, 3.00 eq) and N,N-diisopropylethylamine (0.081 g, 0.110 mL, 0.63 mmol, 6.00 eq) in chloroform (4 mL) was added. The beads were agitated overnight at RT. Afterwards, the bright yellow supernatant was removed and the resin washed with chloroform (3x 5 mL), MeOH (3x 5 mL) and again chloroform (2x 5 mL). The resin was treated with TFA/TIPS/water 95/2.5/2.5 (5 mL) for 45 min. The supernatant was injected into ice-cold diethyl ether (100 mL) under stirring, resulting in the slow formation of a white flocculate. This cleavage and precipitation procedure was repeated twice. The solids were collected by filtration through a disposable PE filter, giving crude product as a white solid. The material was impregnated on celite (250 mg, 1:2.5 loading ratio) from chloroform/MeOH (1:2) solution. The impregnated crude product was purified by automated column chromatography (reversed-phase (C18); product:C18-silica 1:150; detection: 200-400 nm), eluting with water/THF 60/40-0/100. The combined product fractions were lyophilized and then purified again by automated column chromatography (normal phase (silica); product: silica 1:250; detection: 200-400 nm), eluting with chloroform/MeOH 95/5-60/40. Pure fractions were concentrated in vacuo, giving the product as a white solid (0.030 g, 21%).

¹H NMR (400 MHz, CDCl₃+MeOD) δ 7.39 - 7.27 (m, 5H), 5.25 (p, J = 5.3 Hz, 1H), 4.89 (d, J = 3.5 Hz, 1H), 4.73 (d, J = 11.9 Hz, 1H), 4.50 (d, J = 11.9 Hz, 1H), 4.42 - 4.31 (m, 2H), 4.31 - 4.19 (m, 4H), 4.15 (dd, J = 11.9, 6.2 Hz, 2H), 4.10 - 3.97 (m, 1H), 3.87 - 3.75 (m, 2H), 3.72 - 3.53 (m, 3H), 3.12 (t, J = 6.9 Hz, 2H), 2.37 - 2.27 (m, 6H), 2.26 - 2.13 (m, 1H), 1.93 (s, 3H), 1.92 - 1.74 (m, 2H), 1.71 - 1.56 (m, 6H), 1.56 - 1.47 (m, 2H), 1.43 (d, 7.0 Hz, 3H), 1.40 (d, 7.0 Hz, 3H), 1.36 - 1.19 (s, 56H), 0.89 ppm (t, J = 6.7 Hz, 6H). ¹³C NMR (100 MHz, CDCl₃+MeOD) δ 175.80, 174.79, 174.30, 173.86, 173.76, 173.44, 173.25, 171.70, 156.58, 137.07, 128.36, 128.14, 127.95, 96.52, 79.36, 76.83, 72.38, 69.48, 69.43, 69.37, 62.50, 62.29, 61.30, 53.52, 53.24, 51.71, 49.46, 40.33, 34.13, 33.97, 31.82, 31.29, 31.08, 29.58, 29.54, 29.52, 29.41, 29.39, 29.25, 29.20, 29.18, 29.09, 29.01, 28.98, 28.16, 24.80, 24.77, 22.88, 22.55, 22.35, 18.61, 16.89, 13.77 ppm. HPLC-MS (water/ THF, gradient: 55-95% THF): t (product) = 6.20 min. Found: m/z = 1360.9 [M+H]⁺ and 1382.9 [M+Na]⁺ (SIM mode).

### Example 14. Synthesis of MTP(Bn)-a-DPPE (14) (Reference example)

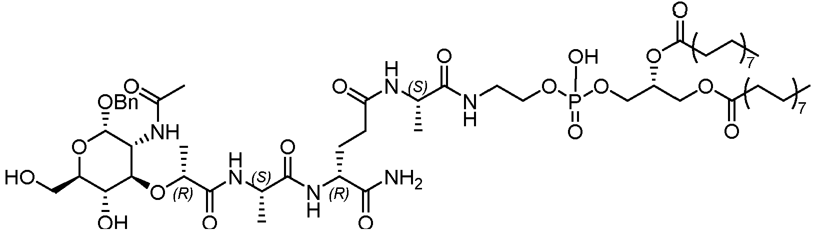

Molecular weight: 1328 Dalton. CLogP = 12.88 (uncharged) and 7.09 (negatively charged)

### Building block (CBz)-Ala-DPPE

N-CBz-protected L-alanine (390 mg, 1.7 mmol) and N-hydroxysuccinimide (222 mg, 1.89 mmol, 1.1 eq) were dissolved in chloroform (6 mL), yielding an almost clear solution. N,N'-Diisopropylcarbodiimide (DIC; 0.32 mL, 2.0 mmol, 1.2 eq) was added and the mixture was stirred at r.t. for 40 min (after 1 min the solution turns hazy and after 25 min 1H-NMR shows full conversion). This solution was then added to a 60 °C solution containing 1,2-dipalmitoyl-sn-glycero-3-phosphoethanolamine (DPPE; 1.06 g, 1.5 mmol, 0.9 eq) and triethylamine (600 µL, 0.71 mmol, 2.5 eq) in chloroform (12 mL; DPPE dissolved at reflux and triethylamine was added at a lowered temperature). The resulting clear solution was stirred at 60 °C for 1 h (the solution remains clear and after 1h ¹H-NMR shows full conversion). Chloroform (360 mL) was added and the organic layer was gently washed with 0.1 M HCl (100 mL). The organic layer was dried using Na₂SO₄, filtrated and the solvent was removed in vacuo. Column chromatography (flash SiO₂) using an elution gradient of 2% to 30% methanol in chloroform yielded the title compound that was partly contaminated with triethylamine. The impure fractions were dissolved in chloroform and the organic layer was gently washed with 0.1 M HCl. The organic layer was dried using Na₂SO₄, filtrated and the solvent was removed in vacuo. This effectively removed triethylamine and the pure fractions were combined yielding pure product (1.22 g, 1.4 mmol, 91 %) as a colorless waxy solid.

¹H-NMR (400 MHz, DMSO-d6): δ = 8.06 (t, J = 5.7 Hz, 1H), 7.47-7.22 (m, 6H), 5.15 (dq, J = 8.3, 4.6 Hz, 1H), 5.01 (q, J = 12.6 Hz, 2H), 4.28 (dd, J = 12.0, 3.2 Hz, 1H), 4.11 (dd, J = 12.1, 7.0 Hz, 1H), 4.06-3.92 (m, 3H), 3.82 (q, J = 6.4 Hz, 2H), 3.33-3.18 (m, 2H), 2.27 (dt, J = 12.8, 5.0 Hz, 4H), 1.50 (q, J = 6.9 Hz, 4H), 1.32-1.16 (m, 51H), 0.85 (t, J = 6.7 Hz, 6H). ³¹P-NMR (162 MHz, DMSO-d6): δ = -1.4.

### Building block Ala-DPPE

In a 2-neck round-bottom flask (CBz)-Ala-DPPE (308 mg, 0.34 mmol) and Pd/C (374 mg, 10 % Pd, pre-wetted Degussa / Evonik type) were combined in chloroform / ethanol 1 : 2 (36 mL). The flask was evacuated and back-filled with Ar three times. A H₂-balloon was attached, the flask was evacuated and back-filled with H₂ three times and the mixture was stirred under a positive H₂ pressure for 3 h at room temperature. The solution was filtrated over Celite which was copiously washed with ethanol, chloroform / ethanol 1 : 1 and chloroform. The combined filtrates were evaporated to dryness, the resulting compound was dissolved in chloroform / ethanol 2 : 1 (90 mL) and dried using Na₂SO₄. The solution was filtrated over Celite which was copiously washed with chloroform / ethanol 2 : 1. The filtrate was evaporated to dryness yielding the product (224 mg, 0.29 mmol, 86 %) as a slightly yellowish waxy solid, which contained trace amounts of Pd.

¹H-NMR (400 MHz, DMSO-d6): δ = 8.60 (br, 1H), 8.08 (br, 2H), 5.15 (br, 1H), 4.28 (d, J = 13.2 Hz, 1H), 4.12 (dd, J = 6.9 Hz, 1H), 4.00 (m, 2H), 3.88 (m, 2H), 3.80 (br, 1H), 3.09 (br, 1H), 2.35-2.23 (m, 4H), 1.50 (br, 4H), 1.41-1.14 (m, 51H), 0.85 (t, J = 6.6 Hz, 6H). ³¹P-NMR (DMSO-d6): δ = -1.4.

### MTP(Bn)-a-DPPE (14)

MDP(Bn) (20.0 mg, 34 µmol) and Ala-DPPE (26.2 mg, 34 µmol, 1.0 eq) were combined in DMAc (0.3 mL) and N,N-diisopropylethylamine (24 µL, 0.14 mmol, 4 eq) and PyBOP (22 mg, 41 µmol, 1.2 eq) were added consecutively. The resulting suspension was stirred at 50 °C for 1h, after which the mixture had almost cleared. The volatiles were removed in vacuo (oil pump, 45 °C) and the mixture was flushed once with chloroform. Column chromatography (flash SiO₂) using an elution gradient of 15% to 40% methanol in chloroform was followed by automated column chromatography (reversed-phase C18; product:C18-silica 1:200; detection: λ = 200-220 nm), using an elution gradient of 30% to 80% THF in H₂O. This yielded product **14** (8.0 mg, 6 µmol, 18 %) as a white fluffy solid after lyophilization. HPLC-MS: t[product] = 3.92 min.; *m*/*z* = 1327.80 [M+H]⁺ (SIM mode). HPLC-ELSD: t[prod] = 3.48 min; 99.2% relative peak area.

### Example 15. MTP-a-chol (Reference example)

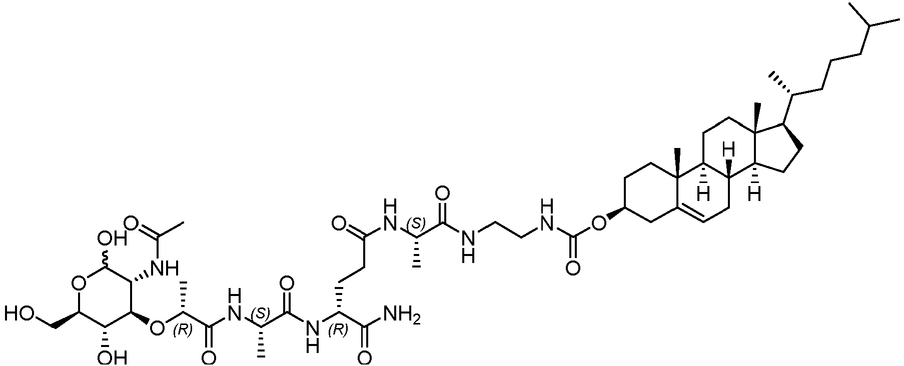

Molecular weight: 1018 Dalton. CLogP = 5.64.

### N-(2-aminoethyl)-cholesterol carbamate building block.

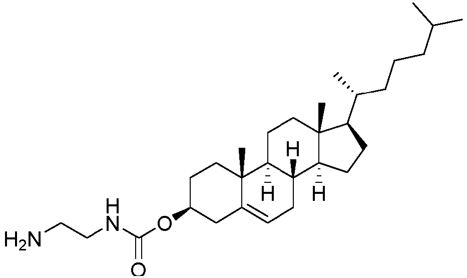

A solution of cholesterol chloroformate (0.95 g, 2.1 mmol) in 20 mL DCM was slowly added to a solution of ethylenediamine (2 mL, 14 eq.) in 30 mL DCM in about 2 hours. The reaction was allowed to proceed for another 30 minutes, after which the reaction mixture was evaporated to dryness. The resulting white material was purified via column chromatography (SiO₂, CHCl₃/MeOH/formic acid 78:20:2), yielding 720 mg (72%) of the desired compound as a white solid.

¹H NMR (400 MHz, CDCl3) δ 5.46 - 5.27 (m, 1H), 4.99 (br. s, 1H), 4.50 (br.m, 1H), 3.22 (q, J = 5.6 Hz, 2H), 2.82 (t, J = 5.9 Hz, 2H), 2.43 - 2.19 (m, 2H), 2.06 - 1.75 (m, 5H), 1.64 - 0.80 (m, 35H), 0.68 (s, 3H) ppm. ¹³C NMR (101 MHz, CDCl3) δ 156.41, 139.83, 122.47, 74.31, 56.68, 56.13, 50.00, 43.63, 42.30, 41.79, 39.73, 39.51, 38.57, 36.99, 36.56, 36.18, 35.79, 31.90, 31.87, 28.22, 28.17, 28.00, 24.28, 23.82, 22.81, 22.55, 21.03, 19.33, 18.71, 11.85. MALDI: m/z = 472.40 (calc.), found: 495.39 (M+Na+). A prominent peak is observed at m/z = 369.37, which is attributed to a 3,4-eliminated product formed in MALDI (not observed in NMR).

This building block can be coupled to N-Boc-L-Alanine (CAS [15761-38-3]) via amidation; next the Boc-group can be deprotected; finally, the formed amine functional molecule can be coupled to MDP to arrive at MTP-a-chol.

### Example 16. MDP(Bn)-chol (Reference example)

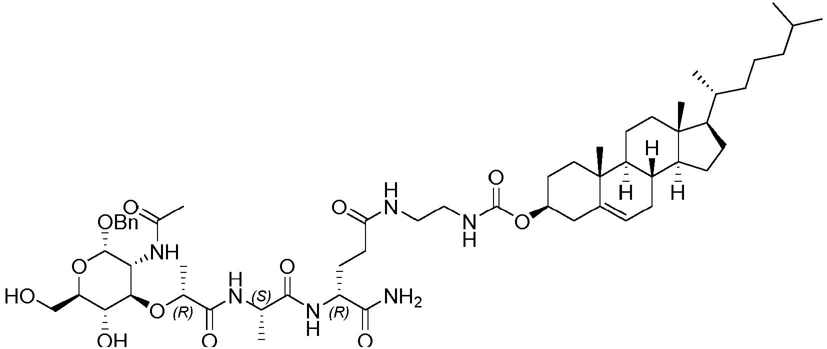

Molecular weight: 1037 Dalton. CLogP = 7.69.

The N-(2-aminoethyl)-cholesterol carbamate building block (see Example 15) can be coupled to MDP(Bn) via amidation, arriving at molecule MDP(Bn)-chol.

### Example 17 (MTP-a-DSPE), Example 18 (MTP(Bn)-a-DSPE) and Example 19 (MDP(Bn)-DSPE) (Reference example)

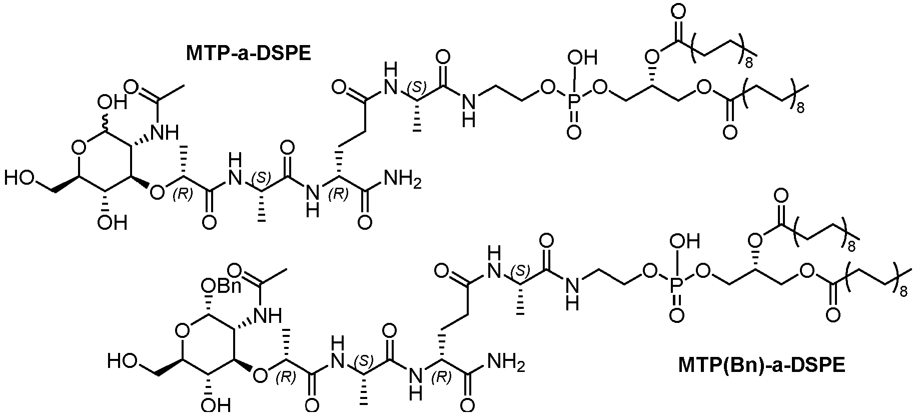

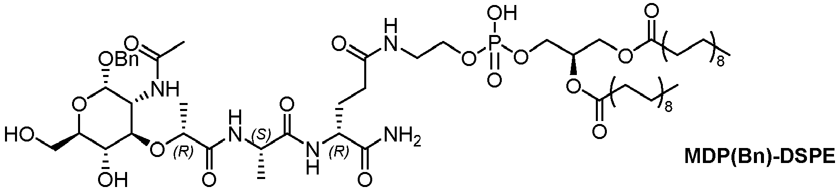

MTP-a-DSPE: MW is 1294 Dalton. CLogP = 12.70 and 6.92 (uncharged and charged).
MTP(Bn)-a-DSPE: MW is 1384 Dalton. CLogP = 14.99 and 9.21 (uncharged and charged).
MDP(Bn)-DSPE: MW is 1313 Dalton. CLogP = 15.25 and 9.46 (uncharged and charged).

1,2-Distearoyl-sn-glycero-3-phosphoethanolamine (DSPE; CAS [1069-79-0]) can be connected to N-Boc-L-Alanine (CAS [15761-38-3]) via amidation; next the Boc-group can be deprotected; finally, the formed amine functional molecule can be either coupled to MDP, to arrive at MTP-a-DSPE, or to MDP(Bn), to arrive at MTP(Bn)-a-DSPE.

Alternatively, DSPE can be coupled to MDP(Bn) via amidation, arriving at molecule MDP(Bn)-DSPE.

### Example 20: Lipophilicity studies

The ClogP values of the exemplary compounds of the disclosure were assessed, using Perkin Elmer ChemDraw Professional, version 18.0.0231 (4029) software. The results show values from about 4.15 to 18.28. At the physiological pH of about 7.4 (i.e. COOH and PO₃H groups become charged), 2 molecules of the invention have CLogP values between 4 and 5, three have values between 10 and 20, and the rest of the molecules have values between 5 and 10.

Experimentally, one can also compare lipophilicities of molecules by performing HPLC using the same elution gradient. Molecules that have higher affinities with the hydrophobic C18-material of the column are more lipophilic and as a result have a higher retention time. The below Table shows that the Example molecules of the invention (Entries 1-4) have higher retention times as compared to the Comparative Example molecules (Entries 5 and 6), and are thus more lipophilic.

Methods: HPLC-MS(SIM) and HPLC-ELSD were performed on a Phenomenex Kinetex 5 micrometer EVO C18 100A LC-column (50x2.1 mm) employing the same gradient from A to B eluent, where A = 20 mM NH₄HCO₂ in H₂O with 0.1 v/v % formic acid, and B = 2-propanol/MeCN/H₂O 85:15:5, also with 20 mM NH₄HCO₂ and 0.1 v/v % formic acid.

**Table 3: HPLC-MS or HPLC-ELSD retention times of molecules with C16, C18 and/or benzyl lipophilic units.**

| **Entry** | **Example** | **Lipophilic unit(s)** | **CLogP (uncharged)** | **CLogP (at pH=7.4)** | **HPLC-MS** | **HPLC-ELSD** |
|---|---|---|---|---|---|---|
| | | | (-) | (-) | t (min) | t (min) |
| **1** | 2 | C18 (2x) | 11.56 | 5.78 | 4.46 | 4.58 |
| **2** | 7 | C18 (2x) / Bn | 13.85 | 8.06 | 5.16 | 5.16 |
| **3** | 13 | C18 (2x) / Bn | 15.05 | 15.05 | n.d. | 5.49 |
| **4** | 14 | C16 (2x) / Bn | 12.88 | 7.09 | 3.61 | 3.5 |
| **5** | Comp-1 | C16 (2x) | 10.59 | 4.8 | 2.85 | 3.04 |
| **6** | Comp-2 | C18 | 5.39 | 1.39 | 0.95 | n.d. |

| | | | | | | |
|---|---|---|---|---|---|---|
| n.d. = not determined; cmpd-1 = mifamurtide; cmpd-2 = MDP-C18[mur] | | | | | | |

### Example 21. Aqueous solubility studies

Compounds of the disclosure were tested for their solubility in PBS-buffer and in water, applying low concentrations.

First, compounds were weighed in a vial and PBS buffer (137, 2.7, 10 and 1.8 mM in NaCl, KCl, Na₂HPO₄, KH₂PO₄, respectively; pH = 7.4) was added, such that the concentration would become 0.2 mg/mL in case full dissolution would take place. The sample was shaken, left to stand for an hour, shaken again, and then the appearance of the solution at room temperature (RT) was checked. Next, the sample was warmed for 1 minute in a water bath of 37°C, and the appearance of the solution was checked again. In the below table the results are compiled.

None of the tested compounds spontaneously dissolve in PBS, not at room temperature and not at 37°C. In contrast, the tested Comparative Example compounds dissolve spontaneously under these conditions. Further treatment of the sample solutions with a heat gun did not give dissolution for entries 4, 5 and 6, while entries 2 and 3 gave hazy solutions after cooling down to RT.

**Table 4: Solubility tests on solutions in PBS, at RT and at 37°C.**

| **Entry** | **Example** | **CLogP at pH=7.4** | **Appearance at RT** | **Appearance at 37°C** |
|---|---|---|---|---|
| **1** | Ex-2 | 5.78 | hazy | hazy |
| **2** | Ex-3 | 5.04 | suspension | hazy |
| **3** | Ex-7 | 8.06 | suspension | hazy |
| **4** | Ex-8 | 6.83 | suspension | suspension |
| **5** | Ex-11 | 4.79 | suspension | suspension |
| **6** | Ex-13 | 15.05 | suspension | suspension |
| **7** | Comp-1 | 4.80 | hazy | clear |
| **8** | Comp-2 | 1.39 | clear | clear |

| | | | | |
|---|---|---|---|---|
| cmpd-1 = mifamurtide; cmpd-2 = MDP-C18[mur] | | | | |

Next, compounds were weighed and dissolved in chloroform/methanol. The solutions were left to dry in a vial forming a film of the materials. The vials were put in vacuo to remove traces of organic solvent. Demineralized water was added, such that the concentration of the compounds would become 0.3 mM in case full dissolution would take place (0.3 mM corresponds to 0.3 mg/mL for a MW= 1000 Dalton compound). The vial was briefly sonicated in a water bath, left to stand overnight, and once again sonicated (sonication at RT). The appearances of the solutions at RT were checked to assess solubilities. In the below table the results are compiled.

None of the tested compounds of the disclosure spontaneously dissolve in water at room temperature. In contrast, the tested Comparative Example compounds dissolve spontaneously under these conditions.

**Table 5: Solubility tests on solutions in water at RT.**

| **Entry** | **Example** | **CLogP at pH=7.4** | **Appearance at RT** |
|---|---|---|---|
| **1** | Ex-2 | 5.78 | Gelly / hazy / precipitate |
| **2** | Ex-3 | 5.04 | Gelly / hazy / precipitate |
| **3** | Ex-7 | 8.06 | Hazy |
| **4** | Ex-8 | 6.83 | Precipitate |
| **5** | Ex-9 | 5.68 | Precipitate |
| **6** | Ex-10 | 5.31 | Precipitate |
| **7** | Ex-11 | 4.79 | Precipitate |
| **8** | Ex-13 | 15.05 | Gel / precipitate |
| **9** | Comp-Ex-1 | 4.80 | Clear |
| **10** | Comp-Ex-2 | 1.39 | Clear |

| | | | |
|---|---|---|---|
| cmpd-1 = mifamurtide; cmpd-2 = MDP-C18[mur] | | | |

Finally, comparative Examples 1 and 2 were also tested with respect to their solubilities in PBS (0.01 M, pH = 7.4) and demineralized water at a level of 1 mg/mL. The same results as indicated in the above two tables were found at this concentration.

Taken together, these results show that a series of compounds of the invention do not spontaneously dissolve in PBS or water at concentrations as low as 0.2 mg/mL (and higher). In contrast, comparative Example materials are soluble in PBS or water to give clear and transparent solutions at concentrations as high as at least 0.2 mg/mL or even 1 mg/mL.

Because the disclosed compounds have a low solubility in aqueous solution, their physico-chemical properties find particular use in producing stable HDL-derived NPs. Without being bound by theory, it is thought that the disclosed compounds provide improved anchoring into the NPs, reducing leakage, and providing products with greater stability and shelf-life.

### Example 22. Degradation by augmented oxidation test

The reference compounds MDP and MDP(Bn) as well as the Bn-substituted compounds from Example 7 (i.e. MDP(Bn)-DSPE[click] and Example 14 (i.e. MTP(Bn)-a-DPPE) were mixed with 12% hydrogen peroxide in water and were heated to 80°C for 4 hours, in order to get a fast degradation of the molecules by oxidation, mimicking slower in-vivo oxidation events.

The resulting reaction mixtures were diluted with acetonitrile and water (1:1) for the MDP and MDP(Bn) test solutions, or with iPrOH, acetonitrile and water (40:7.5:52.5) with 0.1% formic acid and 20 mM ammonium formate for the MDP(Bn)-DSPE[click] and the MTP(Bn)-a-DPPE solutions. The 4 diluted samples were analyzed by HPLC-MS. For reference, the 4 starting materials were also analyzed by HPLC-MS, as well as MDP-DSPE[click] and MTP-a-DPPE, i.e. the de-benzylated reference compounds to the Bn-substituted test molecules.

For all 4 test solutions, the un-affected starting compounds were traced. In addition, multiple derivatives with masses of +14, +16, +28, +30 and +32 were found, indicating oxidations from CH₂ to CO moieties (+14) and from C-H to C-OH moieties (+16), and combinations of these oxidation events. The tested MDP(Bn)-DSPE[click] and the MTP(Bn)-a-DPPE compounds mainly degraded via oxidation of the Bn-group to a benzoate group (+14) followed by hydrolysis of the benzoate (-104). This was testified by the dominant presence of the de-benzylated MDP-DSPE[click] and MTP-a-DPPE compounds as degradation products: corroborating retention times in HPLC were found as well as corroborating masses in MS (-90 relative to the starting compounds).

The results indicate that the Bn-groups in the compounds of the invention have the highest propensity for in-vivo oxidative degradation. After Bn-oxidation and cleavage, the regular MDP- or MTP-group is formed, and these groups will degrade in-vivo in a similar fashion as other MDP/MTPs - that are known in the art - do.

### Example 23. Nanobiologic Synthesis

### METHOD 1 - FILM

The phospholipids, (pro-)drug and optional triglycerides or polymer are dissolved (typically in chloroform, ethanol or acetonitrile). This solution is then evaporated under vacuum to form a film of the components. Subsequently, a buffer solution is added to hydrate the film and generate a vesicle suspension. The phospholipids, (pro-)drug and optional triglycerides or polymer are dissolved (typically in chloroform, ethanol or acetonitrile). This solution is infused - or added drop-wise - to a mildly heated buffer solution under stirring, until complete evaporation of the organic solvents, generating a vesicle suspension.

To the vesicle suspension, generated using A or B, apolipoprotein A-I (apoA-I) (note that apoA-I can also already be in B) - use dropwise to avoid denature, is added and the resulting mixture is sonicated for 30 minutes using a tip sonicator while being thoroughly cooled using an external ice-water bath. The obtained solution containing the nanobiologics and other by products is transferred to a Sartorius Vivaspin tube with a molecular weight cut-off depending on the estimated size of the nanobiologics (typically Vivaspin tubes with cut-offs of 10.000-100.000 kDa are used). The tubes are centrifuged until ~90 % of the solvent volume has passed through the filter. Subsequently, a volume of buffer, roughly equal to the volume of the remaining solution, is added and the tubes are spun again until roughly half the volume has passed through the filter. This is repeated twice after which the remaining solution is passed through a polyethersulfone 0.22 pm syringe filter, resulting in the final nanobiologic solution.

### METHOD 2 - MICROFLUIDICS

In an alternative approach, the phospholipids, (pro-)drug and optional triglycerides, cholesterol, steryl esters, or polymer are dissolved (typically in ethanol or acetonitrile) and loaded into a syringe. Additionally, a solution of apolipoprotein A-I (apoA-I) in phosphate buffered saline is loaded into a second syringe. Using microfluidics pumps, the content of both syringes is mixed using a microvortex platform. The obtained solution containing the nanobiologics and other by products is transferred to a Sartorius Vivaspin tube with a molecular weight cut-off depending on the estimate size of the particles (typically Vivaspin tubes with cut-offs of 10.000-100.000 kDa are used). The tubes are centrifuged until ~90 % of the solvent volume has passed through the filter. Subsequently, a volume of phosphate buffered saline roughly equal to the volume of the remaining solution is added and the tubes are spun again until roughly half the volume has passed through the filter. This is repeated twice after which the remaining solution is passed through a polyethersulfone 0.22 pm syringe filter, resulting in the final nanobiologic solution.

### METHOD 3 - MICROFLUIDIZER

In another method according to the invention, microfluidizer technology is used to prepare the nanoscale assembly and the final nanobiologic composition. Microfluidizers are devices for preparing small particle size materials operating on the submerged jet principle. In operating a microfluidizer to obtain nanoparticulates, a premix flow is forced by a high pressure pump through a so-called interaction chamber consisting of a system of channels in a ceramic block which split the premix into two streams. Precisely controlled shear, turbulent and cavitational forces are generated within the interaction chamber during microfluidization. The two streams are recombined at high velocity to produce shear. The so-obtained product can be recycled into the microfluidizer to obtain smaller and smaller particles. Advantages of microfluidization over conventional milling processes include substantial reduction of contamination of the final product, and the ease of production scaleup.

### Formulation 1

The below Table provides details on the preparation of HDL-derived nanoparticle formulations. First, DMPC, cholesterol and the compound of the invention were dissolved in the given molar ratios in ethanol (entries A, B and D) or in ethanol/DMSO 4/1 (entries C, E and F), while protein apoA-1 was separately dissolved in PBS buffer (pH=7.5). In these formulations, the amount of applied apoA-I was related to the amount of DMPC, by weight. The organic solution was mixed with the PBS buffer solution by bringing them together by T-junction mixing.

Purification of the resulting solutions was performed by TFF (tangential flow fractionation), thereby getting rid of the organic solvents and dissolving the nanoparticles in PBS. Concentration of the NP solutions was performed by spin-filter centrifugation. Finally, the HDL-derived nanoparticle solutions were filtered over 0.2 micrometer Acrodisk PES filters.

The final HDL-derived nanoparticle solutions had typical recoveries of the used compounds (Examples 2, 3, 7, 8 and 13), of DMPC and of cholesterol that exceeded 80%. Recoveries were determined by HPLC (for the compounds), and using assays that are known in the art (for DMPC and cholesterol). Concentrations of the final HDL-derived nanoparticle solutions were about 2 to 4 mg/mL in compound.

**Table 6: Formulation compositions of HDL-derived nanoparticles**

| **Entry** | **Compound** | **DMPC mol%** | **Compound mol%** | **Cholesterol mol%** | **APO-A1 * mg-to-mg** |
|---|---|---|---|---|---|
| **A** | None (Blank) | 90 | 0 | 10 | 1-to-2 |
| **B** | Example 2 | 90 | 10 | 10 | 1-to-2 |
| **C** | Example 3 | 90 | 10 | 10 | 1-to-2 |
| **D** | Example 7 | 90 | 10 | 10 | 1-to-2 |
| **E** | Example 8 | 90 | 10 | 10 | 1-to-2 |
| **F** | Example 13 | 90 | 10 | 10 | 1-to-2 |

| | | | | | |
|---|---|---|---|---|---|
| * APO-A1 is employed in about half the amount in weight (mg) as DMPC | | | | | |

The stability of the nano-biologics as assessed by dynamic light scattering (DLS). The formulations of entries A to F were characterized by DLS over a time period of 8 weeks. The nanoparticles in the Example 2, 3, 7 and 13 formulations had Z-averaged (intensity weighted mean hydrodynamic size) diameters of about 20, 30, 20 and 45 nm, respectively. The dimensions of these nanoparticles stayed constant in time, with also the dispersity in particle size (PDI) remaining constant. The unloaded particles (Entry A) were also stable in time (at about 30 nm diameter). The nanoparticles in the Example 8 formulation showed diameters that grew as of the 2-week time point to the 5-week time-point, from about 50 to about 225 nm. Dimensions stabilized as of the 5-week time point. Using other processing conditions, also this Example 8 material can most likely be formulated to stable 10 to 50 nm sized particles.

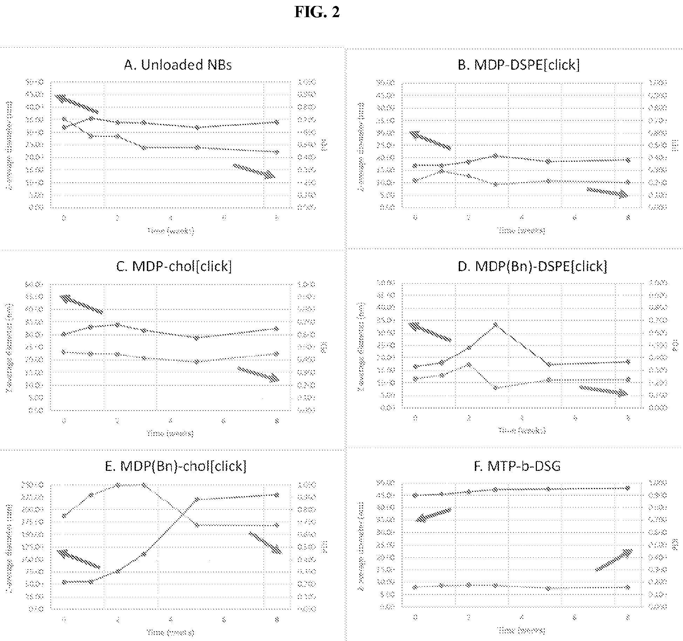

DLS-determined Z-averaged diameters and PDI-values for nanoparticles in formulations A to F are shown in **FIG.2****.**

*Tip sonication formulation A:* DSPC ([816-94-4]; 2.7 mg), cholesterol (0.26 mg) and the compound (0.46 mg) were dissolved in a glass vial with chloroform/methanol (9:1). The solvents were removed by an argon gas flow and the resulting film was dried in vacuo for >1h. A solution of apoA-I PBS (6 mL) was added to the vial, which was subsequently bath sonicated for 5 minutes, incubated at 37°C for 20 minutes, and then tip-sonicated for 10 minutes. The resulting dispersion was centrifuged to remove larger aggregates. The supernatant was transferred to a Vivapin 20 ultrafiltration unit (cutoff 10 kDa) and spun down to a volume of approximately 1 mL. The resulting dispersion was diluted with PBS and spun down to 1 mL, and this procedure was repeated twice. Finally, the volume was diluted to 2 mL using PBS to afford the desired nanoparticle solution.

*Tip sonication formulation B:* DMPC (2.7 mg), cholesterol (0.30 mg) and the compound (0.57 mg) were dissolved in a glass vial with chloroform/methanol (9:1). The solvents were removed by an argon gas flow and the resulting film was dried in vacuo for >1h. A solution of peptide-2F (an apoA-I mimetic 18-mer; sequence 257 in Table 2) in PBS (6 mL) was added to the vial, which was subsequently bath sonicated for 5 minutes, incubated at 37°C for 20 minutes, and then tip-sonicated for 5 minutes. The resulting dispersion was centrifuged to remove larger aggregates. The supernatant was transferred to a Vivapin 20 ultrafiltration unit (cutoff 10 kDa) and spun down to approximately 1 mL. The resulting dispersion was diluted with PBS and spun down to 1 mL, and this procedure was repeated twice. Finally, the volume was diluted to 2 mL using PBS to afford the desired nanoparticle solution.

*T-junctionformulation C:* DMPC, cholesterol and the compound were dissolved in ethanol, while apoA-1 was dissolved in PBS buffer (pH=7.5). The organic solution was mixed with the buffer solution applying T-junction mixing. Purification of the resulting solutions was performed by TFF (tangential flow filtration), thereby getting rid of the organic solvents. Samples were concentrated by spin-filtration. The final HDL-derived nanoparticle solutions had typical recoveries for compound, DMPC and cholesterol that exceeded 75%. Concentrations of the final HDL-derived nanoparticle solutions were about 2 to 4 mg/mL in the compound.

**Table 7. Formulations of HDL-derived nanoparticle**

| **Entry** | **PC-type** | **Compound** | **Cholesterol** | **apoA-I or 2F** | **DLS ^{#} nm** |
|---|---|---|---|---|---|
| | **mol%** | **mol%** | **mol%** | *** mg-to-mg** | **(error)** |
| **A** | DSPC 100 | Example 2 10 | 20 | apoA-I 2-to-5 * | 51 (19) |
| **B** | DMPC 100 | Example 7 10 | 20 | 2F 2-to-5 * | 12.9 (0.7) |
| **C** | DMPC 80 | Example 2 20 | 20 | apoA-I 1-to-1 ** | 16.4 (4.2) |

| | | | | | |
|---|---|---|---|---|---|
| *apoA-I or the 2F-peptide are employed in mg-to-mg phosphocholine PC); ** apoA-I is employed in mg-to-mg compound; ^{#} number averaged diameter. | | | | | |

The above examples highlight, that DSPC can be used instead of DMPC (POPC can also be employed e.g.), that peptidomimetics instead of apoA-I can be used, and that high levels of compounds of the disclosure can be incorporated. Furthermore, tip-sonication can be used as processing technique, instead of e.g. T-junction mixing or micro-fluidic mixing.

APOLIPOPROTEIN A-I (apoA-I) ISOLATION Human apoA-I was isolated from human HDL concentrates (Bioresource Technology) following a previously described procedure (Zamanian-Daryoush et al., 2013). Briefly, a potassium bromide solution (density: 1.20 g/mL) was layered on top of the concentrate and purified HDL was obtained by ultracentrifugation. The purified fraction was added to a chloroform/methanol solution for delipidation. The resulting milky solution was filtered and the apoA-I precipitate was allowed to dry overnight. The protein was renatured in 6 M guanidine hydrochloride, and the resulting solution dialyzed against PBS. Finally, the apoA-I PBS solution was filtered through a 0.22 pm filter and the protein's identity and purity were established by gel electrophoresis and size exclusion chromatography.

### Example 24. Cryo-TEM measurements on HDL-derived nanoparticle formulations

The molecule compounds of Example 2 and Example 7, respectively, i.e. MDP-DSPE[click] and MDP(Bn)-DSPE[click] were formulated together with 1,2-dimyristoyl-sn-glycero-3-phosphocholine (DMPC; (CAS [18194-24-6]) and APO-A1, and with varying amounts of cholesterol, to create HDL-derived nanoparticle formulations. The following table shows the employed relative molar amounts of the DMPC, compound and cholesterol components. APO-A1 was used in twice the amount (in mg) as the compound of the invention (in mg). The dynamic scattering data (DLS) of the processed formulation are also given: the recorded diameter and its error in brackets are based on the number-averaged DLS data. The DLS-recorded polydispersity in the dimensions of the particles is also given.

**Table 8: Formulations for Cryo-TEM measurements**

| Entry | Compound | DMPC | Compound | Cholesterol | APO-A1 * | DLS | DLS |
|---|---|---|---|---|---|---|---|
| | | mol% | mol% | mol% | mg-to-mg | nm (error) | PDI |
| **A** | Example 2 | 90 | 10 | 0 | 2-to-1 | 10.1 (2.1) | 0.42 |
| **B** | Example 7 | 90 | 10 | 0 | 2-to-1 | 9.9 (1.8) | 0.57 |
| **C** | Example 2 | 90 | 10 | 10 | 2-to-1 | 11.9 (3.0) | 0.32 |
| **D** | Example 7 | 90 | 10 | 10 | 2-to-1 | 11.4 (2.1) | 0.42 |
| **E** | Example 2 | 90 | 10 | 20 | 2-to-1 | 22.2 (6.1) | 0.18 |
| **F** | Example 7 | 90 | 10 | 20 | 2-to-1 | 31.0 (6.0) | 0.48 |

| | | | | | | | |
|---|---|---|---|---|---|---|---|
| * APO-A1 is employed in twice the amount in weight (mg) as the compound | | | | | | | |

Formulation: DMPC, cholesterol and the compound were dissolved in ethanol (entries A, C and E) or in ethanol/DMSO (entries B, D, F), while apoA-1 was dissolved in PBS buffer (pH=7.5).The organic solution was mixed with the buffer solution applying T-junction mixing. Purification of the resulting solutions was performed by TFF (tangential flow filtration), thereby getting rid of the organic solvents. Samples were concentrated by spin-filtration. The final HDL-derived nanoparticle solutions had typical recoveries of the used compounds (Example 2 and 7), DMPC and cholesterol that exceeded 75%. Recoveries were determined by HPLC (for the compounds), and using assays that are known in the art (for DMPC and cholesterol). Concentrations of the final HDL-derived nanoparticle solutions were about 2 to 4 mg/mL in the compound (Example 2 or 7).

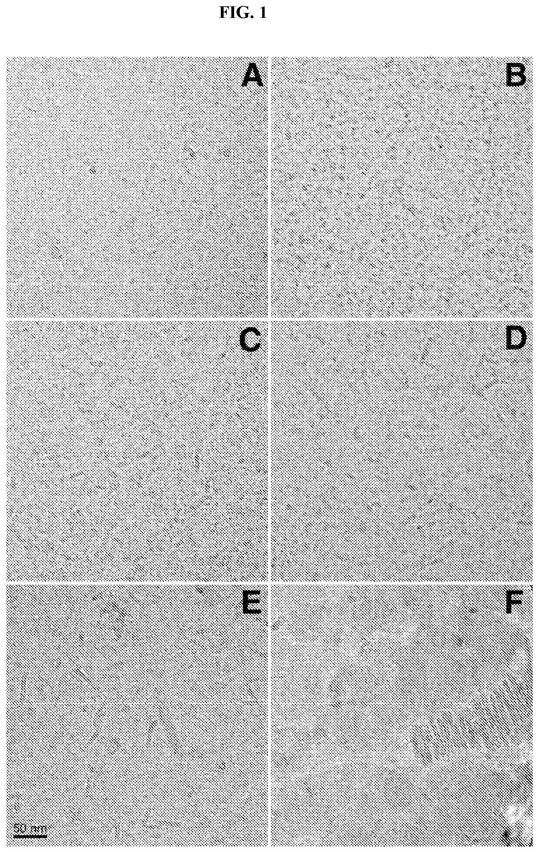

**FIG. 1** shows the recorded Cryo-TEM pictures for the HDL-derived nanoparticle of entries A to F. The 50 nm bar applies to all 6 pictures. When 0% cholesterol is used, spherical disc-like particles are dominantly observed (A and B; sizes about 5 to 10 nm). Applying 10% of cholesterol mostly shows slightly extended discs (C and D; lengths about 5 to 25 nm and thicknesses about 5 nm). Using 20% cholesterol shows clearly extended worm-like particles. The lengths of these worm-like particles is about 20 to about 50 nm and the thicknesses are about 5 nm (picture C). In picture F, the lengths of the worms are about 50 to 100 nm; again, the thicknesses are about 5 nm. In F one also observes that the worm-like particles aggregate to larger stacks.

The results highlight that the HDL-derived nanoparticle particle dimensions can be steered using the cholesterol content of the formulation (compare C with E, and D with F), but also with the lipophilicity of the compound and/or the substitution of the R₂ position in Formula (I) of the compounds of the invention (clear: compare E with F; less clear: in D the particles seem to be a bit more extended than in C).

Method: Just before processing the samples, 200-mesh lacey carbon supported copper grids (Electron Microscopy Sciences) were surface plasma treated for 40 seconds using a Cressington 208 carbon coater. Next, 3 µL of the HDL-derived nanoparticle sample solutions was transferred to the grids. A thin film of sample solution was then vitrified on the grid by plunge vitrification in liquid ethane, using an automated vitrification robot (FEI Vitrobot Mark IV). Processed films were stored until measurement took place. Cryo-TEM imaging of the prepared films was carried out on a CryoTITAN microscope (Thermo Fisher) equipped with a field emission gun (FEG), a post-column Gatan imaging filter (model 2002), and a post-GIF 2k × 2k Gatan CCD camera (model 794).

### Example 25: In vitro NOD2 activation assay

The stimulation of human NOD2 (hNOD2) by compounds disclossed herein was studied by monitoring activation of NF-κB in HEK-Blue^{™} hNOD2 cells (Invitrogen). 50,000 HEK-Blue^{™} hNOD2 cells were seeded in HEK-Blue^{™} Detection Medium in flat-bottom tissue culture plates. Concentration ranges of the test article (compounds diluted from DMSO-solutions (17.8 mmol/L), first with demineralized water and then with PBS to the desired concentrations) were added to the cells in the tissue culture plates. Cells were incubated overnight at 37°C and 5% CO₂. The following day, supernatants were collected in an ELISA plate and the OD was measured at 620 nm using a spectrophotometer.

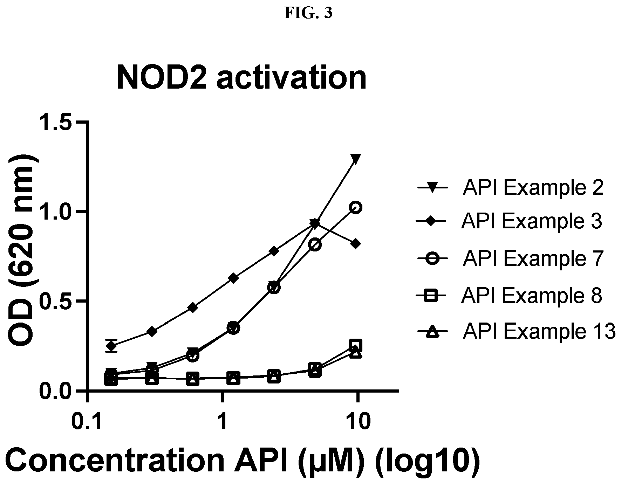

The signal in this assay is based on NOD2 stimulation with a ligand which subsequently activates NF-κB and AP-1, resulting in the production of SEAP. Levels of SEAP were then determined with HEK-Blue^{™} Detection medium (Invitrogen). The hydrolysis of the substrate in the medium by SEAP produces a purple/blue color that was then measured with an Absorbance microplate reader. OD values are mapped based on test article concentration and are depicted in **FIG. 3****.** Assay performance was validated with muramyl dipeptide (CAS number [53678-77-6]).

All tested compounds of the invention (APIs) are capable of activating NOD2. Potency for Example 2, 3 and 7 compounds is comparable. Example 8 and 13 compounds are also able to activate NOD2, but seemingly to a lesser extent than the Example 2, 3 and 7 compounds.

### Example 26: combination activity of (i) HDL-derived nanoparticles and (ii) immune checkpoint inhibition in the B16F10 mouse model

Formulations in-vivo study 1.

The formulations for these in-vivo studies were prepared by T-junctions mixing, followed by tangential flow filtration (TFF). Processing and purification procedures were applied as those highlighted in the formulations for the Cryo-TEM measurements (Table 8).

Compounds used were those of Example 2, 3, 7, 8 and 13 (i.e. these Example materials are collectively named APIs in these descriptions of the in-vivo studies). For the 5 administered formulations, the following relative ratio of components have been used.

| **Entry** | **Compound** | **DMPC** | **Compound** | **Cholesterol** | **apoA-1*** |
|---|---|---|---|---|---|
| | | Mol% | Mol% | Mol% | mg-to-mg |
| **1** | Example 2 | 90 | 10 | 10 | 2-to-1 |
| **2** | Example 3 | 90 | 10 | 10 | 2-to-1 |
| **3** | Example 7 | 90 | 10 | 10 | 2-to-1 |
| **4** | Example 8 | 90 | 10 | 10 | 1-to-1 |
| **5** | Example 1 | 90 | 10 | 10 | 1-to-1 |

| | | | | | |
|---|---|---|---|---|---|
| * relative to the compound | | | | | |

### Protocol and results

The panel of Example APIs was formulated to HDL-derived nanoparticles (or nanobiologics, NBs) that were screened for their anti-tumor activity in combination with immune checkpoint inhibitors in the B16F10 syngeneic mouse tumor model. To this end, B16F10 murine melanoma cells were cultured in Dulbecco's modified Eagle's medium (DMEM)(Gibco) supplemented with 10% FBS (Gibco) and 1% penicillin/streptomycine (P/S). At the day of injection, cells were harvested and resuspended at 1x10⁶ viable cells/mL in PBS with 0.5% FBS. During counting the cells were checked for viability using Trypan Blue Solution, 0.4% (Gibco). At the start of the experiment 1x10⁵ B16F10 tumor cells in 100 µL PBS supplemented with 0.5% fetal bovine serum (FBS) were injected subcutaneously in the flank of 7-week-old female C57BL/6 mice (The Jackson Laboratory).

Seven days after tumor inoculation, mice were randomized in groups with similar average group size (n=10). Average tumor size of groups was 3.26 mm³. After randomization mice were ear notched and weighed. Subsequently doses were calculated and aliquoted. Aliquoted doses were stored until use at 4° C.

The study consisted of the following: a PBS control group, an immune checkpoint inhibitors group (CI) and 6 treatment groups. The immune checkpoint inhibitors treated mice received an intraperitoneal injection on day 2, 4 and 8, using doses of 200 µg anti-CTLA-4 (clone, 9H10, BioXcell) and/or 200 µg anti-PD-1 (clone, RMP1-14, BioXcell). Treatment groups consisted of NBs prepared from the Example 2 compound, NBs from Example 3, NBs from Example 7, NBs from Example 8, NBs from Example 13 combined with the immune checkpoint inhibitors therapy (CI) as described above. Dosing for treatment groups was about 9 mg MDP/kg (or about 27 mg/kg of the respective APIs contained in the NBs) in question on day 0, 2 and 4.

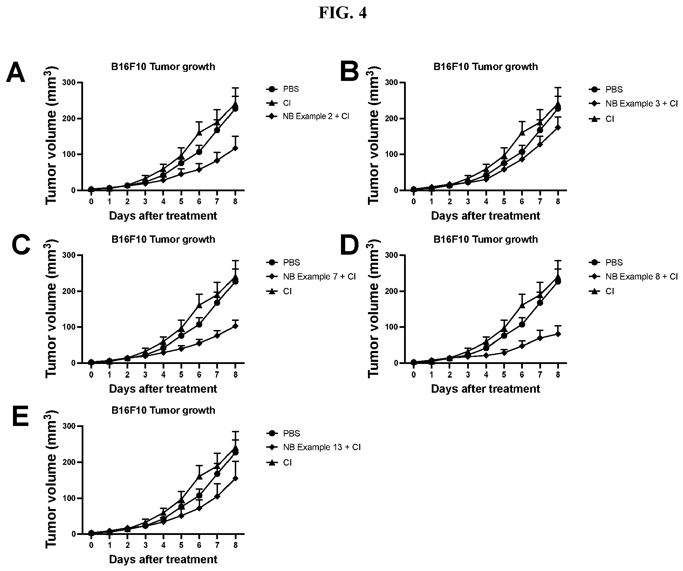

Tumor growth curves are depicted in FIG. 4A**-E** and in each graph the same PBS and CI groups have been mapped to allow for comparison between graphs.

PBS treated animals or animals that were treated with immune checkpoint inhibitors alone did not show tumor growth inhibition. The groups of animals treated with combination therapy all showed tumor growth inhibition and it was most pronounced for those groups in which NBs of Example 2 **(****FIG. 4A****),** NBs of Example 7 **(****FIG. 4C****)** or NBs of Example 8 **(****FIG. 4D****)** was part of the combination therapy.
Note that the above results on the combination therapy were obtained with an applied formulation that contained 10 mol% cholesterol relative to the applied 90 mol% DMPC (see the above Table), and thus these particles had dimensions of approximately 5 to maximally 10 nm (see the Cryo-TEM panels C and D in Figure 1).

### Example 27: Single agent activity of nanobiologies in B16F10 mouse model

The formulations for these in-vivo studies were prepared by T-junctions mixing, followed by tangential flow filtration (TFF). Processing and purification procedures were applied as those highlighted in the formulations for the Cryo-TEM measurements (Table 8). The below table shows the employed relative ratios of components to prepare the HDL-derived nanoparticles.

| **Entry** | **Compound** | **DMPC** | **Compound** | **Cholesterol** | **apoA-1*** | **DLS #** | **DLS #** |
|---|---|---|---|---|---|---|---|
| | | Mol% | Mol% | Mol% | mg-to-mg | (nm) | PDI (-) |
| **1** | Example 2 | 90 | 10 | 10 | 2-to-1 | 11.9 (3.0) | 0.32 |
| **2** | Example 7 | 90 | 10 | 10 | 2-to-1 | 11.4 (2.1) | 0.42 |
| **3** | Example 2 | 90 | 10 | 20 | 2-to-1 | 22.2 (6.1) | 0.18 |
| **4** | Example 2 | 80 | 20 | 20 | 1-to-1 | 16.4 (4.2) | 0.21 |
| **5** | Example 7 | 80 | 20 | 20 | 1-to-1 | 24.4 (4.4) | 0.47 |

| | | | | | | | |
|---|---|---|---|---|---|---|---|
| * relative to the compound; # number averaged diameters. | | | | | | | |

### Protocol and results

Two APIs (the Example 2 and Example 7 compounds) were used to generate a set of different HDL-derived nanoparticle formulations (or nanobiologic formulations; nanobiologics; NBs) to determine their potencies. The resulting nanobiologics were screened for their single agent anti-tumor activity in the B16F10 syngeneic mouse tumor model. To this end B16F10 murine melanoma cells were cultured in Dulbecco's modified Eagle's medium (DMEM)(Gibco) supplemented with 10% FBS (Gibco) and 1% penicillin/streptomycine (P/S). The day of injection cells were harvested and resuspended at 1x10⁶ viable cells/mL in PBS with 0.5% FBS. During counting cells were checked for viability using a Cell Counter and Analyzer (Casy). At the start of the experiment 1x10⁵ B16F10 tumor cells in 100 µL PBS supplemented with 0.5% fetal bovine serum (FBS) were injected subcutaneously in the flank of 7-week-old female C57BL/6J mice (Charles River).

Seven days after tumor inoculation, mice were randomized in groups with similar average group size. Groups consisted of 8-10 mice. Average tumor size of groups was 6.33 mm³. After randomization mice were tattooed with a number on the tail. Subsequently doses were calculated and aliquoted. Aliquoted doses were stored until use at 4° C.

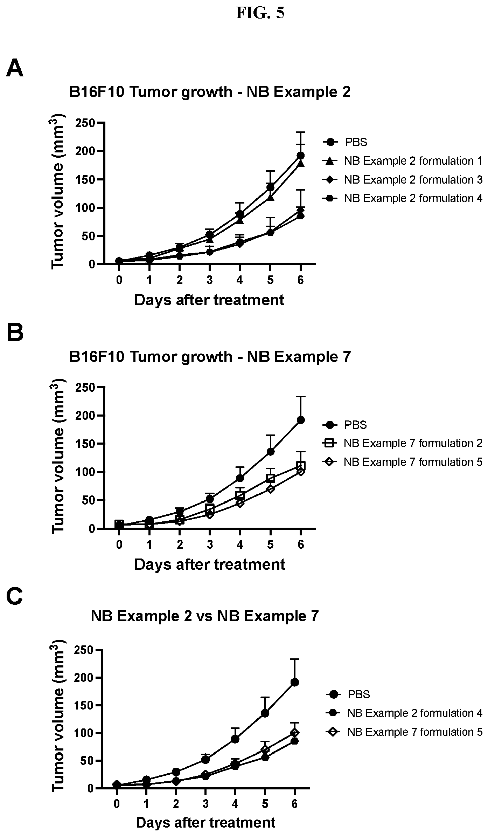

The study consisted of the following: a PBS control group and 5 treatment groups: NBs of Example 2 (formulation Entries 1, 3 and 4) and NBs of Example 7 (formulation Entries 2 and 5). Dosing for treatment groups was about 3 mg MDP/kg (or about 9 mg/kg of the respective APIs contained in the NBs) in question on day 0, 2 and 4. Tumor size was measured at set times during the course of the study. Tumor growth curves are depicted in FIG. 5A**-C** and in each graph the same PBS group is mapped to allow for comparison between graphs. Apart from the NBs of Example 2 (formulation Entry 1), all nanobiologics clearly showed a reduction in the tumor growth in comparison with the PBS control group. For both Example 2 **(****FIG. 5A****)** and Example 7 **(****FIG. 5B****)** the nanobiologics formulations with 20 mol% cholesterol performed best. **FIG. 5C** shows that NBs of Example 2 slightly outperform NBs of Example 7 (using the formulations of Entries 4 and 5, respectively).

Note that in Figure **FIG. 5A****,** the Example-2 formulation containing 10 mol% cholesterol (Entry 1 in the above Table) showed a minor single agent activity (compare to PBS). Surprisingly, and in comparison, the Example-2 formulations with 20 mol% cholesterol (Entries 3 and 4 in the above Table) show extraordinarily enhanced tumor suppressions. For the Example-7 formulations, this can also be seen **(****FIG. 5B****).** Apparently, the dimensions of the formed particles play a decisive role in the activity of the prepared HDL-derived nanoparticles: the 10% cholesterol formulations give spherical or only slightly stretched disk-like particles with 5 to 10 nm dimensions, while the 20% cholesterol formulations give elongated worm-like particles about 15 to 50 nm in length, and about 5 nm in thickness (compare panels C and E in Figure 1; cryo-TEM data).

The disclosed HDL-derived nanoparticles have certain dimensions, and these features find particular use in producing stable and potent HDL-derived NPs. Without being bound by theory, it is thought that the disclosed HDL-derived nanoparticles provide improved (multivalent) presentation of the MDP (or MDP(Bn) or MTP or MTP(Bn)) moieties to cells, dramatically improving their potency.

## Claims

1. A compound of formula (I): 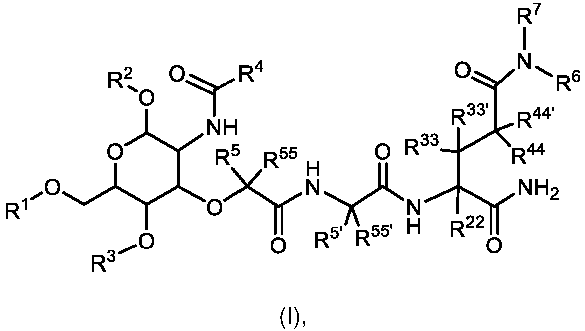
or a pharmaceutically acceptable salt thereof,
wherein:
R¹ is -H or -C(O)-R^{X};
R² and R³ are each independently selected from the group consisting of -H, alkyl, aryl, alkylene-aryl, -C(O)-alkyl, and -C(O)-aryl;
R⁴, R⁵, and R^{5'} are each alkyl;
R⁶ and R¹¹ are each independently -H, or alkyl;
R⁷ is -Y-triazolyl-L;
Y is alkylene;
L is selected from the group consisting of a fatty acid chain, -alkylene-C(O)-W, - alkylene-O-C(O)-W, -alkylene-N-(alkylene-C(O)-NR¹¹-alkylene-NR¹¹-C(O)-W)₂, and - alkylene-N-(alkylene-C(O)-W)₂;
W is a fatty acid chain, -O-alkylene-C(H)(OR⁸)-alkylene-OR⁹, a phospholipid, or a sterol;
R⁸ and R⁹ are each independently R^{X} or -C(O)-R^{X};
R²², R³³, R^{33'}, R⁴⁴, R^{44'}, R⁵⁵, and R^{55'} are each independently H or R^{A};
R^{X} is a fatty acid chain;
wherein each aforementioned alkyl, alkylene, alkylene-aryl, aryl, and triazolyl is optionally substituted with one or more R^{A}, wherein R^{A} is independently selected for each occurrence from the group consisting of hydrogen, halo, alkoxy, haloalkoxy, cyano, hydroxyl, -N(R^{C})(R^{D}), -C(O)N(R^{C})(R^{D}), -N(R^{C})C(O)R^{B}, -OC(O)NR^{C}R^{D}, -NR^{C}C(O)OR^{B}, - OC(O)R^{B}, -C(O)OR^{B}, -C(O)R^{B} , -CO₂H, -NO₂, -SH, S(O)ₓR^{B}, aryl, arylalkyl, heteroaryl, heteroarylalkyl, cycloalkyl, heterocycloalkyl, and R^{B}; wherein X is 0, 1, or 2;
R^{C} and R^{D} are independently selected for each occurrence from the group consisting of hydrogen, alkyl, haloalkyl -C(O)R^{B}, and -C(O)OR^{B}; or R^{C} and R^{D} are taken together with the nitrogen to which they are attached to form a heterocyclic ring optionally substituted with R^{A}; and
R^{B} is alkyl, alkenyl, or alkynyl optionally substituted with one or more fluoro.

2. The compound of claim 1, wherein the compound of formula (I) is a compound of formula (IA): 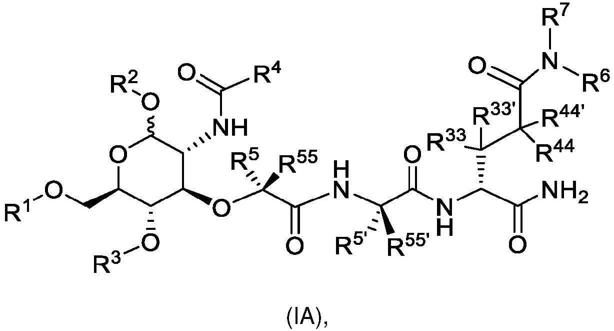 or a pharmaceutically acceptable salt thereof.

3. The compound of claim 1 or 2, wherein R² is -H or benzyl; optionally wherein R² is -H.

4. The compound of any one of claims 1-3, wherein R²², R³³, R^{33'}, R⁴⁴, R^{44'}, R⁵⁵, and R^{55'} are each -H.

5. The compound of any one of claims 1-4, wherein R⁴ is alkyl; optionally methyl.

6. The compound of any one of claims 1-5, wherein R³ and R⁶ are both -H.

7. The compound of any one of claims 1-6, wherein Y is -CH₂-.

8. The compound of any one of claims 1-7, wherein the compound of formula (1) is:
(a) a compound of formula (II): 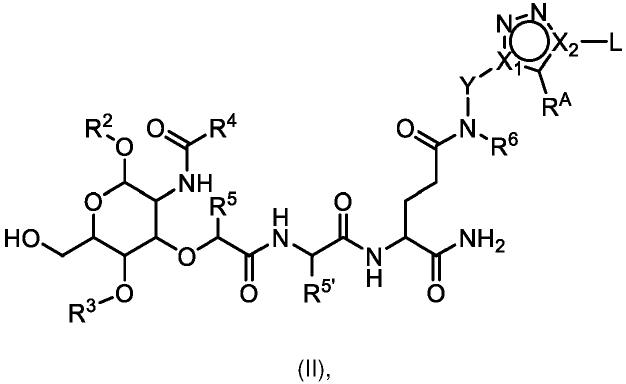 or a pharmaceutically acceptable salt thereof;
or (b) a compound of formula (II-1): 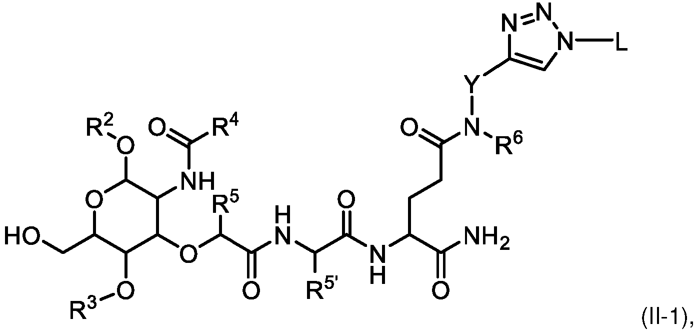 or a pharmaceutically acceptable salt thereof;
or
(c) a compound of formula (IIA): 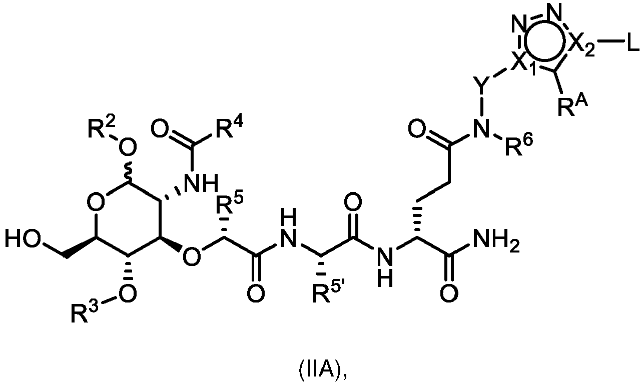 or a pharmaceutically acceptable salt thereof;
wherein: X₁ is -N- and X₂ is -C-; or X₁ is -C- and X₂ is -N-.

9. The compound of claim 8, wherein Y is C₁₋₆alkylene.

10. The compound of any one of claims 8-9, wherein R^{A} is -H.

11. The compound of any one of claims 1-10, wherein L is-CH₂-C(O)-W.

12. The compound of any one of claims 1-11, wherein W is a C₈₋₃₀alkyl.

13. The compound of any one of claims 1-11, wherein W is: 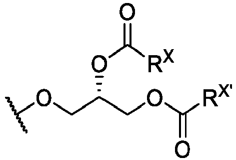 wherein R^{X} and R^{X'} are each independently a -C₈₋₃₀fatty acid chain; optionally wherein R^{X} and R^{X'} are each independently a -C₈₋₃₀alkyl; further optionally wherein R^{X} and R^{X'} are both -(CH₂CH₂)₈-CH₃.

14. The compound of any one of claims 1-11, wherein W is:
(a) cholesterol: 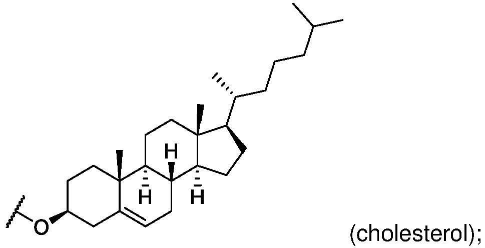 or
(b) wherein W is a phospholipid selected from the group consisting of: a phosphatidylcholine (PC), a phosphatidylglycerol (PG), a phosphatidylserine (PS), a phosphatidylethanolamine (PE), a phosphatidic acid (PA), and a lysophosphatidylcholine.; or
(c) a phospholipid having the structure: wherein 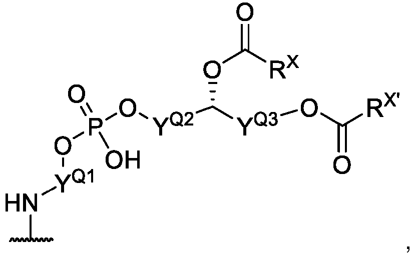 Y^{Q1}, Y^{Q2}, and Y^{Q3} are each independently alkylene and R^{X} and R^{X'} are each independently a fatty acid chain having at least 15 carbons; optionally wherein R^{X} and R^{X'} are each independently a C₁₅₋₂₀ alkyl or a C₁₅₋₂₀ alkenyl; or
(d) a phospholipid having the structure: 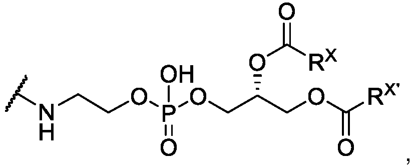 or a pharmaceutically acceptable salt thereof; wherein R^{X} and R^{X'} are each independently an optionally saturated C₈₋₃₀ fatty acid chain; optionally wherein R^{X} and R^{X'} are each independently an optionally saturated C₁₂₋₁₈ fatty acid; further optionally wherein R^{X} and R^{X'} are both -(CH₂CH₂)₈-CH₃.

15. The compound of any one of claims 1-14 selected from the group consisting of: 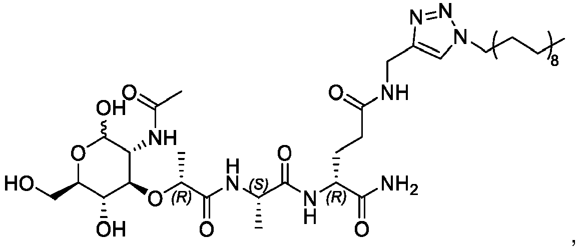 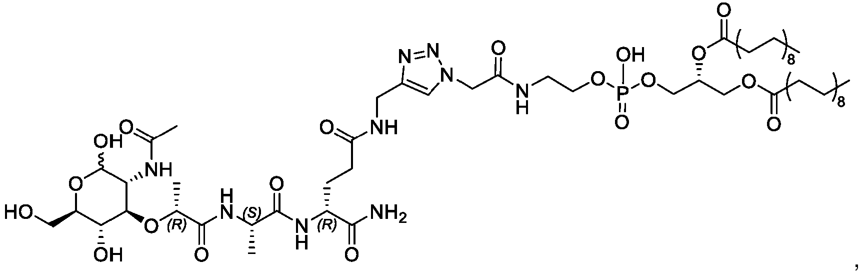 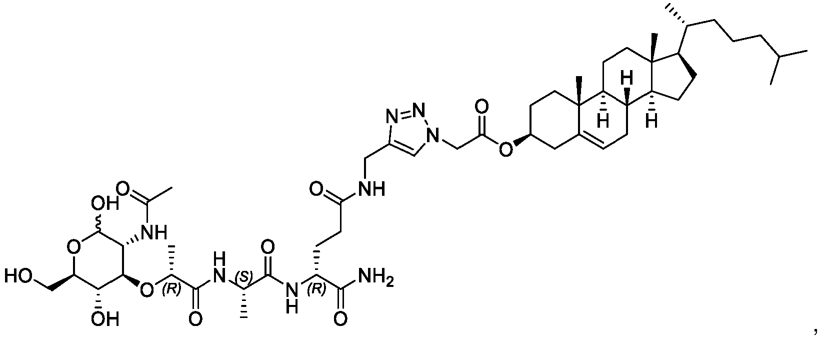 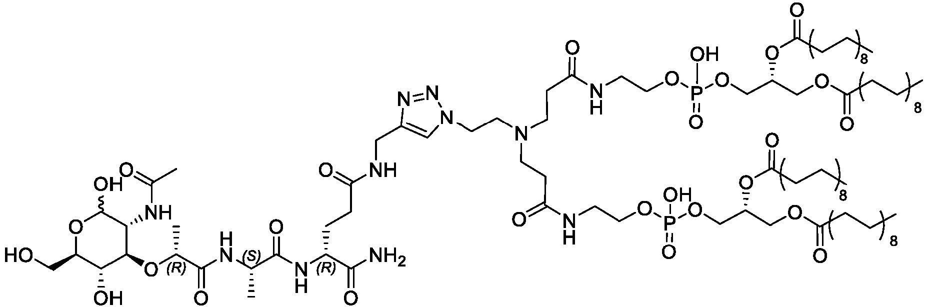 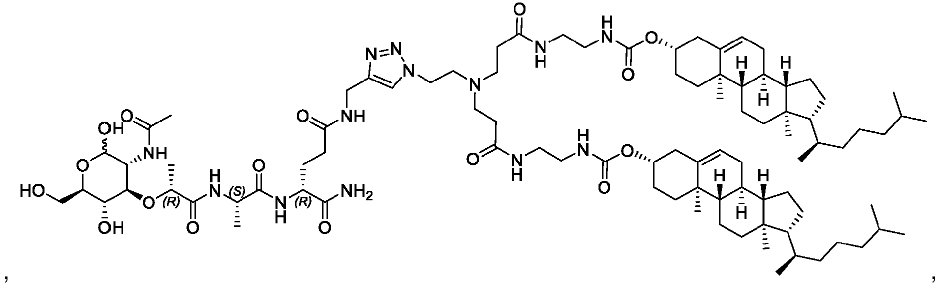 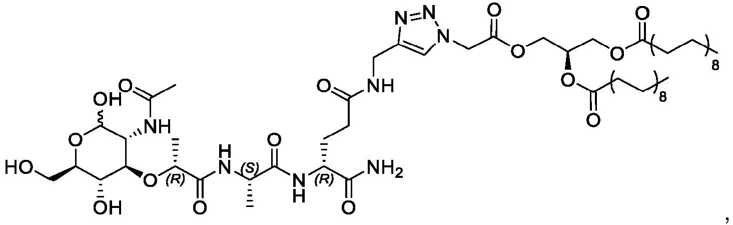 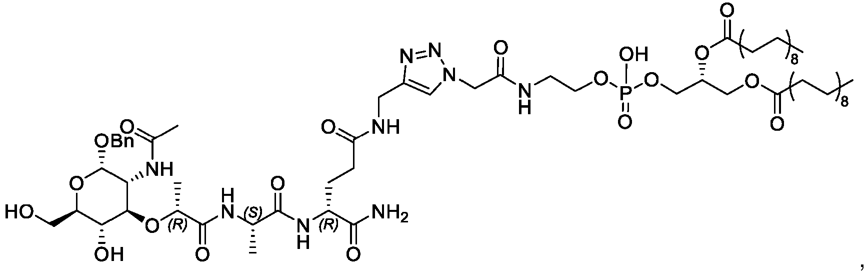 and 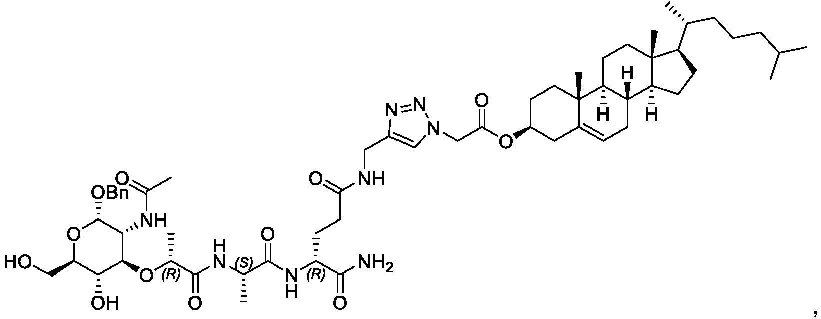 and a pharmaceutically acceptable salt thereof, wherein Bn is a benzyl group.

16. The compound of claim 15, wherein the compound is:
(a) 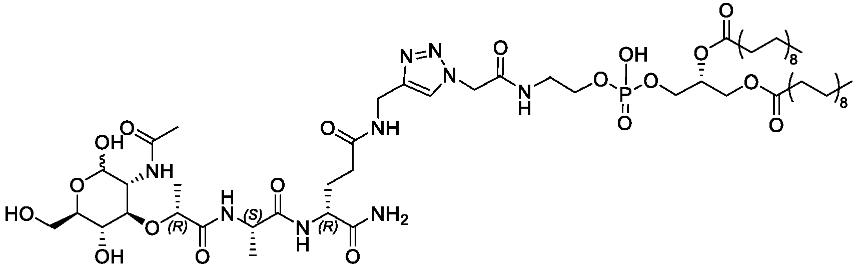 or a pharmaceutically acceptable salt thereof; or
(b) 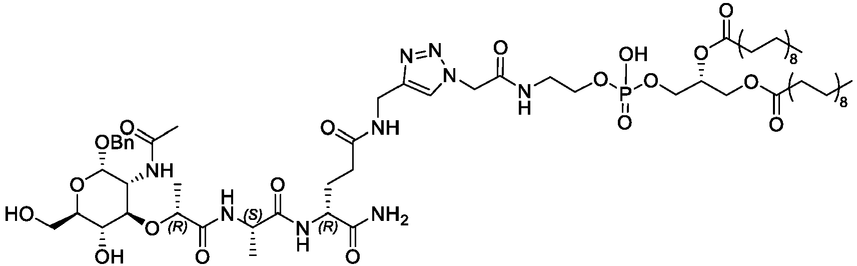 or a pharmaceutically acceptable salt thereof; or
(c) 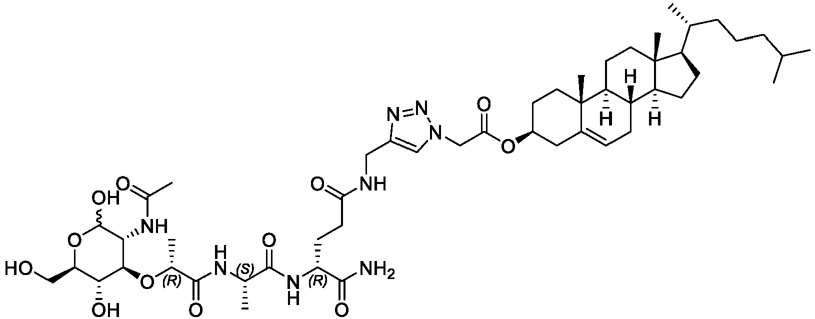 or a pharmaceutically acceptable salt thereof; or
(d) 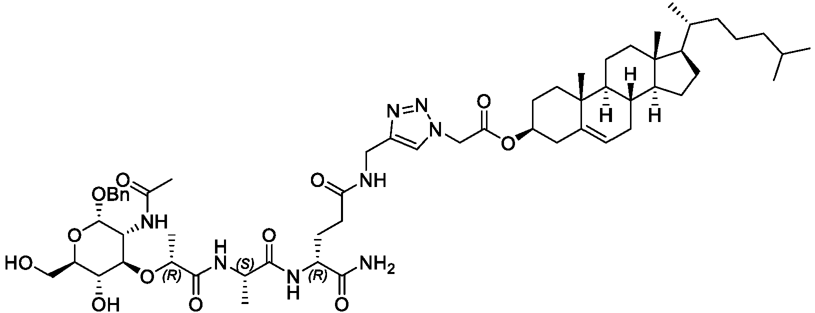 or a pharmaceutically acceptable salt thereof.

17. A nanobiologic composition, comprising a high-density lipoprotein (HDL)-derived nanoparticle, wherein the nanoparticle comprises one or more phospholipids and a compound of any one of claims 1-16.

18. The nanobiologic composition of claim 17, wherein the phospholipid is independently selected from the group consisting of a phosphatidylcholine, a phosphatidylethanolamine, a phosphatidylinositol, a phosphatidylserine, a sphingomyelin or other ceramide, a phospholipid-containing oil, a phosphatidylglycerol, a phosphatidic acid, a lysophosphatidylcholine, and combinations thereof.

19. The nanobiologic composition of claim 17, wherein the nanoparticle comprises:
(a) a phospholipid which is I,2-dimyristoyl-sn-glycero-3-phosphatidylcholine (DMPC) and a lysolipid which is I-myristoyl-2-hydroxy-sn-glycero-phosphocholine (MHPC); or
(b) a phospholipid which is 1-palmitoyl-2-oleoyl-sn-glycero-3-phosphocholine (POPC), and a lysolipid which is 1-palmitoyl-2-hydroxy-sn-glycero-3-phosphocholine (PHPC).

20. The nanobiologic composition of any one of claims 17-19, wherein the HDL-derived nanoparticle further comprises:
(a) apoA-I or a peptide mimetic of apoA-I; and/or
(b) cholesterol;
optionally wherein the HDL-derived nanoparticle comprises one or more phospholipids and cholesterol in a molar ratio in the range of about 1:0.05 to about 1:0.25.

21. A pharmaceutical composition comprising a compound of any one of claims 1-16 or a pharmaceutically acceptable salt thereof, and a pharmaceutically acceptable carrier.

22. A nanobiologic composition of any one of claims 17-20 or pharmaceutical composition of claim 21 for use in a method for treating a cell proliferation disorder in a patient in need thereof.

## Patentansprüche

1. Verbindung der Formel (I): 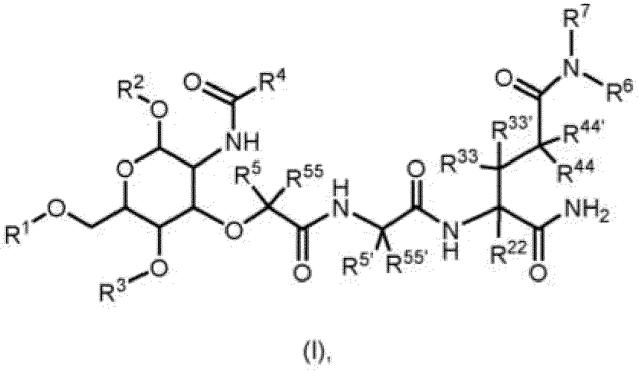
oder pharmazeutisch verträgliches Salz davon,
wobei:
R¹ -H oder -C(O)-R^{X} ist;
R² und R³ jeweils unabhängig voneinander aus der Gruppe ausgewählt sind bestehend aus -H, Alkyl, Aryl, Alkylenaryl, -C(O)-Alkyl und -C(O)-Aryl;
R⁴, R⁵ und R^{5'} jeweils Alkyl sind;
R⁶ und R¹¹ jeweils unabhängig voneinander -H oder Alkyl sind;
R⁷ -Y-Triazolyl-L ist;
Y Alkylen ist;
L ausgewählt ist aus der Gruppe bestehend aus einer Fettsäurekette, -Alkylen-C(O)-W, -Alkylen-O-C(O)-W, -Alkylen-N-(Alkylen-C(O)-NR¹¹-Alkylen-NR¹¹-C(O)-W)₂ und -Alkylen-N-(Alkylen-C(O)-W)₂;
W eine Fettsäurekette, -O-Alkylen-C(H)(OR⁸)-Alkylen-OR⁹, ein Phospholipid oder ein Sterol ist;
R⁸ und R⁹ jeweils unabhängig voneinander R^{X} oder -C(O)-R^{X} sind;
R²², R³³, R³³, R⁴⁴, R^{44'}, R⁵⁵ und R^{55'} jeweils unabhängig voneinander H oder R^{A} sind;
R^{X} eine Fettsäurekette ist;
wobei jedes der oben genannten Alkyl, Alkylen, Alkylenaryl, Aryl und Triazolyl gegebenenfalls mit einem oder mehreren R^{A} substituiert ist, wobei R^{A} für jedes Vorkommen unabhängig aus der Gruppe ausgewählt ist, bestehend aus Wasserstoff, Halogen, Alkoxy, Haloalkoxy, Cyano, Hydroxyl, -N(R^{C})(R^{D}), -C(O)N(R^{C})(R^{D}), -N(R^{C})C(O)R^{B}, -OC(O)NR^{C}R^{D}, -NR^{C}C(O)OR^{B}, -OC(O)R^{B}, -C(O)OR^{B}, -C(O)R^{B}, -CO₂H, -NO₂, -SH, S(O)ₓR^{B}, Aryl, Arylalkyl, Heteroaryl, Heteroarylalkyl, Cycloalkyl, Heterocycloalkyl und R^{B}, wobei X 0, 1 oder 2 ist;
R^{c} und R^{D} für jedes Vorkommen unabhängig aus der Gruppe ausgewählt sind, bestehend aus Wasserstoff, Alkyl, Haloalkyl -C(O)R^{B} und -C(O)OR^{B}; oder R^{c} und R^{D} zusammen mit dem Stickstoff, an den sie gebunden sind, genommen werden, um einen heterocyclischen Ring zu bilden, der gegebenenfalls mit R^{A} substituiert ist; und
R^{B} Alkyl-, Alkenyl- oder Alkinyl ist, gegebenenfalls substituiert mit einem oder mehreren Fluorresten.

2. Verbindung nach Anspruch 1, wobei die Verbindung der Formel (I) eine Verbindung der Formel (IA): 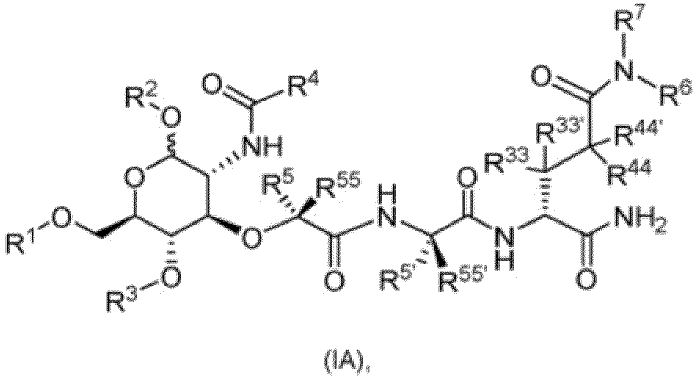 oder ein pharmazeutisch verträgliches Salz davon ist.

3. Verbindung nach Anspruch 1 oder 2, wobei R² -H oder Benzyl ist; wobei R² gegebenenfalls -H ist.

4. Verbindung nach einem der Ansprüche 1 - 3, wobei R²², R³³, R³³, R⁴⁴, R^{44'}, R⁵⁵ und R^{55`} jeweils -H sind.

5. Verbindung nach einem der Ansprüche 1-4, wobei R⁴ Alkyl ist; gegebenenfalls Methyl.

6. Verbindung nach einem der Ansprüche 1 - 5, wobei R³ und R⁶ beide -H sind.

7. Verbindung nach einem der Ansprüche 1 - 6, wobei Y -CH₂- ist.

8. Verbindung nach einem der Ansprüche 1 - 7, wobei es sich bei der Verbindung der Formel (I) handelt um:
(a) eine Verbindung der Formel (II): 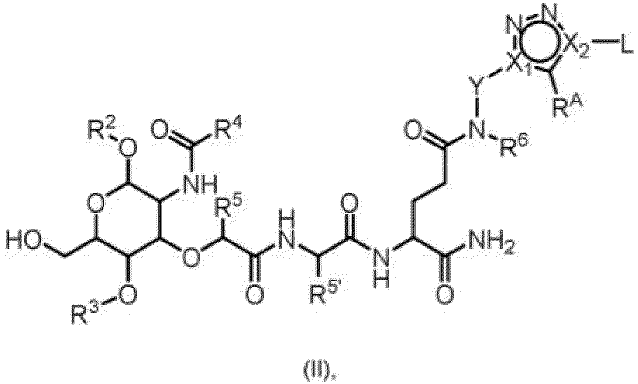 oder ein pharmazeutisch verträgliches Salz davon;
oder
(b) eine Verbindung der Formel (II-1): 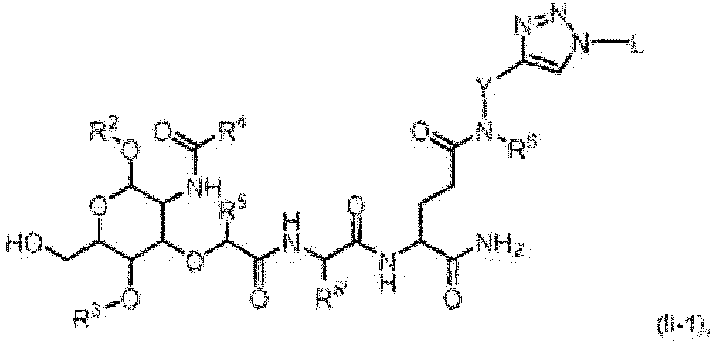 oder ein pharmazeutisch verträgliches Salz davon;
oder
(c) eine Verbindung der Formel (IIA): 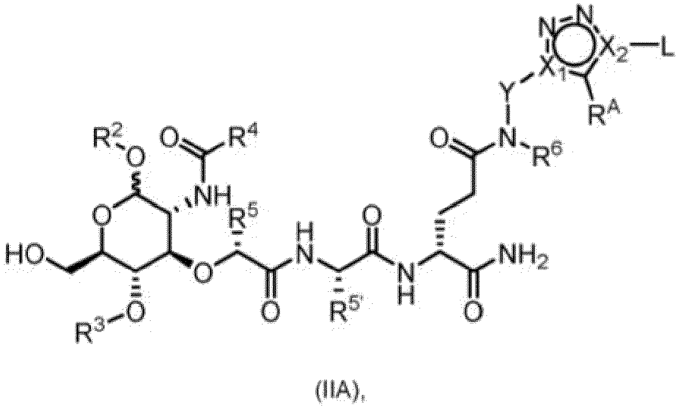 oder ein pharmazeutisch verträgliches Salz davon;
wobei:
X₁ -N- und X₂ -C- ist; oder X₁ -C- und X₂ -N- ist.

9. Verbindung nach Anspruch 8, wobei Y C₁₋₆-Alkylen ist.

10. Verbindung nach einem der Ansprüche 8 - 9, wobei R^{A} -H ist.

11. Verbindung nach einem der Ansprüche 1 - 10, wobei L -CH₂-C(O)-W ist.

12. Verbindung nach einem der Ansprüche 1 - 11, wobei W ein C₈₋₃₀-Alkyl ist.

13. Verbindung nach einem der Ansprüche 1 - 11, wobei W: 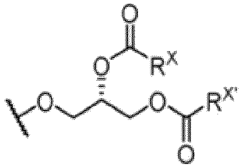 ist,
wobei R^{X} und R^{X'} jeweils unabhängig voneinander eine -C₈₋₃₀-Fettsäurekette sind; wobei gegebenenfalls R^{X} und R^{X'} jeweils unabhängig voneinander ein -C₈₋₃₀-Alkyl sind; wobei ferner gegebenenfalls R^{X} und R^{X'} beide -(CH₂CH₂)₈-CH₃ sind.

14. Verbindung nach einem der Ansprüche 1-11, wobei es sich bei W handelt um:
(a) Cholesterin: 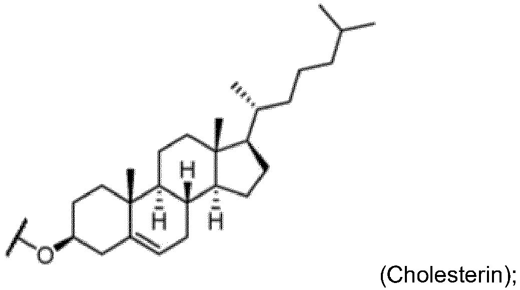 oder
(b) wobei W ein Phospholipid ist, ausgewählt aus der Gruppe bestehend aus: einem Phosphatidylcholin (PC), einem Phosphatidylglycerin (PG), einem Phosphatidylserin (PS), einem Phosphatidylethanolamin (PE), einer Phosphatidsäure (PA) und einem Lysophosphatidylcholin; oder
(c) ein Phospholipid mit der Struktur: 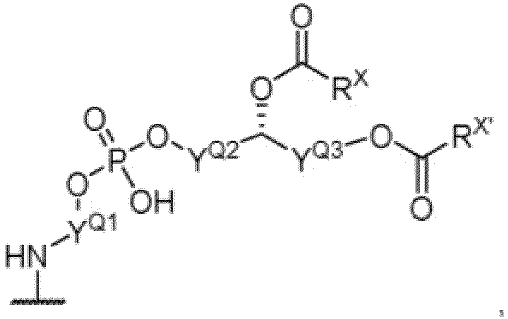 wobei
Y^{Q1}, Y^{Q2} und Y^{Q3} jeweils unabhängig voneinander Alkylen sind und R^{X} und R^{X'} jeweils unabhängig voneinander eine Fettsäurekette mit wenigstens 15 Kohlenstoffatomen sind; wobei gegebenenfalls R^{X} und R^{X'} jeweils unabhängig voneinander ein C₁₅₋₂₀-Alkyl oder ein C₁₅₋₂₀-Alkenyl sind; oder
(d) ein Phospholipid mit der Struktur: 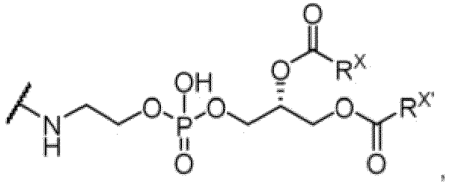 oder ein pharmazeutisch verträgliches Salz davon;
wobei R^{X} und R^{X'} jeweils unabhängig voneinander eine gegebenenfalls gesättigte C₈₋₃₀-Fettsäurekette sind; wobei R^{X} und R^{X'} gegebenenfalls jeweils unabhängig voneinander eine gegebenenfalls gesättigte C₁₂₋₁₈-Fettsäure sind; wobei ferner gegebenenfalls R^{X} und R^{X'} beide -(CH₂CH₂)₈-CH₃ sind.

15. Verbindung nach einem der Ansprüche 1-14, ausgewählt aus der Gruppe bestehend aus: 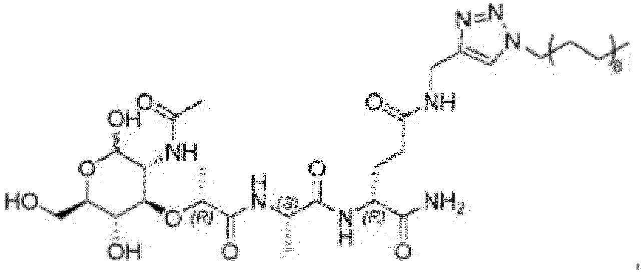 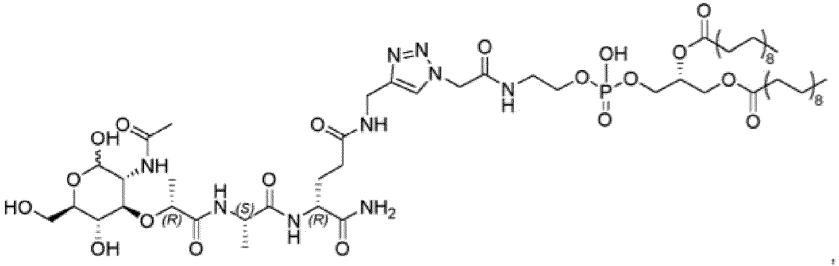 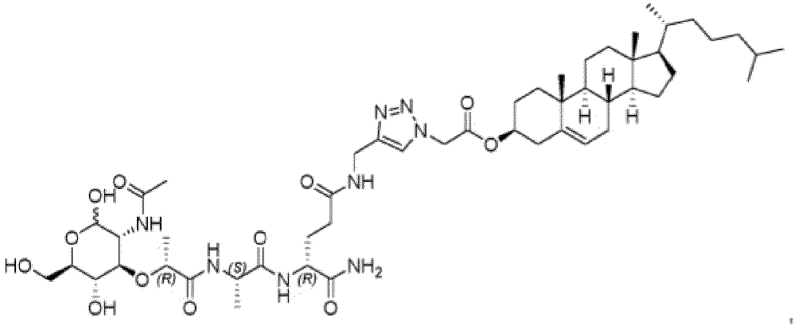 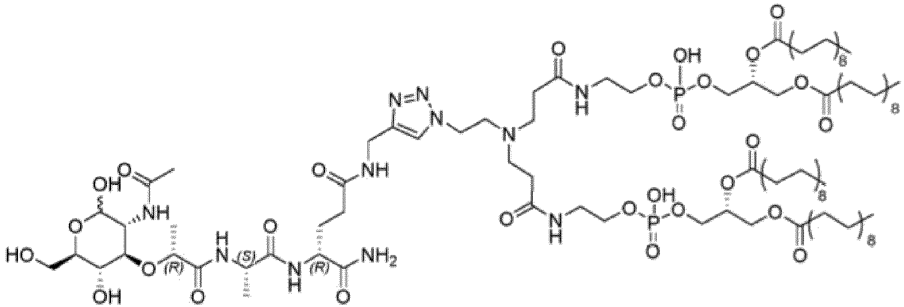 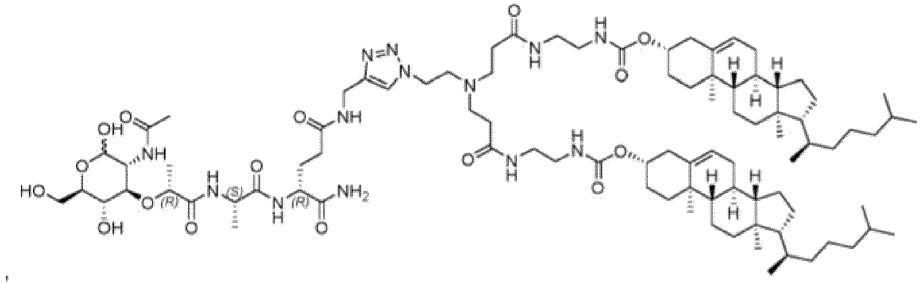 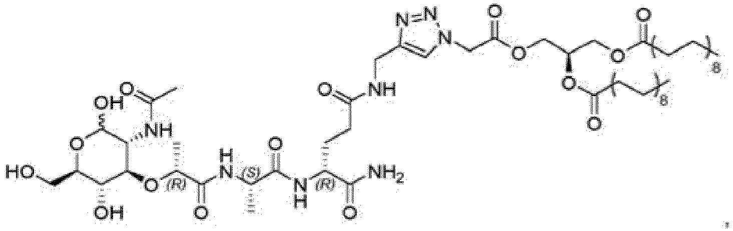 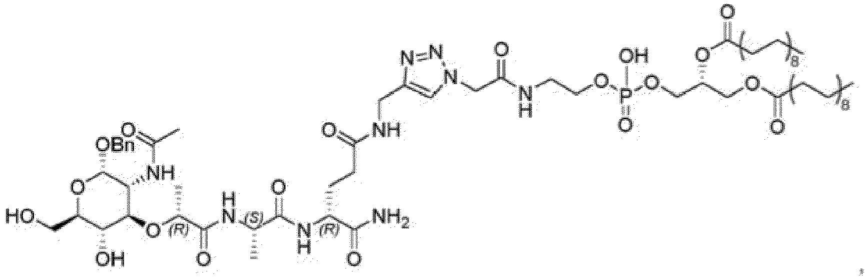 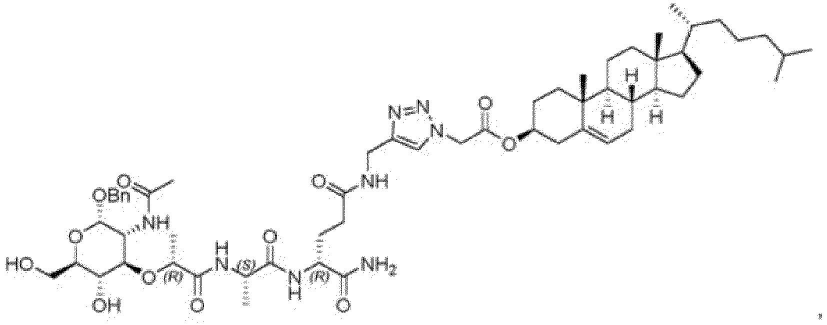 und einem pharmazeutisch verträglichen Salz davon, wobei Bn eine Benzylgruppe ist.

16. Verbindung nach Anspruch 15, wobei es sich bei der Verbindung handelt um:
(a) 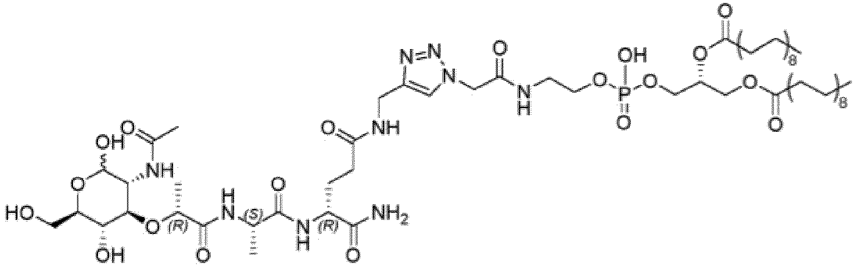 oder ein pharmazeutisch verträgliches Salz davon; oder
(b) 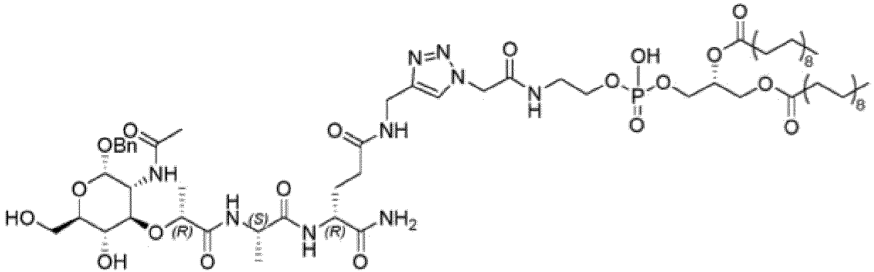 oder ein pharmazeutisch verträgliches Salz davon; oder
(c) 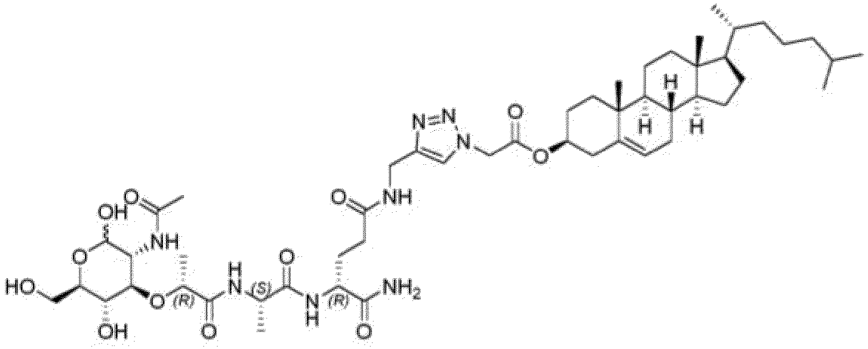 oder ein pharmazeutisch verträgliches Salz davon; oder
(d) 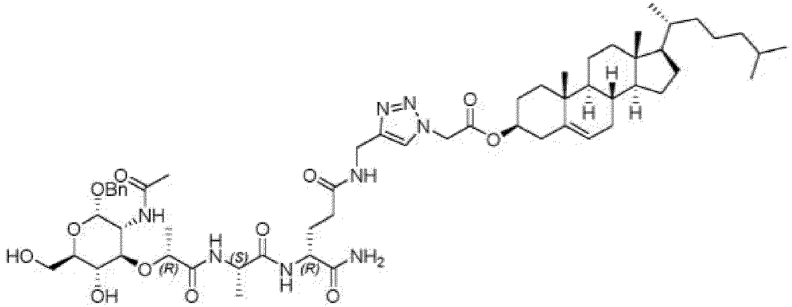 oder ein pharmazeutisch verträgliches Salz davon.

17. Nanobiologische Zusammensetzung, umfassend ein von Lipoprotein hoher Dichte (HDL) abgeleitetes Nanopartikel, wobei das Nanopartikel ein oder mehrere Phospholipide und eine Verbindung nach einem der Ansprüche 1 - 16 umfasst.

18. Nanobiologische Zusammensetzung nach Anspruch 17, wobei das Phospholipid unabhängig ausgewählt ist aus der Gruppe bestehend aus einem Phosphatidylcholin, einem Phosphatidylethanolamin, einem Phosphatidylinositol, einem Phosphatidylserin, einem Sphingomyelin oder einem anderen Ceramid, einem phospholipidhaltigen Öl, einem Phosphatidylglycerin, einer Phosphatidsäure, einem Lysophosphatidylcholin und Kombinationen davon.

19. Nanobiologische Zusammensetzung nach Anspruch 17, wobei das Nanopartikel umfasst:
(a) ein Phospholipid, das 1,2-Dimyristoyl-sn-glycero-3-phosphatidylcholin (DMPC) ist, und ein Lysolipid, das 1-Myristoyl-2-hydroxy-sn-glycero-phosphocholin (MHPC) ist; oder
(b) ein Phospholipid, das 1-Palmitoyl-2-oleoyl-sn-glycero-3-phosphocholin (POPC) ist, und ein Lysolipid, das 1-Palmitoyl-2-hydroxy-sn-glycero-3-phosphocholin (PHPC) ist.

20. Nanobiologische Zusammensetzung nach einem der Ansprüche 17 - 19, wobei das von HDL abgeleitete Nanopartikel ferner umfasst:
(a) ApoA-I oder ein Peptidmimetikum von ApoA-I; und/oder
(b) Cholesterin;
wobei das von HDL abgeleitete Nanopartikel gegebenenfalls ein oder mehrere Phospholipide und Cholesterin in einem molaren Verhältnis im Bereich von etwa 1 : 0,05 bis etwa 1 : 0,25 umfasst.

21. Pharmazeutische Zusammensetzung, umfassend eine Verbindung nach einem der Ansprüche 1 - 16 oder ein pharmazeutisch verträgliches Salz davon und einen pharmazeutisch verträglichen Träger.

22. Nanobiologische Zusammensetzung nach einem der Ansprüche 17 - 20 oder pharmazeutische Zusammensetzung nach Anspruch 21 zur Verwendung bei einem Verfahren zum Behandeln einer Zellproliferationsstörung bei einem Patienten, der dies benötigt.

## Revendications

1. Composé de formule (I) : 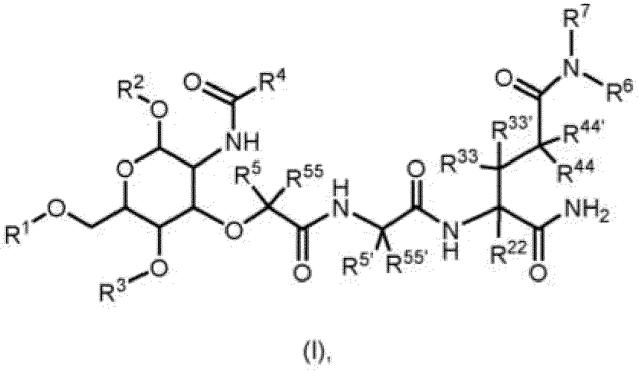
ou sel pharmaceutiquement acceptable de celui-ci,
dans lequel :
R¹ est -H ou -C(O)-R^{x} ;
R² et R³ sont chacun indépendamment choisis dans le groupe constitué de -H, alkyle, aryle, alkylène-aryle, -C(O)-alkyle et -C(O)-aryle ;
R⁴, R⁵ et R^{5'} sont chacun un alkyle ;
R⁶ et R¹¹ sont chacun indépendamment -H, ou un alkyle ;
R⁷ est -Y-triazolyle-L ;
Y est un alkylène ;
L est choisi dans le groupe constitué d'une chaîne d'acide gras, -alkylène-C(O)-W, -alkylène-O-C(O)-W, -alkylène-N-(alkylène-C(O)-NR¹¹-alkylène-NR¹¹-C(O)-W)₂ et -alkylène-N-(alkylène-C(O)-W)₂ ;
W est une chaîne d'acide gras, -O-alkylène-C(H)(OR⁸)-alkylène-OR⁹, un phospholipide ou un stérol ;
R⁸ et R⁹ sont chacun indépendamment R^{x} ou -C(O)-R^{x} ;
R²², R³³, R^{33'}, R⁴⁴, R^{44'}, R⁵⁵ et R^{55'} sont chacun indépendamment H ou R^{A} ;
R^{x} est une chaîne d'acide gras ;
dans lequel chaque alkyle, alkylène, alkylène-aryle, aryle et triazolyle susmentionné est éventuellement substitué par un ou plusieurs R^{A}, R^{A} étant indépendamment choisi à chaque occurrence dans le groupe constitué d'hydrogène, halogène, alcoxy, halogénoalcoxy, cyano, hydroxyle, -N(R^{c})(R^{D}), -C(O)N(R^{c})(R^{D}), -N(R^{c})C(O)R^{B}, -OC(O)NR^{c}R^{D}, -NR^{c}C(O)OR^{B}, -OC(O)R^{B}, - C(O)OR^{B}, -C(O)R^{B}, -CO₂H, -NO₂, -SH, S(O)ₓR^{B}, aryle, arylalkyle, hétéroaryle, hétéroarylalkyle, cycloalkyle, hétérocycloalkyle et R^{B} ; X étant 0, 1 ou 2 ;
R^{c} et R^{D} sont indépendamment choisis à chaque occurrence dans le groupe constitué d'hydrogène, alkyle, halogénoalkyle -C(O)R^{B} et -C(O)OR^{B} ; ou R^{c} et R^{D} sont pris ensemble avec l'azote auquel ils sont liés pour former un cycle hétérocyclique éventuellement substitué par R^{A} ; et
R^{B} est un alkyle, un alcényle ou un alcynyle éventuellement substitué par un ou plusieurs fluoro.

2. Composé selon la revendication 1, dans lequel le composé de formule (I) est un composé de formule (IA) : 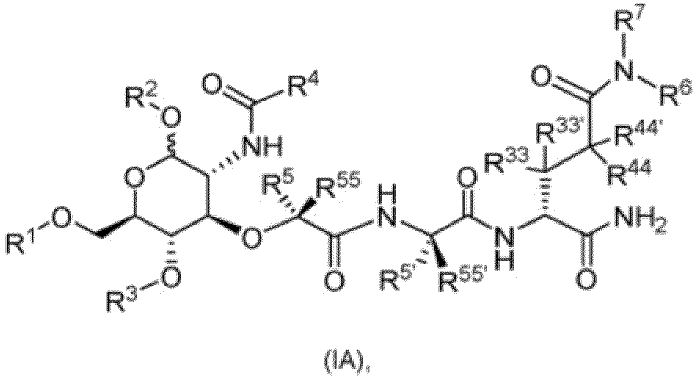 ou un sel pharmaceutiquement acceptable de celui-ci.

3. Composé selon la revendication 1 ou 2, dans lequel R² est -H ou benzyle ; éventuellement dans lequel R² est -H.

4. Composé selon l'une quelconque des revendications 1-3, dans lequel R²², R³³, R^{33'}, R⁴⁴, R^{44'}, R⁵⁵ et R^{55'} sont chacun -H.

5. Composé selon l'une quelconque des revendications 1-4, dans lequel R⁴ est un alkyle ; éventuellement un méthyle.

6. Composé selon l'une quelconque des revendications 1-5, dans lequel R³ et R⁶ sont tous deux -H.

7. Composé selon l'une quelconque des revendications 1-6, dans lequel Y est -CH₂--

8. Composé selon l'une quelconque des revendications 1-7, dans lequel le composé de formule (I) est :
(a) un composé de formule (II) : 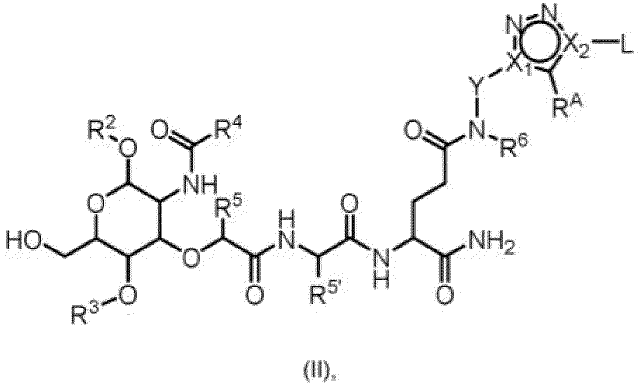 ou un sel pharmaceutiquement acceptable de celui-ci ;
ou
(b) un composé de formule (I1-1) : 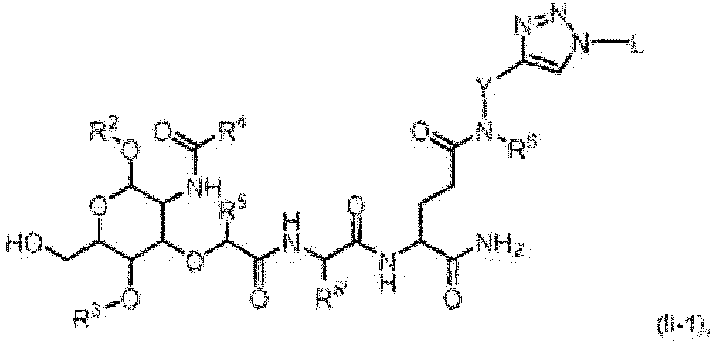 ou un sel pharmaceutiquement acceptable de celui-ci ;
ou
(c) un composé de formule (IIA) : 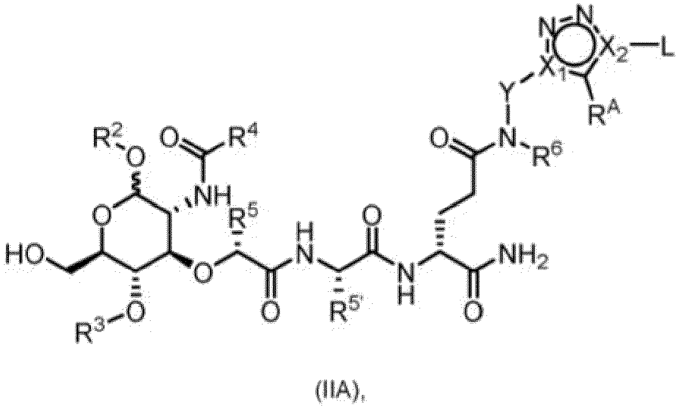 ou un sel pharmaceutiquement acceptable de celui-ci ;
dans lequel :
X₁ est -N- et X₂ est -C- ; ou X₁ est -C- et X₂ est -N-.

9. Composé selon la revendication 8, dans lequel Y est un alkylène en C₁-₆.

10. Composé selon l'une quelconque des revendications 8-9, dans lequel R^{A} est -H.

11. Composé selon l'une quelconque des revendications 1-10, dans lequel L est -CH₂-C(O)-W.

12. Composé selon l'une quelconque des revendications 1-11, dans lequel W est un alkyle en C₈-₃₀.

13. Composé selon l'une quelconque des revendications 1-11, dans lequel W est : 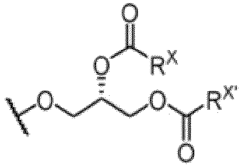 dans lequel R^{x} et R^{x}' sont chacun indépendamment une chaîne d'acide gras en -C₈-₃₀ ; éventuellement dans lequel R^{x} et R^{x}' sont chacun indépendamment un alkyle en -C₈-₃₀ ; en outre éventuellement dans lequel R^{x} et R^{x}' sont tous deux -(CH₂CH₂)ₛ-CH₃-

14. Composé selon l'une quelconque des revendications 1-11, dans lequel W est :
(a) du cholestérol : 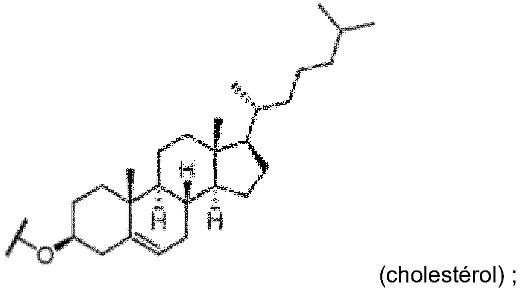 ou
(b) dans lequel W est un phospholipide choisi dans le groupe constitué de : une phosphatidylcholine (PC), un phosphatidylglycérol (PG), une phosphatidylsérine (PS), une phosphatidyléthanolamine (PE), un acide phosphatidique (PA) et une lysophosphatidylcholine; ou
(c) un phospholipide ayant la structure : 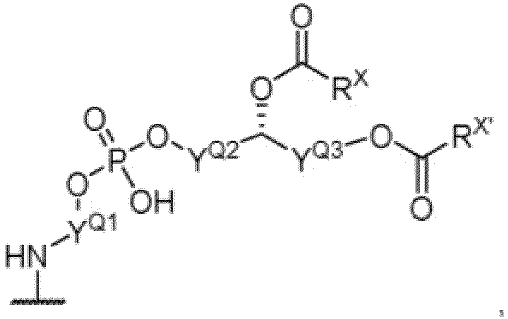 dans lequel
Y^{Q1}, Y^{Q2} et Y^{Q3} sont chacun indépendamment un alkylène et R^{x} et R^{x}' sont chacun indépendamment une chaîne d'acide gras ayant au moins 15 carbones ; éventuellement dans lequel R^{x} et R^{x}' sont chacun indépendamment un alkyle en C₁₅-₂₀ ou un alcényle en C₁₅-₂₀ ; ou
(d) un phospholipide ayant la structure : 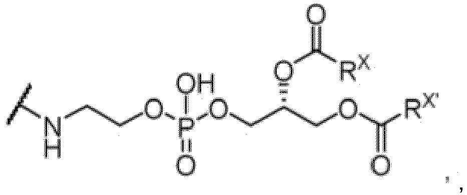 ou un sel pharmaceutiquement acceptable de celui-ci ;
dans lequel R^{x} et R^{x}' sont chacun indépendamment une chaîne d'acide gras en C₈-₃₀ éventuellement saturée ; éventuellement dans lequel R^{x} et R^{x}' sont chacun indépendamment un acide gras en C₁₂-₁₈ éventuellement saturé ; en outre éventuellement dans lequel R^{x} et R^{x}' sont tous deux -(CH₂CH₂)₈-CH₃.

15. Composé selon l'une quelconque des revendications 1-14 choisi dans le groupe constitué de : 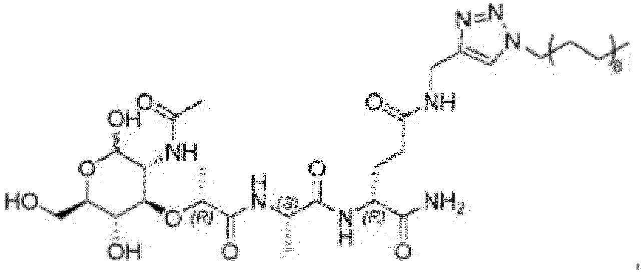 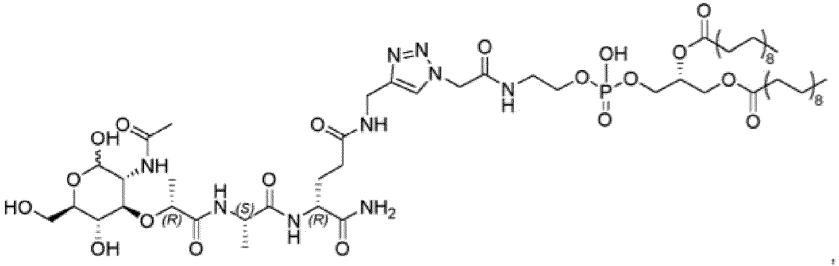 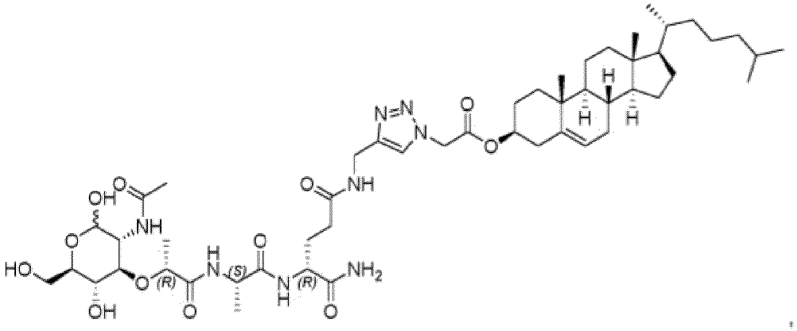 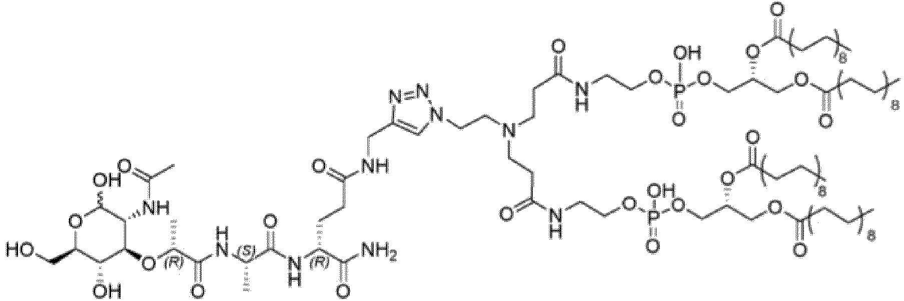 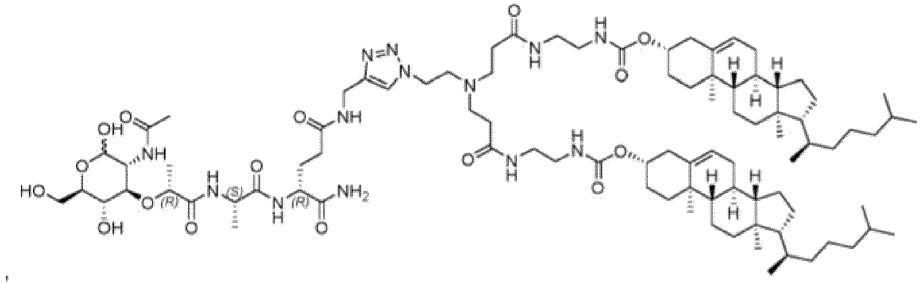 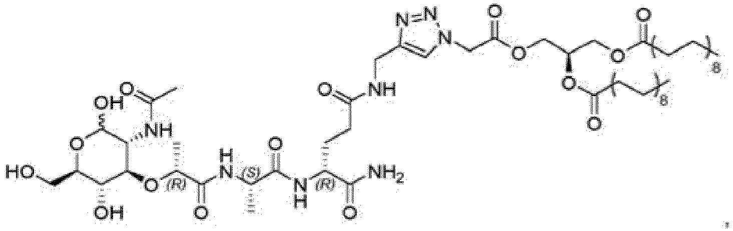 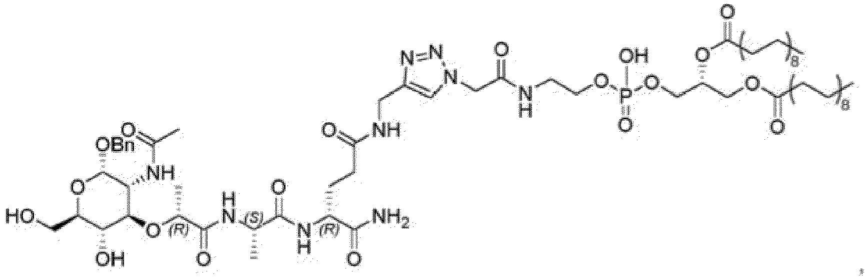 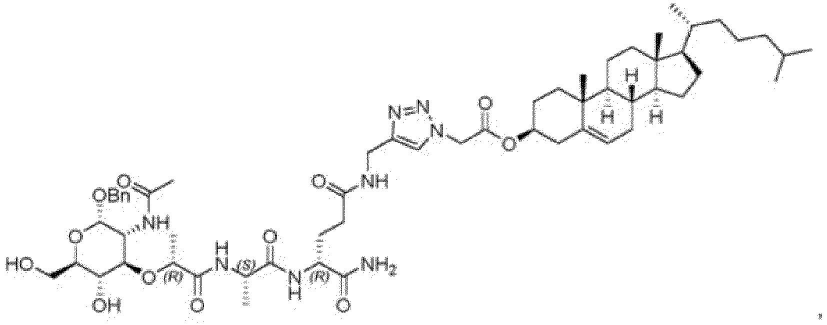 et d'un sel pharmaceutiquement acceptable de celui-ci, dans lequel Bn est un groupe benzyle.

16. Composé selon la revendication 15, dans lequel le composé est :
(a) 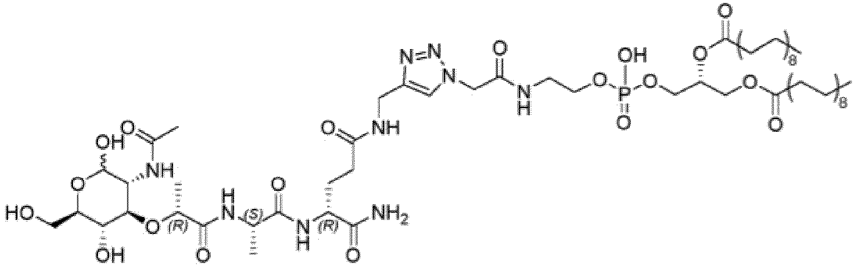 ou un sel pharmaceutiquement acceptable de celui-ci ; ou
(b) 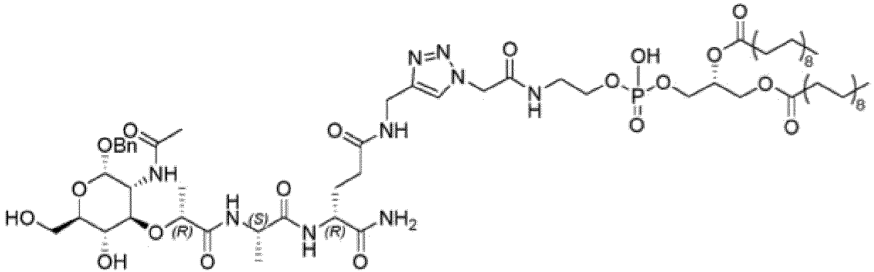 ou un sel pharmaceutiquement acceptable de celui-ci ; ou
(c) 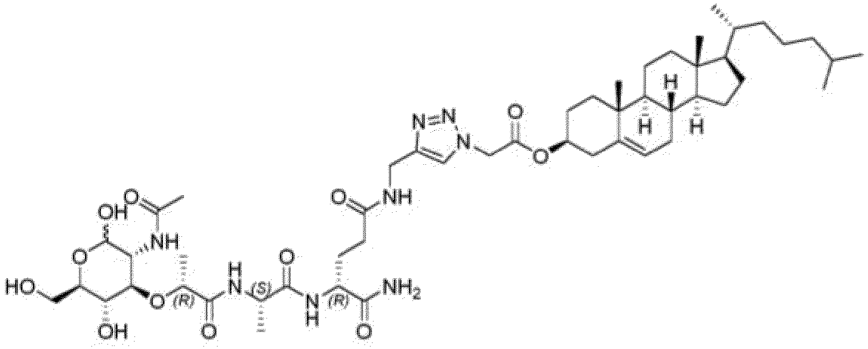 ou un sel pharmaceutiquement acceptable de celui-ci ; ou
(d) 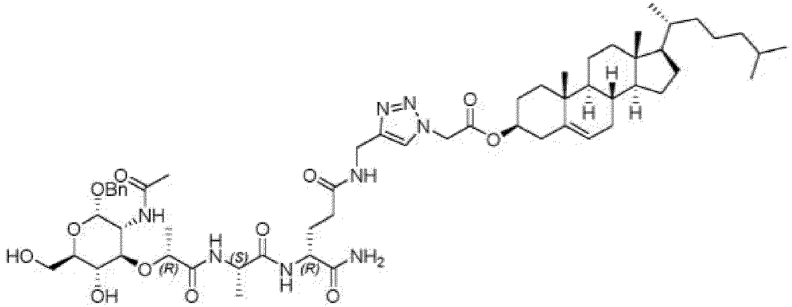 ou un sel pharmaceutiquement acceptable de celui-ci.

17. Composition nanobiologique, comprenant une nanoparticule dérivée d'une lipoprotéine de haute densité (HDL), dans laquelle la nanoparticule comprend un ou plusieurs phospholipides et un composé selon l'une quelconque des revendications 1-16.

18. Composition nanobiologique selon la revendication 17, dans laquelle le phospholipide est indépendamment choisi dans le groupe constitué d'une phosphatidylcholine, d'une phosphatidyléthanolamine, d'un phosphatidylinositol, d'une phosphatidylsérine, d'une sphingomyéline ou d'un autre céramide, d'une huile contenant des phospholipides, d'un phosphatidylglycérol, d'un acide phosphatidique, d'une lysophosphatidylcholine et de combinaisons de ceux-ci.

19. Composition nanobiologique selon la revendication 17, dans laquelle la nanoparticule comprend :
(a) un phospholipide qui est la 1,2-dimyristoyl-sn-glycéro-3-phosphatidylcholine (DMPC) et un lysolipide qui est la 1-myristoyl-2-hydroxy-sn-glycéro-phosphocholine (MHPC) ; ou
(b) un phospholipide qui est la 1-palmitoyl-2-oléoyl-sn-glycéro-3-phosphocholine (POPC), et un lysolipide qui est la 1-palmitoyl-2-hydroxy-sn-glycéro-3-phosphocholine (PHPC).

20. Composition nanobiologique selon l'une quelconque des revendications 17-19, dans laquelle la nanoparticule dérivée de HDL comprend en outre :
(a) de l'apoA-I ou un mimétique peptidique de l'apoA-I ; et/ou
(b) du cholestérol ;
éventuellement dans laquelle la nanoparticule dérivée de HDL comprend un ou plusieurs phospholipides et du cholestérol en un rapport molaire dans la plage d'environ 1:0,05 à environ 1:0,25.

21. Composition pharmaceutique comprenant un composé selon l'une quelconque des revendications 1-16 ou un sel pharmaceutiquement acceptable de celui-ci, et un véhicule pharmaceutiquement acceptable.

22. Composition nanobiologique selon l'une quelconque des revendications 17-20 ou composition pharmaceutique selon la revendication 21 pour une utilisation dans un procédé de traitement d'un trouble de prolifération cellulaire chez un patient en ayant besoin.
